# EUROPEAN PATENT APPLICATION

(11) **EP 3 725 802 A2**
(43) Date of publication of application: **21.10.2020**
(21) Application number: 18879299.8
(22) Date of filing: 16.11.2018
(51) Int. Cl.: C07K 16/18, G01N 33/68, A61P 25/16, A61P 25/28

(54) **ANTIBODIES TO ALPHA-SYNUCLEIN AND USES THEREOF**

(30) Priority: 17.11.2017 US 201762588018 P; 21.06.2018 US 201862687848 P
(71) Applicant: ABL Bio Inc., Seongnam-si, Gyeonggi-do 13488 (KR)
(72) Inventor: LEE, Sang Hoon, Seongnam-si Gyeonggi-do 13488 (KR); YOU, Weonkyoo, Seongnam-si Gyeonggi-do 13488 (KR); AHN, Jinhyung, Seongnam-si Gyeonggi-do 13488 (KR); AN, Sungwon, Seongnam-si Gyeonggi-do 13488 (KR); JUNG, Jinwon, Seongnam-si Gyeonggi-do 13488 (KR); LEE, Bora, Seongnam-si Gyeonggi-do 13488 (KR); KIM, Dongin, Seongnam-si Gyeonggi-do 13488 (KR); SUNG, Byungje, Seongnam-si Gyeonggi-do 13488 (KR); SON, Yonggyu, Seongnam-si Gyeonggi-do 13488 (KR); KIM, Yeunju, Seongnam-si Gyeonggi-do 13488 (KR); EOM, Jaehyun, Seongnam-si Gyeonggi-do 13488 (KR); AHN, Seawon, Seongnam-si Gyeonggi-do 13488 (KR); PAK, Youngdon, Seongnam-si Gyeonggi-do 13488 (KR); YEOM, Donghoon, Seongnam-si Gyeonggi-do 13488 (KR); SONG, Daehae, Seongnam-si Gyeonggi-do 13488 (KR)
(74) Representative: Hoppe, Georg Johannes
(86) International application number: PCT/KR2018/014123
(87) International publication number: WO 2019/098763

(57) **Abstract**

The present invention relates to an anti-alpha-synuclein antibody preferentially recognizing alpha-synuclein aggregates and a use of detection, diagnosis, and/or treatment or prevention of various diseases caused by accumulation of alpha-synuclein aggregates, or their related symptom diseases by using the anti-alpha-synuclein antibody.

<110>    ABL Bio Inc.

<120>    Anti alpha-synuclein antibody and its use

<130>    OPP20184675KR

<150>    62/588,018
<151>    2017-11-17

<150>    62/687,848
<151>    2018-06-21

<160>    126

<170>    KopatentIn 2.0

<210>    1
<211>    10
<212>    PRT
<213>    Artificial Sequence

<220>
<223>    Hu11F11 H-CDR1

<400>    1
Gly Phe Thr Phe Ser Asp Phe Tyr Met Glu
  1               5                  10

<210>    2
<211>    19
<212>    PRT
<213>    Artificial Sequence

<220>
<223>    Hu11F11 H-CDR2

<400>    2
Ala Ser Arg Asn Lys Ala Asn Asp Tyr Thr Thr Glu Tyr Ser Ala Ser
  1               5                  10                  15

Val Lys Gly

<210>    3
<211>    19
<212>    PRT
<213>    Artificial Sequence

<220>
<223>    Hu11F11 H-CDR2

<400>    3
Ala Ile Arg Asn Lys Ala Asn Asp Tyr Thr Thr Glu Tyr Ala Ala Ser
1               5                   10                  15

Val Lys Gly

<210>    4
<211>    19
<212>    PRT
<213>    Artificial Sequence

<220>
<223>    Hu11F11 H-CDR2

<400>    4
Ala Thr Arg Asn Lys Ala Asn Asp Tyr Thr Thr Glu Tyr Ser Ala Ser
1               5                   10                  15

Val Lys Gly

<210>    5
<211>    9
<212>    PRT
<213>    Artificial Sequence

<220>
<223>    Hu11F11 H-CDR3

<400>    5
Asp Ala His Gly Lys Pro Phe Ala Tyr
1               5

<210>    6
<211>    10
<212>    PRT
<213>    Artificial Sequence

<220>
<223>    Hu3A9 H-CDR1

<400>    6
Gly Phe Thr Phe Ser Ser Tyr Ala Met Ser
1               5                   10

<210>    7
<211>    17
<212>    PRT
<213>    Artificial Sequence

<220>
<223>    Hu3A9 H-CDR2

<400>    7
Thr Ile Ser Asn Gly Gly Gly Tyr Thr Tyr Tyr Pro Asp Ser Val Lys
1               5                   10                  15

Gly

<210>    8
<211>    17
<212>    PRT
<213>    Artificial Sequence

<220>
<223>    Hu3A9 H-CDR2

<400>    8
Thr Ile Ser Asn Gly Gly Gly Tyr Thr Tyr Tyr Ala Asp Ser Val Lys
1               5                   10                  15

Gly

<210>    9
<211>    13
<212>    PRT
<213>    Artificial Sequence

<220>
<223>    Hu3A9 H-CDR3

<400>    9

His Ile Thr Thr Val Arg Pro Thr Lys Tyr Phe Asp Tyr
1               5                   10

<210>   10
<211>   17
<212>   PRT
<213>   Artificial Sequence

<220>
<223>   Hu11F11 L-CDR1

<400>   10
Lys Ser Ser Gln Ser Leu Leu Tyr Ser Ser Asn Gln Lys Asn Tyr Leu
1               5                   10                  15

Ala

<210>   11
<211>   7
<212>   PRT
<213>   Artificial Sequence

<220>
<223>   Hu11F11 L-CDR2

<400>   11
Trp Ala Ser Thr Arg Glu Ser
1               5

<210>   12
<211>   9
<212>   PRT
<213>   Artificial Sequence

<220>
<223>   Hu11F11 L-CDR3

<400>   12
Gln Gln Tyr Tyr Ser Tyr Pro Trp Thr
1               5

<210>   13
<211>   11

<212>	PRT
<213>	Artificial Sequence

<220>
<223>	Hu3A9 L-CDR1

<400>	13
Lys Ala Ser Gln Asn Val Gly Thr Thr Val Ala
1               5                   10

<210>	14
<211>	7
<212>	PRT
<213>	Artificial Sequence

<220>
<223>	Hu3A9 L-CDR2

<400>	14
Ser Ala Ser Asn Arg Tyr Thr
1               5

<210>	15
<211>	9
<212>	PRT
<213>	Artificial Sequence

<220>
<223>	Hu3A9 L-CDR3

<400>	15
Gln Gln Tyr Ser Asn Tyr Pro Leu Thr
1               5

<210>	16
<211>	25
<212>	PRT
<213>	Artificial Sequence

<220>
<223>	VH Framwork 1 of ch11F11

<400>	16
Glu Val Gln Leu Gln Glu Ser Gly Gly Gly Leu Val Gln Pro Gly Gly

1               5                   10                  15

Ser Leu Arg Leu Ser Cys Ala Thr Ser
            20                  25

<210>   17
<211>   25
<212>   PRT
<213>   Artificial Sequence

<220>
<223>   VH Framwork 1 of  Hu11F11

<400>   17

<210>   18
<211>   14
<212>   PRT
<213>   Artificial Sequence

<220>
<223>   VH Framwork 2 of  Hu11F11

<400>   18
Trp Val Arg Gln Pro Pro Gly Lys Arg Leu Glu Trp Ile Ala
1               5                   10

<210>   19
<211>   14
<212>   PRT
<213>   Artificial Sequence

<220>
<223>   VH Framwork 2 of  Hu11F11

<400>   19
Trp Val Arg Gln Ala Pro Gly Lys Gly Leu Glu Trp Ile Ala
1               5                   10

<210> 20
<211> 32
<212> PRT
<213> Artificial Sequence

<220>
<223> VH Framwork 3 of Hu11F11

<400> 20
Arg Phe Ile Val Ser Arg Asp Thr Ser Gln Ser Ile Leu Tyr Leu Gln
1               5                   10                  15

Met Asn Ala Leu Arg Ala Glu Asp Thr Ala Ile Tyr Tyr Cys Ala Arg
            20                  25                  30

<210> 21
<211> 32
<212> PRT
<213> Artificial Sequence

<220>
<223> VH Framwork 3 of Hu11F11

<400> 21
Arg Phe Thr Val Ser Arg Asp Thr Ser Lys Asn Ser Leu Tyr Leu Gln
1               5                   10                  15

Met Asn Ser Leu Lys Thr Glu Asp Thr Ala Val Tyr Tyr Cys Ala Arg
            20                  25                  30

<210> 22
<211> 32
<212> PRT
<213> Artificial Sequence

<220>
<223> VH Framwork 3 of Hu11F11

<400> 22
Arg Phe Thr Ile Ser Arg Asp Thr Ser Lys Asn Ser Leu Tyr Leu Gln
1               5                   10                  15

Met Asn Ser Leu Lys Thr Glu Asp Thr Ala Val Tyr Tyr Cys Ala Arg
            20                  25                  30

<210> 23
<211> 32
<212> PRT
<213> Artificial Sequence

<220>
<223> VH Framwork 3 of Hu11F11

<400> 23
Arg Phe Thr Val Ser Arg Asp Thr Ser Gln Asn Thr Leu Tyr Leu Gln
1               5                   10                  15

Met Asn Ser Leu Arg Ala Glu Asp Thr Ala Val Tyr Tyr Cys Ala Arg
            20                  25                  30

<210> 24
<211> 32
<212> PRT
<213> Artificial Sequence

<220>
<223> VH Framwork 3 of Hu11F11

<400> 24
Arg Phe Thr Ile Ser Ala Asp Thr Ser Lys Asn Thr Ala Tyr Leu Gln
1               5                   10                  15

Met Asn Ser Leu Arg Ala Glu Asp Thr Ala Val Tyr Tyr Cys Ser Arg
            20                  25                  30

<210> 25
<211> 32
<212> PRT
<213> Artificial Sequence

<220>
<223> VH Framwork 3 of Hu11F11

<400>    25
Arg Phe Thr Ile Ser Arg Asp Asp Ser Lys Ser Ser Leu Tyr Leu Gln
1               5                   10                  15

Met Asn Ser Leu Arg Ala Glu Asp Thr Ala Ile Tyr Tyr Cys Ala Arg
            20                  25                  30

<210>    26
<211>    32
<212>    PRT
<213>    Artificial Sequence

<220>
<223>    VH Framwork 3 of  Hu11F11

<400>    26
Arg Phe Thr Ile Ser Arg Asp Thr Ser Gln Ser Ser Leu Tyr Leu Gln
1               5                   10                  15

Met Asn Ser Leu Lys Thr Glu Asp Thr Ala Val Tyr Tyr Cys Ala Arg
            20                  25                  30

<210>    27
<211>    32
<212>    PRT
<213>    Artificial Sequence

<220>
<223>    VH Framwork 3 of  Hu11F11

<400>    27
Arg Phe Thr Ile Ser Arg Asp Thr Ser Gln Ser Ser Leu Tyr Leu Gln
1               5                   10                  15

Met Asn Ser Leu Arg Ala Glu Asp Thr Ala Ile Tyr Tyr Cys Ala Arg
            20                  25                  30

<210>    28

<211>	32
<212>	PRT
<213>	Artificial Sequence

<220>
<223>	VH Framwork 3 of  Hu11F11

<400>	28
Arg Phe Thr Ile Ser Arg Asp Asp Ser Lys Ser Ser Leu Tyr Leu Gln
1               5                   10                  15

Met Asn Ser Leu Arg Ala Glu Asp Thr Ala Ile Tyr Tyr Cys Ala Arg
            20                  25                  30

<210>	29
<211>	32
<212>	PRT
<213>	Artificial Sequence

<220>
<223>	VH Framwork 3 of  Hu11F11

<400>	29
Arg Phe Thr Val Ser Arg Asp Asp Ser Lys Ser Ser Leu Tyr Leu Gln
1               5                   10                  15

Met Asn Ser Leu Arg Ala Glu Asp Thr Ala Ile Tyr Tyr Cys Ala Arg
            20                  25                  30

<210>	30
<211>	32
<212>	PRT
<213>	Artificial Sequence

<220>
<223>	VH Framwork 3 of  Hu11F11

<400>	30
Arg Phe Thr Ile Ser Arg Asp Thr Ser Lys Ser Ser Leu Tyr Leu Gln
1               5                   10                  15

Met Asn Ser Leu Arg Ala Glu Asp Thr Ala Ile Tyr Tyr Cys Ala Arg

            20                  25                  30

<210> 31
<211> 32
<212> PRT
<213> Artificial Sequence

<220>
<223> VH Framwork 3 of Hu11F11

<400> 31
Arg Phe Thr Val Ser Arg Asp Thr Ser Lys Ser Ser Leu Tyr Leu Gln
1               5                   10                  15

Met Asn Ser Leu Arg Ala Glu Asp Thr Ala Ile Tyr Tyr Cys Ala Arg
            20                  25                  30

<210> 32
<211> 11
<212> PRT
<213> Artificial Sequence

<220>
<223> VH Framwork 4 of Hu11F11

<400> 32
Trp Gly Gln Gly Thr Leu Val Thr Val Ser Ala
1               5                   10

<210> 33
<211> 11
<212> PRT
<213> Artificial Sequence

<220>
<223> VH Framwork 4 of Hu11F11

<400> 33
Trp Gly Gln Gly Thr Leu Val Thr Val Ser Ser
1               5                   10

<210>	34
<211>	11
<212>	PRT
<213>	Artificial Sequence

<220>
<223>	VH Framwork 4 of  Hu11F11


<400>	34
Trp Gly Gln Gly Thr Thr Val Thr Val Ser Ser
1               5                   10


<210>	35
<211>	25
<212>	PRT
<213>	Artificial Sequence

<220>
<223>	VH Framwork 1 of ch3A9


<400>	35
Glu Val Gln Leu Gln Glu Ser Gly Gly Gly Leu Val Lys Pro Gly Gly
1               5                   10                  15

Ser Leu Lys Leu Ser Cys Ala Ala Ser
            20                  25


<210>	36
<211>	25
<212>	PRT
<213>	Artificial Sequence

<220>
<223>	VH Framwork 1 of Hu3A9


<400>	36
Gln Val Gln Leu Leu Glu Ser Gly Gly Gly Leu Val Gln Pro Gly Gly
1               5                   10                  15

Ser Leu Arg Leu Ser Cys Ala Ala Ser
            20                  25


<210>	37
<211>	25

<212>    PRT
<213>    Artificial Sequence

<220>
<223>    VH Framwork 1 of Hu3A9


<400>    37
Glu Val Gln Leu Val Gln Ser Gly Gly Gly Leu Val Gln Pro Gly Gly
1               5                   10                  15

Ser Leu Arg Leu Ser Cys Ala Ala Ser
            20                  25


<210>    38
<211>    25
<212>    PRT
<213>    Artificial Sequence

<220>
<223>    VH Framwork 1 of Hu3A9


<400>    38


<210>    39
<211>    25
<212>    PRT
<213>    Artificial Sequence

<220>
<223>    VH Framwork 1 of Hu3A9


<400>    39
Glu Val Gln Leu Leu Glu Ser Gly Gly Gly Leu Val Gln Pro Gly Gly
1               5                   10                  15

Ser Leu Arg Leu Ser Cys Ala Ala Ser
            20                  25


<210>    40
<211>    25

<212>    PRT
<213>    Artificial Sequence

<220>
<223>    VH Framwork 1 of Hu3A9


<400>    40
Glu Val Gln Leu Leu Glu Ser Gly Gly Gly Leu Val Gln Pro Gly Gly
1               5                   10                  15

Ser Leu Lys Leu Ser Cys Ala Ala Ser
            20                  25


<210>    41
<211>    14
<212>    PRT
<213>    Artificial Sequence

<220>
<223>    VH Framwork 2 of Hu3A9


<400>    41
Trp Val Arg Gln Thr Pro Glu Lys Arg Leu Glu Trp Val Ala
1               5                   10


<210>    42
<211>    14
<212>    PRT
<213>    Artificial Sequence

<220>
<223>    VH Framwork 2 of Hu3A9


<400>    42
Trp Val Arg Gln Ala Pro Gly Lys Gly Leu Glu Trp Val Ala
1               5                   10


<210>    43
<211>    14
<212>    PRT
<213>    Artificial Sequence

<220>
<223>    VH Framwork 2 of Hu3A9

<400>    43
Trp Val Arg Gln Ala Pro Asp Lys Gly Leu Glu Trp Val Ala
1               5                   10

<210>    44
<211>    14
<212>    PRT
<213>    Artificial Sequence

<220>
<223>    VH Framwork 2 of Hu3A9

<400>    44
Trp Val Arg Gln Thr Pro Glu Lys Gly Leu Glu Trp Val Ala
1               5                   10

<210>    45
<211>    32
<212>    PRT
<213>    Artificial Sequence

<220>
<223>    VH Framwork 3 of Hu3A9

<400>    45
Arg Phe Thr Ile Ser Arg Asp Asn Ala Lys Asn Thr Leu Tyr Leu Gln
1               5                   10                  15

Met Ser Ser Leu Arg Ser Glu Asp Thr Ala Met Tyr Tyr Cys Ala Arg
            20                  25                  30

<210>    46
<211>    32
<212>    PRT
<213>    Artificial Sequence

<220>
<223>    VH Framwork 3 of Hu3A9

<400>    46
Arg Phe Thr Ile Ser Arg Asp Asn Ala Lys Asn Thr Leu Tyr Leu Gln
1               5                   10                  15

Met Asn Ser Leu Arg Ser Glu Asp Ser Ala Met Tyr Tyr Cys Ala Arg
            20                  25                  30

<210> 47
<211> 32
<212> PRT
<213> Artificial Sequence

<220>
<223> VH Framwork 3 of Hu3A9

<400> 47
Arg Phe Thr Ile Ser Arg Asp Asn Ala Lys Asn Thr Leu Tyr Leu Gln
1               5                   10                  15

Met Ser Ser Leu Lys Ala Glu Asp Ser Ala Val Tyr Tyr Cys Ala Arg
            20                  25                  30

<210> 48
<211> 32
<212> PRT
<213> Artificial Sequence

<220>
<223> VH Framwork 3 of Hu3A9

<400> 48
Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Ala Arg
            20                  25                  30

<210> 49
<211> 32
<212> PRT
<213> Artificial Sequence

<220>

<223>    VH Framwork 3 of Hu3A9

<400>    49
Arg Phe Thr Ile Ser Ala Asp Thr Ser Lys Asn Thr Ala Tyr Leu Gln
1               5                   10                  15

Met Asn Ser Leu Arg Ala Glu Asp Thr Ala Val Tyr Tyr Cys Ala Arg
            20                  25                  30

<210>    50
<211>    32
<212>    PRT
<213>    Artificial Sequence

<220>
<223>    VH Framwork 3 of Hu3A9

<400>    50
Arg Phe Thr Ile Ser Arg Asp Asn Ser Lys Asn Thr Leu Tyr Leu Gln
1               5                   10                  15

Met Ser Ser Leu Arg Ala Glu Asp Thr Ala Met Tyr Tyr Cys Ala Arg
            20                  25                  30

<210>    51
<211>    11
<212>    PRT
<213>    Artificial Sequence

<220>
<223>    VH Framwork 4 of Hu3A9

<400>    51
Trp Gly Gln Gly Thr Thr Leu Thr Val Ser Ser
1               5                   10

<210>    52
<211>    11
<212>    PRT
<213>    Artificial Sequence

<220>
<223>    VH Framwork 4 of Hu3A9

<400>    52
Trp Gly Gln Gly Thr Leu Val Thr Val Ser Ser
1               5                   10

<210>    53
<211>    23
<212>    PRT
<213>    Artificial Sequence

<220>
<223>    VL Framwork 1 of ch11F11

<400>    53
Asp Ile Val Met Thr Gln Ser Pro Ser Ser Leu Ala Val Ser Val Gly
1               5                   10                  15

Glu Lys Val Thr Met Ser Cys
            20

<210>    54
<211>    23
<212>    PRT
<213>    Artificial Sequence

<220>
<223>    VL Framwork 1 of  Hu11F11

<400>    54
Asp Ile Val Met Thr Gln Ser Pro Asp Ser Leu Ala Val Ser Leu Gly
1               5                   10                  15

Glu Arg Ala Thr Ile Asn Cys
            20

<210>    55
<211>    23
<212>    PRT
<213>    Artificial Sequence

<220>
<223>    VL Framwork 1 of  Hu11F11

<400>    55
Asp Ile Val Met Thr Gln Ser Pro Ser Ser Leu Ala Val Ser Leu Gly
1               5                   10                  15

Glu Arg Val Thr Ile Thr Cys
            20

<210>    56
<211>    23
<212>    PRT
<213>    Artificial Sequence

<220>
<223>    VL Framwork 1 of  Hu11F11

<400>    56
Asp Ile Val Met Thr Gln Ser Pro Ser Ser Leu Ala Val Ser Leu Gly
1               5                   10                  15

Glu Arg Ala Thr Ile Asn Cys
            20

<210>    57
<211>    23
<212>    PRT
<213>    Artificial Sequence

<220>
<223>    VL Framwork 1 of  Hu11F11

<400>    57
Asp Ile Gln Met Thr Gln Ser Pro Ser Ser Leu Ser Ala Ser Val Gly
1               5                   10                  15

Asp Arg Val Thr Ile Thr Cys
            20

<210>    58
<211>    23
<212>    PRT
<213>    Artificial Sequence

<220>
<223>    VL Framwork 1 of  Hu11F11

<400>    58
Asp Ile Val Met Thr Gln Ser Pro Ser Ser Leu Ala Val Ser Leu Gly
1               5                   10                  15

Glu Arg Val Thr Met Ser Cys
            20

<210>    59
<211>    15
<212>    PRT
<213>    Artificial Sequence

<220>
<223>    VL Framwork 2 of ch11F11

<400>    59
Trp Tyr Gln Gln Lys Pro Gly Gln Ser Pro Lys Leu Leu Ile Tyr
1               5                   10                  15

<210>    60
<211>    15
<212>    PRT
<213>    Artificial Sequence

<220>
<223>    VL Framwork 2 of  Hu11F11

<400>    60
Trp Tyr Gln Gln Lys Pro Gly Gln Pro Pro Lys Leu Leu Ile Tyr
1               5                   10                  15

<210>    61
<211>    15
<212>    PRT
<213>    Artificial Sequence

<220>
<223>    VL Framwork 2 of  Hu11F11

<400>    61
Trp Tyr Gln Gln Lys Pro Gly Lys Ala Pro Lys Leu Leu Ile Tyr
1               5                   10                  15

<210>    62
<211>    32
<212>    PRT
<213>    Artificial Sequence

<220>
<223>    VH Framwork 3 of ch11F11


<400>    62
Gly Val Pro Asp Arg Phe Thr Gly Ser Gly Ser Gly Thr Asp Phe Thr
1               5                   10                  15

Leu Thr Ile Ser Ser Val Lys Ala Glu Asp Leu Ala Val Tyr Tyr Cys
            20                  25                  30


<210>    63
<211>    32
<212>    PRT
<213>    Artificial Sequence

<220>
<223>    VL Framwork 3 of  Hu11F11


<400>    63
Gly Val Pro Asp Arg Phe Ser Gly Ser Gly Ser Gly Thr Asp Phe Thr
1               5                   10                  15

Leu Thr Ile Ser Ser Leu Gln Ala Glu Asp Val Ala Val Tyr Tyr Cys
            20                  25                  30


<210>    64
<211>    32
<212>    PRT
<213>    Artificial Sequence

<220>
<223>    VL Framwork 3 of  Hu11F11


<400>    64
Gly Val Pro Asp Arg Phe Ser Gly Ser Gly Ser Gly Thr Asp Phe Thr
1               5                   10                  15

Leu Thr Ile Ser Ser Val Gln Ala Glu Asp Val Ala Val Tyr Tyr Cys
            20                  25                  30

<210>   65
<211>   32
<212>   PRT
<213>   Artificial Sequence

<220>
<223>   VL Framwork 3 of  Hu11F11

<400>   65
Gly Val Pro Ser Arg Phe Ser Gly Ser Gly Ser Gly Thr Asp Phe Thr
 1               5                  10                  15

Leu Thr Ile Ser Ser Leu Gln Pro Glu Asp Phe Ala Thr Tyr Tyr Cys
            20                  25                  30

<210>   66
<211>   32
<212>   PRT
<213>   Artificial Sequence

<220>
<223>   VL Framwork 3 of  Hu11F11

<400>   66
Gly Val Pro Asp Arg Phe Ser Gly Ser Gly Ser Gly Thr Asp Phe Thr
 1               5                  10                  15

Leu Thr Ile Ser Ser Val Gln Ala Glu Asp Leu Ala Val Tyr Tyr Cys
            20                  25                  30

<210>   67
<211>   32
<212>   PRT
<213>   Artificial Sequence

<220>
<223>   VL Framwork 3 of  Hu11F12

<400>    67
Gly Val Pro Asp Arg Phe Thr Gly Ser Gly Ser Gly Thr Asp Phe Thr
1               5                   10                  15

Leu Thr Ile Ser Ser Val Lys Ala Glu Asp Val Ala Val Tyr Tyr Cys
            20                  25                  30

<210>    68
<211>    10
<212>    PRT
<213>    Artificial Sequence

<220>
<223>    VH Framwork 4 of ch11F11

<400>    68
Phe Gly Gly Gly Thr Lys Leu Glu Ile Lys
1               5                   10

<210>    69
<211>    10
<212>    PRT
<213>    Artificial Sequence

<220>
<223>    VL Framwork 4 of  Hu11F12

<400>    69
Phe Gly Gly Gly Thr Lys Val Glu Ile Lys
1               5                   10

<210>    70
<211>    10
<212>    PRT
<213>    Artificial Sequence

<220>
<223>    VL Framwork 4 of  Hu11F12

<400>    70
Phe Gly Gln Gly Thr Lys Val Glu Ile Lys

1                   5                   10

<210>   71
<211>   23
<212>   PRT
<213>   Artificial Sequence

<220>
<223>   VL Framwork 1 of ch3A9

<400>   71
Asp Ile Val Met Thr Gln Ser Pro Lys Phe Met Ser Thr Ser Val Gly
1               5                   10                  15

Asp Arg Val Ser Ile Thr Cys
            20

<210>   72
<211>   23
<212>   PRT
<213>   Artificial Sequence

<220>
<223>   VL Framwork 1 of Hu3A9

<400>   72
Asp Ile Gln Met Thr Gln Ser Pro Ser Ser Leu Ser Ala Ser Val Gly
1               5                   10                  15

Asp Arg Val Thr Ile Thr Cys
            20

<210>   73
<211>   23
<212>   PRT
<213>   Artificial Sequence

<220>
<223>   VL Framwork 1 of Hu3A9

<400>   73
Asp Ile Val Met Thr Gln Ser Pro Ser Thr Leu Ser Ala Ser Val Gly
1               5                   10                  15

Asp Arg Val Thr Ile Thr Cys

            20

<210>    74
<211>    23
<212>    PRT
<213>    Artificial Sequence

<220>
<223>    VL Framwork 1 of Hu3A9

<400>    74
Asp Ile Val Met Thr Gln Ser Pro Ala Thr Leu Ser Val Ser Leu Gly
 1               5                   10                  15

Glu Arg Ala Thr Leu Ser Cys
            20

<210>    75
<211>    23
<212>    PRT
<213>    Artificial Sequence

<220>
<223>    VL Framwork 1 of Hu3A9

<400>    75
Asp Ile Gln Met Thr Gln Ser Pro Ser Ser Leu Ser Ala Ser Val Gly
 1               5                   10                  15

Asp Arg Val Thr Ile Thr Cys
            20

<210>    76
<211>    23
<212>    PRT
<213>    Artificial Sequence

<220>
<223>    VL Framwork 1 of Hu3A9

<400>    76
Asp Ile Gln Met Thr Gln Ser Pro Ser Ser Leu Ser Ala Ser Val Gly
 1               5                   10                  15

Asp Arg Val Thr Ile Thr Cys

            20

<210>	77
<211>	23
<212>	PRT
<213>	Artificial Sequence

<220>
<223>	VL Framwork 1 of Hu3A9

<400>	77
Asp Ile Val Met Thr Gln Ser Pro Ser Ser Met Ser Thr Ser Val Gly
1               5                   10                  15

Asp Arg Val Thr Ile Thr Cys
            20

<210>	78
<211>	15
<212>	PRT
<213>	Artificial Sequence

<220>
<223>	VL Framwork 2 of Hu3A9

<400>	78
Trp Tyr Gln Gln Lys Pro Gly Gln Ser Pro Lys Leu Leu Ile Tyr
1               5                   10                  15

<210>	79
<211>	15
<212>	PRT
<213>	Artificial Sequence

<220>
<223>	VL Framwork 2 of Hu3A9

<400>	79
Trp Tyr Gln Gln Lys Pro Gly Lys Ala Pro Lys Leu Leu Ile Tyr
1               5                   10                  15

<210>	80
<211>	16
<212>	PRT

<213>    Artificial Sequence

<220>
<223>    VL Framwork 2 of Hu3A9

<400>    80
Ala Trp Tyr Gln Gln Lys Pro Gly Lys Ala Pro Lys Leu Leu Ile Tyr
1               5                   10                  15

<210>    81
<211>    15
<212>    PRT
<213>    Artificial Sequence

<220>
<223>    VL Framwork 2 of Hu3A9

<400>    81
Trp Tyr Gln Gln Lys Pro Gly Gln Ala Pro Arg Leu Leu Ile Tyr
1               5                   10                  15

<210>    82
<211>    32
<212>    PRT
<213>    Artificial Sequence

<220>
<223>    VL Framwork 3 of Hu3A9

<400>    82
Gly Val Pro Asp Arg Phe Thr Gly Ser Gly Ser Gly Thr Asp Phe Thr
1               5                   10                  15

Leu Thr Ile Ser Asn Met Gln Ser Glu Asp Leu Ala Asp Tyr Phe Cys
            20                  25                  30

<210>    83
<211>    32
<212>    PRT
<213>    Artificial Sequence

<220>
<223>	VL Framwork 3 of Hu3A9

<400>	83
Gly Val Pro Asp Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>	84
<211>	32
<212>	PRT
<213>	Artificial Sequence

<220>
<223>	VL Framwork 3 of Hu3A9

<400>	84
Gly Val Pro Ser Arg Phe Ser Gly Ser Gly Ser Gly Thr Glu Phe Thr
1               5                   10                  15

Leu Thr Ile Ser Ser Leu Gln Pro Asp Asp Phe Ala Ser Tyr Tyr Cys
            20                  25                  30

<210>	85
<211>	32
<212>	PRT
<213>	Artificial Sequence

<220>
<223>	VL Framwork 3 of Hu3A9

<400>	85
Gly Ile Pro Ala Arg Phe Ser Gly Ser Gly Ser Gly Thr Glu Phe Thr
1               5                   10                  15

Leu Thr Ile Ser Ser Leu Gln Ser Glu Asp Phe Ala Val Tyr Tyr Cys
            20                  25                  30

<210>    86
<211>    32
<212>    PRT
<213>    Artificial Sequence

<220>
<223>    VL Framwork 3 of Hu3A9


<400>    86
Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30




<210>    87
<211>    32
<212>    PRT
<213>    Artificial Sequence

<220>
<223>    VL Framwork 3 of Hu3A9


<400>    87
Gly Val Pro Asp Arg Phe Ser Gly Ser Gly Ser Gly Thr Asp Phe Thr
1               5                   10                  15

Phe Thr Ile Ser Ser Met Gln Ser Glu Asp Ile Ala Thr Tyr Phe Cys
            20                  25                  30




<210>    88
<211>    32
<212>    PRT
<213>    Artificial Sequence

<220>
<223>    VL Framwork 3 of Hu3A9


<400>    88
Gly Val Pro Asp Arg Phe Ser Gly Ser Gly Ser Gly Thr Asp Phe Thr
1               5                   10                  15

Leu Thr Ile Ser Ser Met Gln Ser Glu Asp Leu Ala Asp Tyr Tyr Cys
            20                  25                  30

<210> 89
<211> 10
<212> PRT
<213> Artificial Sequence

<220>
<223> VL Framwork 4 of Hu3A9

<400> 89
Phe Gly Ala Gly Thr Lys Leu Glu Leu Arg
1               5                   10

<210> 90
<211> 120
<212> PRT
<213> Artificial Sequence

<220>
<223> Heavy chain variable region of ch11F11-VH

<400> 90
Glu Val Gln Leu Gln Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
    50                  55                  60

Ser Val Lys Gly Arg Phe Ile Val Ser Arg Asp Thr Ser Gln Ser Ile
65                  70                  75                  80

Leu Tyr Leu Gln Met Asn Ala Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Leu Val Thr Val Ser Ala
        115                 120

<210>   91
<211>   120
<212>   PRT
<213>   Artificial Sequence

<220>
<223>   Heavy chain variable region of Hu11F11-VH1

<400>   91
Glu Val Gln Leu Val Glu Ser Gly Gly Gly Leu Val Gln Pro Gly Gly
  1               5                  10                  15

Ser Leu Arg Leu Ser Cys Ala Ala Ser Gly Phe Thr Phe Ser Asp Phe
             20                  25                  30

Tyr Met Glu Trp Val Arg Gln Ala Pro Gly Lys Gly Leu Glu Trp Ile
         35                  40                  45

Ala Ala Ile Arg Asn Lys Ala Asn Asp Tyr Thr Thr Glu Tyr Ala Ala
     50                  55                  60

Ser Val Lys Gly Arg Phe Thr Val Ser Arg Asp Thr Ser Lys Asn Ser
 65                  70                  75                  80

Leu Tyr Leu Gln Met Asn Ser Leu Lys Thr Glu Asp Thr Ala Val Tyr
                 85                  90                  95

Tyr Cys Ala Arg Asp Ala His Gly Lys Pro Phe Ala Tyr Trp Gly Gln
            100                 105                 110

Gly Thr Leu Val Thr Val Ser Ser
        115                 120

<210>   92
<211>   120
<212>   PRT
<213>   Artificial Sequence

<220>
<223>   Heavy chain variable region of Hu11F11-VH2

<400>   92
Glu Val Gln Leu Val Glu Ser Gly Gly Gly Leu Val Gln Pro Gly Gly

1                   5                   10                  15

Ser Leu Arg Leu Ser Cys Ala Ala Ser Gly Phe Thr Phe Ser Asp Phe
            20                  25                  30

Tyr Met Glu Trp Val Arg Gln Ala Pro Gly Lys Gly Leu Glu Trp Ile
        35                  40                  45

Ala Ala Ile Arg Asn Lys Ala Asn Asp Tyr Thr Thr Glu Tyr Ala Ala
    50                  55                  60

Ser Val Lys Gly Arg Phe Thr Ile Ser Arg Asp Thr Ser Lys Asn Ser
65                  70                  75                  80

Leu Tyr Leu Gln Met Asn Ser Leu Lys Thr Glu Asp Thr Ala Val Tyr
                85                  90                  95

Tyr Cys Ala Arg Asp Ala His Gly Lys Pro Phe Ala Tyr Trp Gly Gln
            100                 105                 110

Gly Thr Leu Val Thr Val Ser Ser
        115                 120

<210>   93
<211>   120
<212>   PRT
<213>   Artificial Sequence

<220>
<223>   Heavy chain variable region of Hu11F11-VH3

<400>   93
Glu Val Gln Leu Val Glu Ser Gly Gly Gly Leu Val Gln Pro Gly Gly
1                   5                   10                  15

Ser Leu Arg Leu Ser Cys Ala Ala Ser Gly Phe Thr Phe Ser Asp Phe
            20                  25                  30

Tyr Met Glu Trp Val Arg Gln Ala Pro Gly Lys Gly Leu Glu Trp Ile
        35                  40                  45

Ala Ala Ile Arg Asn Lys Ala Asn Asp Tyr Thr Thr Glu Tyr Ala Asp
    50                  55                  60

Ser Val Lys Gly Arg Phe Thr Val Ser Arg Asp Thr Ser Gln Asn Thr
65                  70                  75                  80

Leu Tyr Leu Gln Met Asn Ser Leu Arg Ala Glu Asp Thr Ala Val Tyr
                85                  90                  95

Tyr Cys Ala Arg Asp Ala His Gly Lys Pro Phe Ala Tyr Trp Gly Gln
            100                 105                 110

Gly Thr Leu Val Thr Val Ser Ser
        115                 120

<210>   94
<211>   120
<212>   PRT
<213>   Artificial Sequence

<220>
<223>   Heavy chain variable region of Hu11F11-VH4

<400>   94
Glu Val Gln Leu Val Glu Ser Gly Gly Gly Leu Val Gln Pro Gly Gly
 1               5                  10                  15

Ser Leu Arg Leu Ser Cys Ala Ala Ser Gly Phe Thr Phe Ser Asp Phe
            20                  25                  30

Tyr Met Glu Trp Val Arg Gln Ala Pro Gly Lys Gly Leu Glu Trp Val
        35                  40                  45

Ala Ala Ile Arg Asn Lys Ala Asn Asp Tyr Thr Thr Glu Tyr Ala Asp
    50                  55                  60

Ser Val Lys Gly Arg Phe Thr Ile Ser Ala Asp Thr Ser Lys Asn Thr
65                  70                  75                  80

Ala Tyr Leu Gln Met Asn Ser Leu Arg Ala Glu Asp Thr Ala Val Tyr
                85                  90                  95

Tyr Cys Ser Arg Asp Ala His Gly Lys Pro Phe Ala Tyr Trp Gly Gln
            100                 105                 110

Gly Thr Leu Val Thr Val Ser Ser
        115                 120

<210>   95
<211>   120
<212>   PRT
<213>   Artificial Sequence

<220>
<223>   Heavy chain variable region of Hu11F11-VHv3

<400>    95
Glu Val Gln Leu Val Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Thr Arg Asn Lys Ala Asn Asp Tyr Thr Thr Glu Tyr Ser Ala
    50                  55                  60

Ser Val Lys Gly Arg Phe Thr Ile Ser Arg Asp Asp Ser Lys Ser Ser
65                  70                  75                  80

Leu Tyr Leu Gln Met Asn Ser Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Thr Val Thr Val Ser Ser
        115                 120


<210>    96
<211>    120
<212>    PRT
<213>    Artificial Sequence

<220>
<223>    Heavy chain variable region of Hu11F11-VHv1mu1 newmu


<400>    96
Glu Val Gln Leu Val Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Thr Arg Asn Lys Ala Asn Asp Tyr Thr Thr Glu Tyr Ser Ala
    50                  55                  60

Ser Val Lys Gly Arg Phe Thr Ile Ser Arg Asp Thr Ser Gln Ser Ser
65                  70                  75                  80

Leu Tyr Leu Gln Met Asn Ser Leu Lys Thr Glu Asp Thr Ala Val Tyr
                85                  90                  95

Tyr Cys Ala Arg Asp Ala His Gly Lys Pro Phe Ala Tyr Trp Gly Gln
            100                 105                 110

Gly Thr Thr Val Thr Val Ser Ser
        115                 120


<210>   97
<211>   120
<212>   PRT
<213>   Artificial Sequence

<220>
<223>   Heavy chain variable region of Hu11F11-VHv3 newmu


<400>   97
Glu Val Gln Leu Val Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Thr Arg Asn Lys Ala Asn Asp Tyr Thr Thr Glu Tyr Ser Ala
    50                  55                  60

Ser Val Lys Gly Arg Phe Thr Ile Ser Arg Asp Thr Ser Gln Ser Ser
65                  70                  75                  80

Leu Tyr Leu Gln Met Asn Ser Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Thr Val Thr Val Ser Ser
        115                 120


<210>   98
<211>   120
<212>   PRT
<213>   Artificial Sequence

<220>
<223>    Heavy chain variable region of Hu11F11-VH-v1

<400>    98
Glu Val Gln Leu Val Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
    50                  55                  60

Ser Val Lys Gly Arg Phe Thr Ile Ser Arg Asp Asp Ser Lys Ser Ser
65                  70                  75                  80

Leu Tyr Leu Gln Met Asn Ser Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Thr Val Thr Val Ser Ser
        115                 120

<210>    99
<211>    120
<212>    PRT
<213>    Artificial Sequence

<220>
<223>    Heavy chain variable region of Hu11F11-VH-v2

<400>    99
Glu Val Gln Leu Val Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
    50                  55                  60

Ser Val Lys Gly Arg Phe Thr Val Ser Arg Asp Asp Ser Lys Ser Ser
65                  70                  75                  80

Leu Tyr Leu Gln Met Asn Ser Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Thr Val Thr Val Ser Ser
        115                 120


<210>   100
<211>   120
<212>   PRT
<213>   Artificial Sequence

<220>
<223>   Heavy chain variable region of Hu11F11-VH-v3


<400>   100
Glu Val Gln Leu Val Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
    50                  55                  60

Ser Val Lys Gly Arg Phe Thr Ile Ser Arg Asp Thr Ser Lys Ser Ser
65                  70                  75                  80

Leu Tyr Leu Gln Met Asn Ser Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Thr Val Thr Val Ser Ser
        115                 120


<210>   101
<211>   120

<212>	PRT
<213>	Artificial Sequence

<220>
<223>	Heavy chain variable region of Hu11F11-VH-v4


<400>	101
Glu Val Gln Leu Val Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
    50                  55                  60

Ser Val Lys Gly Arg Phe Thr Val Ser Arg Asp Thr Ser Lys Ser Ser
65                  70                  75                  80

Leu Tyr Leu Gln Met Asn Ser Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Thr Val Thr Val Ser Ser
        115                 120


<210>	102
<211>	122
<212>	PRT
<213>	Artificial Sequence

<220>
<223>	Heavy chain variable region of ch3A9-VH


<400>	102
Glu Val Gln Leu Gln Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Thr Pro Glu Lys Arg Leu Glu Trp Val
        35                  40                  45

Ala Thr Ile Ser Asn Gly Gly Gly Tyr Thr Tyr Tyr Pro Asp Ser Val
50                  55                  60

Lys Gly Arg Phe Thr Ile Ser Arg Asp Asn Ala Lys Asn Thr Leu Tyr
65                  70                  75                  80

Leu Gln Met Ser Ser Leu Arg Ser Glu Asp Thr Ala Met Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Thr Leu Thr Val Ser Ser
        115                 120

<210>   103
<211>   122
<212>   PRT
<213>   Artificial Sequence

<220>
<223>   Heavy chain variable region of Hu3A9-VH1

<400>   103
Gln Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ala Thr Ile Ser Asn Gly Gly Gly Tyr Thr Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Ser Glu Asp Ser Ala Met Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Leu Val Thr Val Ser Ser
        115                 120

<210>    104
<211>    122
<212>    PRT
<213>    Artificial Sequence

<220>
<223>    Heavy chain variable region of Hu3A9-VH2


<400>    104
Glu Val Gln Leu Val Gln Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Asp Lys Gly Leu Glu Trp Val
        35                  40                  45

Ala Thr Ile Ser Asn Gly Gly Gly Tyr Thr Tyr Tyr Ala Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ala Glu Asp Ser Ala Val Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Leu Val Thr Val Ser Ser
        115                 120


<210>    105
<211>    122
<212>    PRT
<213>    Artificial Sequence

<220>
<223>    Heavy chain variable region of Hu3A9-VH3


<400>    105
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ala Thr Ile Ser Asn Gly Gly Gly Tyr Thr Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Leu Val Thr Val Ser Ser
        115                 120

<210> 106
<211> 122
<212> PRT
<213> Artificial Sequence

<220>
<223> Heavy chain variable region of Hu3A9-VH4

<400> 106
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ala Thr Ile Ser Asn Gly Gly Gly Tyr Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Leu Val Thr Val Ser Ser
        115                 120

<210>    107
<211>    122
<212>    PRT
<213>    Artificial Sequence

<220>
<223>    Heavy chain variable region of Hu3A9-VH-v1

<400>    107
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Thr Pro Glu Lys Gly Leu Glu Trp Val
        35                  40                  45

Ala Thr Ile Ser Asn Gly Gly Gly Tyr Thr Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Ser Ser Leu Arg Ala Glu Asp Thr Ala Met Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Thr Leu Thr Val Ser Ser
        115                 120

<210>    108
<211>    122
<212>    PRT
<213>    Artificial Sequence

<220>
<223>    Heavy chain variable region of Hu3A9-VH-v2

<400>    108
Glu Val Gln Leu Leu Glu Ser Gly Gly Gly Leu Val Gln Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Thr Pro Glu Lys Gly Leu Glu Trp Val
        35                  40                  45

Ala Thr Ile Ser Asn Gly Gly Gly Tyr Thr Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Ser Ser Leu Arg Ala Glu Asp Thr Ala Met Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Thr Leu Thr Val Ser Ser
        115                 120

<210> 109
<211> 113
<212> PRT
<213> Artificial Sequence

<220>
<223> Light chain variable region of ch11F11-VL

<400> 109
Asp Ile Val Met Thr Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Ser Tyr Pro Trp Thr Phe Gly Gly Gly Thr Lys Leu Glu Ile
            100                 105                 110

Lys

<210> 110
<211> 113
<212> PRT
<213> Artificial Sequence

<220>
<223> Light chain variable region of Hu11F11-VL1

<400> 110
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Tyr Pro Trp Thr Phe Gly Gly Gly Thr Lys Val Glu Ile
            100                 105                 110

Lys

<210> 111
<211> 113
<212> PRT
<213> Artificial Sequence

<220>
<223> Light chain variable region of Hu11F11-VL2

<400>    111
Asp Ile Val Met Thr Gln Ser Pro Ser Ser Leu Ala Val Ser Leu Gly
1               5                   10                  15

Glu Arg Val Thr Ile Thr Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95

Tyr Tyr Ser Tyr Pro Trp Thr Phe Gly Gly Gly Thr Lys Leu Glu Ile
            100                 105                 110

Lys

<210>    112
<211>    113
<212>    PRT
<213>    Artificial Sequence

<220>
<223>    Light chain variable region of u11F11-VL3

<400>    112
Asp Ile Val Met Thr Gln Ser Pro Ser Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Tyr Pro Trp Thr Phe Gly Gly Gly Thr Lys Leu Glu Ile
            100                 105                 110

Lys

<210>    113
<211>    113
<212>    PRT
<213>    Artificial Sequence

<220>
<223>    Light chain variable region of Hu11F11-VL4

<400>    113
Asp Ile Gln Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Tyr Ser
            20                  25                  30

Ser Asn Gln Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Gln Gln
                85                  90                  95

Tyr Tyr Ser Tyr Pro Trp Thr Phe Gly Gln Gly Thr Lys Val Glu Ile
            100                 105                 110

Lys

<210>    114
<211>    113
<212>    PRT
<213>    Artificial Sequence

<220>

<223>     Light chain variable region of Hu11F11-VL5

<400>     114
Asp Ile Val Met Thr Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Gln
                85                  90                  95

Tyr Tyr Ser Tyr Pro Trp Thr Phe Gly Gly Gly Thr Lys Leu Glu Ile
            100                 105                 110

Lys

<210>     115
<211>     113
<212>     PRT
<213>     Artificial Sequence

<220>
<223>     Light chain variable region of Hu11F11-VLv3 4c

<400>     115
Asp Ile Val Met Thr Gln Ser Pro Ser Ser Leu Ala Val Ser Leu Gly
1               5                   10                  15

Glu Arg Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Val Ala Val Tyr Tyr Cys Gln Gln
                85                  90                  95

Tyr Tyr Ser Tyr Pro Trp Thr Phe Gly Gly Gly Thr Lys Leu Glu Ile
            100                 105                 110

Lys

<210>    116
<211>    107
<212>    PRT
<213>    Artificial Sequence

<220>
<223>    Light chain variable region of ch3A9-VL

<400>    116
Asp Ile Val Met Thr Gln Ser Pro Lys Phe Met Ser Thr Ser Val Gly
1               5                   10                  15

Asp Arg Val Ser Ile Thr Cys Lys Ala Ser Gln Asn Val Gly Thr Thr
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Asn Tyr Pro Leu
                85                  90                  95

Thr Phe Gly Ala Gly Thr Lys Leu Glu Leu Arg
            100                 105

<210>    117
<211>    107
<212>    PRT
<213>    Artificial Sequence

<220>

<223>    Light chain variable region of Hu3A9-VL1

<400>    117
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Thr
            20                  25                  30

Val Ala Trp Tyr Gln Gln Lys Pro Gly Lys Ala Pro Lys Leu Leu Ile
        35                  40                  45

Tyr Ser Ala Ser Asn Arg Tyr Thr Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Asn Tyr Pro Leu
                85                  90                  95

Thr Phe Gly Gly Gly Thr Lys Leu Glu Ile Lys
            100                 105

<210>    118
<211>    107
<212>    PRT
<213>    Artificial Sequence

<220>
<223>    Light chain variable region of Hu3A9-VL2

<400>    118
Asp Ile Val Met Thr Gln Ser Pro Ser Thr Leu Ser Ala Ser Val Gly
1               5                   10                  15

Asp Arg Val Thr Ile Thr Cys Lys Ala Ser Gln Asn Val Gly Thr Thr
            20                  25                  30

Val Ala Trp Tyr Gln Gln Lys Pro Gly Lys Ala Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Ser Tyr Tyr Cys Gln Gln Tyr Ser Asn Tyr Pro Leu
                85                  90                  95

Thr Phe Gly Gln Gly Thr Lys Val Glu Ile Lys
            100                 105

<210>   119
<211>   107
<212>   PRT
<213>   Artificial Sequence

<220>
<223>   Light chain variable region of Hu3A9-VL3

<400>   119
Asp Ile Val Met Thr Gln Ser Pro Ala Thr Leu Ser Val Ser Leu Gly
1               5                   10                  15

Glu Arg Ala Thr Leu Ser Cys Lys Ala Ser Gln Asn Val Gly Thr Thr
            20                  25                  30

Val Ala Trp Tyr Gln Gln Lys Pro Gly Gln Ala Pro Arg Leu Leu Ile
        35                  40                  45

Tyr Ser Ala Ser Asn Arg Tyr Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Ser Asn Tyr Pro Leu
                85                  90                  95

Thr Phe Gly Gly Gly Thr Lys Val Glu Ile Lys
            100                 105

<210>   120
<211>   107
<212>   PRT
<213>   Artificial Sequence

<220>
<223>   Light chain variable region of Hu3A9-VL4

<400>   120
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Thr
            20                  25                  30

Val Ala Trp Tyr Gln Gln Lys Pro Gly Lys Ala Pro Lys Leu Leu Ile
        35                  40                  45

Tyr Ser Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Asn Tyr Pro Leu
                85                  90                  95

Thr Phe Gly Gln Gly Thr Lys Val Glu Ile Lys
            100                 105

<210>    121
<211>    107
<212>    PRT
<213>    Artificial Sequence

<220>
<223>    Light chain variable region of Hu3A9-VL-v1

<400>    121
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Gly Thr Thr
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Ser Gly
    50                  55                  60

Ser Gly Ser Gly Thr Asp Phe Thr Phe Thr Ile Ser Ser Met Gln Ser
65                  70                  75                  80

Glu Asp Ile Ala Thr Tyr Phe Cys Gln Gln Tyr Ser Asn Tyr Pro Leu
                85                  90                  95

Thr Phe Gly Gln Gly Thr Lys Leu Glu Ile Lys
            100                 105

<210>    122
<211>    107
<212>    PRT
<213>    Artificial Sequence

<220>
<223>    Light chain variable region of Hu3A9-VL-v2


<400>    122
Asp Ile Val Met Thr Gln Ser Pro Ser Ser Met Ser Thr Ser Val Gly
1               5                   10                  15

Asp Arg Val Thr Ile Thr Cys Lys Ala Ser Gln Asn Val Gly Thr Thr
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Ser Gly
    50                  55                  60

Ser Gly Ser Gly Thr Asp Phe Thr Leu Thr Ile Ser Ser Met Gln Ser,
65                  70                  75                  80

Glu Asp Ile Ala Asp Tyr Tyr Cys Gln Gln Tyr Ser Asn Tyr Pro Leu
                85                  90                  95

Thr Phe Gly Gln Gly Thr Lys Leu Glu Ile Lys
            100                 105


<210>    123
<211>    107
<212>    PRT
<213>    Artificial Sequence

<220>
<223>    Light chain variable region of Hu3A9-VL-v2


<400>    123
Asp Ile Val Met Thr Gln Ser Pro Ser Ser Met Ser Thr Ser Val Gly
1               5                   10                  15

Asp Arg Val Thr Ile Thr Cys Lys Ala Ser Gln Asn Val Gly Thr Thr
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Ser Gly
    50                  55                  60

Ser Gly Ser Gly Thr Asp Phe Thr Leu Thr Ile Ser Ser Met Gln Ser
65                  70                  75                  80

Glu Asp Leu Ala Asp Tyr Tyr Cys Gln Gln Tyr Ser Asn Tyr Pro Leu
                85                  90                  95

Thr Phe Gly Gln Gly Thr Lys Leu Glu Ile Lys
            100                 105

<210> 124
<211> 116
<212> PRT
<213> Artificial Sequence

<220>
<223> ch9B11-VH

<400> 124
Glu Val Gln Leu Gln Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Gln Asp Phe Asp Tyr Trp Gly Gln Gly Thr Thr Leu
            100                 105                 110

Thr Val Ser Ser
        115

<210> 125
<211> 113

<212>    PRT
<213>    Artificial Sequence

<220>
<223>    ch9B11-VL


<400>    125
Asp Ile Val Met Thr Gln Ser Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Leu Thr Phe Gly Ala Gly Thr Lys Leu Glu Gln Lys
            100                 105                 110

Arg




<210>    126
<211>    140
<212>    PRT
<213>    Artificial Sequence

<220>
<223>    human alpha-synuclein


<400>    126

## Description

### [TECHNICAL FIELD]

The present invention relates to an antibody to alpha-synuclein and use thereof.

### [RELATED ART]

Alpha-synuclein (a-Synuclein, α-syn) is expressed primarily in the presynaptic terminals of neurons, and is a naturally unfolded monomer in normal conditions. Alpha-synuclein helps to regulate the release of dopamine that is a neurotransmitter controlling voluntary or involuntary movements. Particularly, the function of alpha-synuclein is important with increased synaptic activity and aging, and is an important factor of neurodegeneration.

However, in the pathological state, alpha-synuclein undergoes structural changes through binding and interaction with droplets, phospholipid bilayers, or lipid membranes to form a folded or folded α-helical secondary structure, thereby dividing the amount. Agglomerates comprising molecules in the form of dimers, oligomers and / or fibers are formed.

These alpha-synuclein aggregates have been known to induce toxicity to cells, and are the major component of an abnormal protein aggregate of Lewy bodies that are found in neurons of Parkinson's disease (PD), Parkinson's disease dementia (PDD), multiple system atrophy (MSA), Dementia with Lewy bodies (DLB), and various diseases. It is also known that post-translational modifications of alpha-synuclein, such as phosphorylation, or ubiquitination, are also associated with aggregation and neurotoxicity of alpha-synuclein. Alpha-synuclein has been known to kill dopamine neurons and cause inflammatory reactions in animal experiments and cell experiments, and to cause motor symptoms similar to Parkinson's disease in experimental animals. In addition, alpha-synuclein aggregation has been known to be related to an etiology of a group of neurodegenerative diseases called α-synucleinopathies, including Parkinson's disease, Parkinson's disease dementia, Lewy body dementia, multiple system atrophy and many other neuro-axonal diseases.

Furthermore, both oligomeric and monomeric forms of alpha-synuclein were found in cerebrospinal fluid and plasma samples of patients with Parkinson's disease, indicating that alpha-synuclein aggregates with small molecular weight can penetrate the cell membrane and access the extracellular space. It has also been shown that folded alpha-synuclein can be released from cells by exocytosis and then transferred from one region of the brain to another region by intercellular transfer like prion proteins.

Therefore, alpha-synuclein is a target for the development of therapeutic agents for α-synucleinopathy such as Parkinson's disease. The main development strategies include inhibition of aggregate formation, gene silencing, and removal of aggregates. In the former case, Epigallocatechin-3-gallate (EGCG), 3-(1,3-benzodioxol- - bromophenyl)-1H-pyrazole (anle138b), CLR0120 and KYP-20479 of prolyl oligopeptidase inhibitor are included.

The compounds with a low molecular weight should be administered at a high dose, due to low target specific binding capacity and short half-life. Compared to the compounds with low molecular weight, the antibody has a target specificity and long half-life. However, in order to increase the therapeutic potential of the disease, antibodies preferentially binding to the aggregates with high affinity are needed. Therefore, for the treatment of diseases associated with the abnormal accumulation of alpha-synuclein aggregates, the development of antibodies being capable of preferentially binding to alpha-synuclein, particularly alpha-synuclein aggregates is needed.

### [DISCLOSURE]

### [TECHNICAL PROBLEM]

In the present specification, it is to provide an antibody or antigen-binding fragment thereof being capable of specifically binding to alpha-synuclein, particularly alpha-synuclein aggregate. The antibody or an antigen-binding fragment thereof against alpha-synuclein can specifically recognize and bind to alpha-synuclein, and in particular, bind to the C-terminal region of alpha-synuclein.

An embodiment of the present invention provides the antibody that specifically binds to synuclein or alpha-synuclein aggregate and reduce the level of alpha-synuclein in nerve system of a subject. More specifically, the antibody or antigen-binding fragment thereof of the present invention has a high binding affinity to alpha-synuclein aggregate, particularly amyloid fibrils, protofibrils and oligomers, or especially amyloid fibrils, but has low binding affinity to alpha-synuclein monomer.

The antibody or antigen-binding fragment thereof of the present invention has at least an property selected from the group consisting of (i) specific binding to alpha-synuclein or alpha-synuclein aggregate, (ii) reduction of level of alpha-synuclein or alpha-synuclein aggregate in nerve system of a subject, (iii) reduction or inhibition of cell-to-cell transmission of alpha-synuclein or alpha-synuclein aggregate between the nerve cells, and (vi) improvement of phagocytic uptake of a nerve cell for alpha-synuclein or alpha-synuclein aggregate. Preferably, the antibody or antigen-binding fragment thereof of the present invention can have all the properties of (i) to (iv).

An embodiment of the present invention relates to a composition for prevention, alleviation, improvement or treatment of synucleinopathies, comprising an antibody or an antigen-binding fragment thereof being capable of specifically binding to alpha-synuclein, especially alpha-synuclein aggregate.

An embodiment of the present invention relates to a method of prevention, alleviation, improvement or treatment of synucleinopathies, comprising a step of administering to a subject in need, an antibody or an antigen-binding fragment thereof being capable of specifically binding to alpha-synuclein, especially alpha-synuclein aggregate. The administered amount of the antibody or antigen-binding fragment thereof can be an effective amount to reduce the level of alpha-synuclein, especially alpha-synuclein aggregate in nerve system, more specifically extracellular region of the nerve cell in the nerve system of a subject.

An embodiment of the present invention relates to a method of reducing the level of alpha-synuclein, especially alpha-synuclein aggregate in nerve system, more specifically extracellular region of the nerve cell in the nerve system of a subject, or an use of reducing the level of alpha-synuclein, especially alpha-synuclein aggregate in an extracellular region of the nerve cell in the nerve system, comprising an antibody or an antigen-binding fragment thereof being capable of specifically binding to alpha-synuclein, especially alpha-synuclein aggregate.

An embodiment of the present invention relates to a method of reducing or inhibiting cell-to-cell transmission of alpha-synuclein or alpha-synuclein aggregate in the nerve system, especially between the nerve cells of a subject, or an use of reducing or inhibiting cell-to-cell transmission of alpha-synuclein or alpha-synuclein aggregate between the nerve cells in the nerve system, comprising an antibody or an antigen-binding fragment thereof being capable of specifically binding to alpha-synuclein, especially alpha-synuclein aggregate.

An embodiment of the present invention relates to a method of increasing the phagocytic uptake of microglia in nerve system to the alpha-synuclein or alpha-synuclein aggregate, or an use of increasing the phagocytic uptake of microglia in nerve system to the alpha-synuclein or alpha-synuclein aggregate, comprising an antibody or an antigen-binding fragment thereof being capable of specifically binding to alpha-synuclein, especially alpha-synuclein aggregate.

### [TECHNICAL SOLUTION]

Hereinafter, the present invention will be described in more detail.

The present invention provides an antibody or an antigen-binding fragment thereof being capable of specifically binding to alpha-synuclein, especially alpha-synuclein aggregate. The antibody or an antigen-binding fragment thereof against alpha-synuclein can specifically recognizes and binds to alpha-synuclein without binding to beta(β)-synuclein or gamma(y)-synuclein, and specifically recognizes and binds to alpha-synuclein aggregate, without binding to amyloid beta aggregate and tau aggregate.

Herein, the term "antigen" or "immunogen" means a molecule or a part of molecule which for example, can be bound by an antigen-binding protein (for example, antibody or its immunologically-functional antigen-binding fragment) and can be used for production of an antibody due to the binding property to an antigen in an animal. The antigen may include one or more of epitopes which can interact with a different antibody or its fragment. In one embodiment according to the present invention, the antigen can be alpha-synuclein protein or alpha-synuclein protein aggregate. Specifically, the alpha-synuclein protein aggregate can be amyloid fibrils, protofibrils and oligomers, especially amyloid firbrils. The antibody or an antigen-binding fragment thereof being capable of specifically binding to alpha-synuclein, or its aggregate according to the present invention can be especially that specifically binding to C-terminal region of alpha-synuclein. The alpha-synuclein antibodies recognizing N-terminus cannot recognize aggregates of other diseases such as multiple system atrophy belonging to synucleinopathies, which collectively refer to alpha-synuclein-related diseases. In contrast, the alpha-synuclein antibodies that recognize C-terminal region have an advantage in recognizing aggregates of other various synucleinopathies as well as Parkinson's disease.

Herein, the term "neutralization" refers to inhibition or reduction of the biological activity of an antigen, when a binding protein specifically binds to the antigen. In an embodiment, the neutralizing protein or neutralizing antibody can bind to an antigen (alpha-synuclein or alpha-synuclein aggregate), and thus, reduce the level of the antigen by at least about 20%, 30%, 40%, 50%, 60%, 70%, 80%, 85%, 90%, 95%, 96%, 97%, 98%, 99%, or 100% in the extracellular level of nerve cells in the nerve system of a subject, or prevent the level of antigen from increasing in the extracellular region.

Herein, "alpha-synuclein" is native alpha-synuclein (NCBI ID: NP_000336) consisting of 140 amino acids encoded by the SNCA gene, and includes processed various forms as well as unprocessed protein with full length. In addition, the alpha-synuclein includes naturally-occurring variants of alpha-synuclein such as mutants, splice variants or allelic variants. The variants are 126 and 112 residue protein forms in which exon 3 and exon 5 are deleted (CAG3339.1), as well as 140 residue protein. Specific polymorphism or missense mutations of the SNCA gene are also known to be associated with the occurrence of Parkinson's disease, and such variants are also included in the alpha-synuclein. Beta--synuclein and gamma-synuclein of the homologues to alpha-synuclein are included. In one embodiment, the antibody according to the present invention specifically recognizes alpha-synuclein. The amino acid sequence of alpha-synuclein is shown in SEQ ID NO: 225.

Herein, "alpha-synuclein aggregate" forms due to the conformational change of alpha-synuclein, and includes an oligomer, a protofibril, a fibril and/or the structure or aggregate including at least one selected thereof.

The alpha-synuclein which can be recognized by the antibody provided herein, can be selected from the mammal alpha-synucleins of human alpha-synuclein, monkey alpha-synuclein (e.g. Rhesus alpha-synuclein), mouse alpha-synuclein, rat alpha-synuclein, and the like. For example, the human alpha-synuclein can be alpha-synuclein (NCBI ID: NP_000336), but not limited thereto. Unless otherwise stated herein, the alpha-synuclein may refer to human alpha-synuclein, and the antibodies or antigen-binding fragments provided herein have a specific binding property to not only human alpha-synuclein, but also monkey (e.g., Rhesus) alpha-synuclein, rat alpha-synuclein, and/or mouse alpha-synuclein.

The antibody or antigen binding fragment thereof can bind to C-terminal region of alpha-synuclein, specifically C-terminal region including the peptide comprised of at least 11 or 12 consecutive amino acids including 110 to 120 residues or 111 to 122 residues in SEQ ID NO: 126 of human alpha-synuclein. It has been confirmed that the antibody or antigen binding fragment of present invention can recognize the antigen recognition region and bind to the alpha-synuclein aggregate with a high binding affinity.

Herein, "affinity" or "affinity degree is the strength of interaction between an antibody or its antigen-binding fragment and an antigen, and can be determined by properties of the antigen such as size, shape and/or charge of antigen, and CDR sequences and/or physiochemical properties (hydrophilic/hydrophobic properties, electrostatic property and etc.) of the antibody or antigen-binding fragment. The methods for determining the affinity are known in the art, and generally indicated as dissociation constant (KD), but not limited thereto.

Herein, "specifically binding to alpha-synuclein or alpha-synuclein aggregate" means that the binding affinity to alpha-synuclein protein or alpha-synuclein aggregate is relatively high compared to other antigens, and for example, may be the affinity of 0.1 x 10⁻¹⁰ M to 2 x 10⁻¹⁰ M, or 0.05 x 10⁻¹⁰ M to 0.3x10⁻⁹ M to alpha-synuclein aggregates, specifically amyloid fibrils, protofibrils and oligomers, particularly amyloid fibrils, as measured by the Octet analysis or the SPR analysis, but not limited thereto.

The humanized alpha-synuclein antibodies including light chain and heavy chain according to an embodiment of the present invention, for examples, Hu11F11_ (ver.1), Hu11F11 (ver.2), and Hu11F11_ABL2-4, exhibit a high activity to promote phagocytic uptake compared to the chimeric alpha-synuclein antibodies. Compared to the chimeric alpha-synuclein antibody, Hu11F11 (ver.1), Hu11F11 (ver.2), Hu11F11 (ver.3), Hu11F11 (ver.4), and ABL2-4 shows high activity of inhibiting the binding of fibril to nerve cell membrane compared to the chimeric alpha-synuclein antibody. Hu11F11 (ver.2), Hu11F11 (ver.4) and ABL2-4 have high activity of inhibiting the propagation of alpha-synuclein secreted from the cells overexpressing alpha-synuclein to other nerve cells compared to the chimeric alpha-synuclein antibody, and show the binding affinity to the alpha-synuclein aggregate, for example, which is has a similar or superior activity to the chimeric alpha-synuclein antibody in a cell-based assay.

The alpha-synuclein antibody according to the present invention can inhibit the function of alpha-synuclein aggregates secreted out of nerve cell in the nervous system of a subject to transfers to other normal cells in an extracellular space and to infect the nerve cells (inhibit cell-to-cell transmission of aggregates), and have an ability of promoting the phagocytic action of microglia to alpha-synuclein aggregates in the extracellular space. The alpha-synuclein aggregates propagates from one cell to other cell like prions, and the alpha-synuclein, especially alpha-synuclein aggregates spread throughout the brain, resulting in synucleinopathies in normal cells. Therefore, alpha-synuclein aggregates are toxic to brain neurons and are well known to cause brain neuron death (neurodegeneration) and neuro-inflammation. Accordingly, as alpha-synuclein aggregates spread to various parts of the brain, the brain cell death and the neuro-inflammation reactions increase and results in occurrence of the brain cell death and the resulting behavior and cognitive impairments which are found with the progression of synucleinopathies such as Parkinson's disease.

Accordingly, the alpha-synuclein antibody of the present invention can prevent the spreading phenomenon of alpha-synuclein aggregates to various regions of the brain by inhibiting the transmission of the alpha-synuclein or alpha-synuclein aggregate between the nerve cells, and reduce the level of the alpha-synuclein aggregatesr which is an important cause of synucleinopathies by reducing or eliminating the alpha-synuclein aggregates themselves in extracellular region of the nerve cells of subject nerve system with the promotion of the microglia phagocytosis of, resulting in reduction of brain nerve cell death and the neuro-inflammatory reaction and further being expected to improve, alleviate or prevent the symptoms and the progress of synucleinopathies such as Parkinson's disease.

In addition, the alpha-synuclein antibody according to the present invention has excellent activities of performing both of two functions of (i) inhibition of the transmission of the alpha-synuclein or alpha-synuclein aggregate between the nerve cells (see the result of cell-based assay disclosed herein), and (ii) reduction of alpha-synuclein aggregates level in the brain nervous system through the promoted phagocytosis of microglial cells. In particular, since the alpha-synuclein antibodies which have been currently in clinical trials or published in the scientific paper have one of the two activities (i) and (ii), it suggests that the alpha-synuclein antibody of the present invention have an advantage in superior prevention or treatment of synucleinopathies to the the known alpha-synuclein antibodies. Therefore, the alpha-synuclein antibody according to the present invention have more excellent efficacies of reduction and elimination of alpha-synuclein aggregates and inhibition of the action of alpha-synuclein aggregates as etiology, and thus is more effective for synucleinopathies or a symptomatic disease related thereto (e.g., cognitive impairment disorder).

The antibodies or antigen-binding fragments according to the present invention having a high affinity for alpha-synuclein aggregates can reduce the formation of alpha-synuclein aggregate, thereby lowering the concentration of aggregates in the brain. In addition, the antibody or antigen-binding fragment according to the present invention with a high affinity for alpha-synuclein aggregates can reduce the formation of alpha-synuclein aggregates outside the central nervous system and finally, change the equilibrium state between the alpha-synuclein forms bounded by of BBB, thereby bringing the effect of lowering the concentration of alpha-synuclein aggregates inside the central nervous system.

This has a great advantage in clinical practice, because the efficacy can be sufficiently obtained even by administering in more convenient route, for example subcutaneous injection, but not limited thereto. Furthermore, because the antibodies or antigen-binding fragments according to the present invention having high affinity for alpha-synuclein aggregates can inhibit and/or reduce formation, accumulation and/or intercellular transmission of alpha-synuclein aggregates, it can lower the concentration of alpha-synuclein aggregates in the brain. In addition, the antibody or antigen-binding fragment according to the present invention with a high affinity for alpha-synuclein aggregates can reduce the formation of alpha-synuclein aggregates outside the central nervous system and thus, changing the equilibrium state between the alpha-synuclein forms bounded by of BBB. It can bring the effect of lowering the concentration of alpha-synuclein aggregates inside the central nervous system. In addition, the antibody or antigen-binding fragment according to the present application can inhibit formation of aggregates by removing monomers, or eliminates both monomers and aggregates, but not limited to this theory.

The antibodies or antigen-binding fragments thereof of the present invention that specifically bind to alpha-synuclein proteins or alpha-synuclein aggregates may not be naturally-occurring product (it can be non-naturally occurring product, for example, by chemical synthesis or recombinant method). The recombination techniques are well known in the art.

Herein, "antibody" means a complete immunoglobulin in any isotype, or an antigen-binding fragment which can compete with a complete antibody for binding to a target antigen. For example, it includes chimeric, humanized, complete human or dual-specific antibodies, or their antigen-binding fragments. The antibody is one kind of antigen binding proteins by itself. Generally, the complete antibody comprises at least 2 full-length heavy chains and 2 full-length light chains, but in some cases, the antibody may comprise only heavy chains.

The antibody or its antigen-binding fragment may be derived from only one source or be chimeric antibody. The chimeric antibody comprises a part derived from two kinds of different antibodies, and is described in more detail below. The antibody or its antigen-binding fragment can be produced by hybridoma, recombinant DNA technique, or enzymatic or chemical cutting of an intact antibody. Unless otherwise stated herein, the term of antibody includes antibodies comprising 2 full-length heavy chains and 2 full-length light chains, and their derivatives, variants, fragments, and mutants, and the examples thereof are as described below.

In one embodiment, the antibody includes a monoclonal antibody, bispecific antibody, minibody, domain antibody, antibody mimetic (or synthetic antibody), chimeric antibody, humanized antibody, human antibody, antibody fusion (or antibody conjugate), and the fragments thereof, and various types of antibodies disclosed herein without limited thereto. In one embodiment, the fragments of the antibodies disclosed herein can be Fab fragments, Fab' fragments, F(ab')₂ fragments, Fv fragments, single-chain fragments (scFv), diabody or a single chain antibody molecule of a single chain prepared by linking a heavy chain variable region and a light chain variable region through spacer.

Herein, "light chain" includes a full-length light chain and its fragments having a variable region sequence to sufficiently provide binding specificity to an antigen or epitope. The full-length light chain comprises a variable region domain VL and a constant region domain CL. The variable region domain of light chain is present in an N terminus of a light chain polypeptide. The kinds of light chain include kappa and lambda chains.

Herein, "heavy chain" includes a full-length heavy chain and its fragments having a variable region sequence to sufficiently provide binding specificity to an antigen or epitope. The full-length heavy chain comprises a variable region domain VH and three (3) constant region domains of CHI, CH2 and CH3. The VH domain is present in N-terminus of a heavy chain polypeptide and the CH domain is present in a carboxy-terminus, and CH3 is positioned closest to C-terminus. The heavy chain includes IgG (including IgG1, IgG2a, IgG2b, IgG3 and IgG4 subtypes), IgA (including IgA1 and IgA2 subtypes), and isotypes of IgM and IgE.

Herein, "antigen-binding fragments" means a part of antibody having specific binding affinity to an antigen, or and a polypeptide including the part. For example, the antigen-binding fragment may be a part of antibody including an amino acid residue that confers specificity and/or affinity for the antigen to the antibody by interacting with an antigen (e.g., an epitope), or a polypeptide including the part. This antigen-binding fragment typically comprises one or more "complementary determining regions (CDR)," and further one or more "framework (FR)" regions. CDRs are amino acid sequences that contribute to the specificity and affinity of antibody for binding to antigen, and the framework regions are the amino acid sequences contributing to the maintenance of an appropriate conformation of these CDRs and promotes the binding between the antigen-binding region and an antigen.

As used herein, "antigen-binding fragment" of a chain (heavy chain or light chain) of an antibody or immunoglobulin includes a part of an antibody which lacks some amino acids compared to a full-length chain, but can specifically bind to an antigen. This fragment can be considered as having biological activity, in an aspect that it can specifically bind to a target antigen, or can compete with other antibodies or an antigen-binding fragment to bind a specific epitope. In one aspect, this fragment comprises at least one CDR present in a full-length light chain or heavy chain, and in some embodiments, it comprises a single chain of heavy chain and/or light chain, or its part. This biologically-active fragment may be produced by a recombinant DNA technique or may be produced for example, by cutting an intact antibody enzymatically or chemically. An immunologically-functional immunoglobulin fragment includes Fab, Fab', F(ab')₂, Fv, domain antibody and single chain antaibody (e.g., scFv, scFv-Fc etc.) but not limited thereto, and may be derived from any mammal including human, mouse, rat, camelid or rabbit, but not limited thereto. The functional part of the antibody such as one or more CDRs described herein may be linked with a secondary protein or small molecular compound by a covalent bond, and be used as a target therapeutic agent to a specific target.

Herein, "Fab fragment" consists of one light chain and one heavy chain comprising a variable region and CH1 only. The heavy chain of Fab molecule cannot form a disulfide bond with other heavy chain.

Herein, "Fc" region comprises two heavy chain fragments comprising CH2 and CH3 domains of an antibody. These 2 heavy chain fragments are combined each other by two or more disulfide bonds and hydrophobic interaction of CH3 domain.

Herein, "Fab' fragment" comprises additionally a region between CH1 and CH2 domains of a heavy chain, in addition to Fab fragment, and can be formed by the disulfide bond between two heavy chains of two Fab' fragments.

Herein, "F(ab')₂ fragment "comprises two light chains, and two heavy chains comprising a variable region, CH1 and a part of a constant region between CH1 and CH2 domains, as aforementioned, and an interchain disulfide bond is formed between 2 heavy chains. Thus, the F(ab')₂ fragment consists of two Fab' fragments, and the two Fab' fragments are combined each other by the disulfide bond formed between them.

Herein, "Fv region" is a fragment of an antibody which comprises each variable region of a heavy chain and a light chain, but does not comprise a constant region.

Herein, "single chain antibody" is a single polypeptide chain of the antigen-binding region formed by connecting heavy chain variable region and light chain variable region via flexible linker. For example, the single chain antibody may be at least one selected from the group consisting of a scFv in which the heavy chain variable region and the light chain variable region are linked in a single chain form, a scFv-Fc in which the heavy chain variable region, a light chain variable region, and Fc are linked in a single chain form, and the like. The single chain antibody may refer to for example, U.S. patent No. 5,260,203.

Herein, "bivalent antigen-binding protein" or "bivalent antibody" comprises two antigen-binding sites. Two antigen-binding sites comprised in this bivalent antibody may have the same antigen specificity or may be a dual-specific antibody binding to different antigens respectively. Herein, "multi-specific antigen-binding protein" or "multi-specific antibody" is targeting two or more of antigens or epitopes.

Herein, "bispecific" or "dual-specific" antigen-binding protein or antibody is a hybrid antigen-binding protein or antibody having two different antigen-binding sites. Such a bispecific antibody is a kind of multi-specific antigen binding protein or multi-specific antibody, and can be produced by various known methods such as fusion of hybridomas or fusion of Fab' fragments.

Herein, "bispecific", "dual-specific" antigen-binding protein or antibody is a hybrid antigen-binding protein or antibody having 2 different antigen-binding sites. This bispecific antibody is one kind of multi-specific antigen-binding protein or multi-specific antibody, and it can be produced by known various methods, for example, methods such as fusion of hybridoma or linking of Fab' fragment. For example, Songsivilai and Lachmann, Clin. Exp. Immunol. 1990, 79:315-321; Kostelny et al., J. Immunol. 1992, 148:1547-1553 and the like may be referred. The two epitopes being different each other to which twos antigen-binding sites of the bispecific antigen-binding protein or antibody bind may be positioned on the same or different target protein. In one embodiment, the antibody of the present invention may be in the form of a bispecific antibody which additionally comprises the binding to a delivery carrier for delivering the antibody through the blood brain barrier. One method for delivering drugs through the blood brain barrier includes the use of delivery systems such as receptor-mediated transcytosis such as glucose transporter, amino acid transporter, insulin receptor or transferrin receptor in a cell.

Herein, "conjugate" means a chimeric molecule of the antibody or its antigen-binding fragment disclosed herein with other molecule, particularly blood brain barrier transports or a therapeutic agent described below. In the conjugate, the antibody or its antigen-binding fragment of the present invention is bound to other molecules by a covalent bond, or physical force such as VAN DEER WAALS or hydrophobic interaction, capsulation, embedding or the combination method thereof. In the conjugate of one embodiment, the antibody or its antigen-binding fragment of the present invention may be connected by a peptide linker.

The present invention also includes one or more amino acid sequences having substantial sequence identity to one or more amino acid sequences se disclosed herein. The substantial sequence identity means that the sequence with the variation maintains the effects disclosed in the present invention. In an embodiment, the sequence has about 90%, 95%, or 99% of sequence identity to the heavy chain variable region disclosed. In an embodiment, the sequence has about 90%, 95%, or 99% of sequence identity to the light chain variable region disclosed. For example, in the case of a variant showing 90%, 95%, or 99% of sequence identity to the sequence of the antibody or antigen-binding fragment disclosed in the present invention, any sequence variation occurs in the framework of the variable region rather than the CDR.

The antibodies or antigen-binding fragments thereof specifically binding to alpha-synuclein or an aggregate thereof according to the present invention include a heavy chain variable region comprising complementarity determining regions of CDRH1, CDRH2 and CDRH3; and a light chain variable region comprising complementarity determining regions of CDRL1, CDRL2, and CDRL3.

In an embodiment, the anti-alpha-synuclein antibody or antigen-binding fragment thereof may include the following CDR sequences:
a heavy chain CDR1 (H-CDR1) selected from the amino acid sequences of SEQ ID NO: 1 and SEQ ID NO: 6
a heavy chain CDR2 (H-CDR2) selected from the amino acid sequences of SEQ ID NOs: 2 to 4 and SEQ ID NOs: 7 to 8,
a heavy chain CDR3 (H-CDR3) selected from the amino acid sequences of SEQ ID NO: 5 and SEQ ID NO: 9,
a light chain CDR1 (L-CDR1) selected from the amino acid sequences of SEQ ID NO: 10 and SEQ ID NO: 13,
a light chain CDR2 (L-CDR2) selected from the amino acid sequences of SEQ ID NO: 11 and SEQ ID NO: 14, and
a light chain CDR3 (L-CDR3) selected from the amino acid sequences of SEQ ID NO: 12 and SEQ ID NO: 15.

The amino acid sequences of the heavy chain CDR1 to CDR3 and the amino acid sequences of the light chain CDR1 to CDR3 are summarized in Tables 1 and 2.

**[Table 1] Amino acid sequences of Heavy chain CDR1 to CDR3**

| Clone ID | SEQ ID NO | VH_CDR1 Amino acid sequence | SEQ ID NO | VH_CDR2 Amino acid sequence | SEQ ID NO | VH_CDR3 Amino acid sequence |
|---|---|---|---|---|---|---|
| ch11F11-VH | 1 | GFTFSDFYME | 2 | ASRNKANDYTTEYSASVK G | 5 | DAHGKPF AY |
| Hu11F11 -VH1 | 1 | GFTFSDFYME | 3 | AIRNKANDYTTEYAASVK G | 5 | DAHGKPF AY |
| Hu11F11 -VH2 | 1 | GFTFSDFYME | 3 | AIRNKANDYTTEYAASVK G | 5 | DAHGKPF AY |
| Hu11F11 -VH3 | 1 | GFTFSDFYME | 3 | AIRNKANDYTTEYADSVK G | 5 | DAHGKPF AY |
| Hu11F11 -VH4 | 1 | GFTFSDFYME | 3 | AIRNKANDYTTEYADSVK G | 5 | DAHGKPF AY |
| Hu11F11 -VHv3 | 1 | GFTFSDFYME | 4 | ATRNKANDYTTEYSASVK G | 5 | DAHGKPF AY |
| Hu11F11 VHvlmu 1 newmu | 1 | GFTFSDFYME | 4 | ATRNKANDYTTEYSASVK G | 5 | DAHGKPF AY |
| Hu11F11 -VHv3 newmu | 1 | GFTFSDFYME | 4 | ATRNKANDYTTEYSASVK G | 5 | DAHGKPF AY |
| Hu11F11 -VH-v1 | 1 | GFTFSDFYME | 2 | ASRNKANDYTTEYSASVK G | 5 | DAHGKPF AY |
| Hu11F11 -VH-v2 | 1 | GFTFSDFYME | 2 | ASRNKANDYTTEYSASVK G | 5 | DAHGKPF AY |
| Hu11F11 -VH-v3 | 1 | GFTFSDFYME | 2 | ASRNKANDYTTEYSASVK G | 5 | DAHGKPF AY |
| Hu11F11 -VH-v4 | 1 | GFTFSDFYME | 2 | ASRNKANDYTTEYSASVK G | 5 | DAHGKPF AY |
| ch3A9-VH | 6 | GFTFSSY AMS | 7 | TISNGGGYTYYPDSVKG | 9 | HITTVRPTKYFD Y |
| Hu3A9-VH1 | 6 | GFTFSSY AMS | 7 | TISNGGGYTYYPDSVKG | 9 | HITTVRPTKYFD Y |
| Hu3A9-VH2 | 6 | GFTFSSY AMS | 8 | TISNGGGYTYYADSVKG | 9 | HITTVRPTKYFDY |
| Hu3A9-VH3 | 6 | GFTFSSY AMS | 7 | TISNGGGYTYYPDSVKG | 9 | HITTVRPTKYFD Y |
| Hu3A9-VH4 | 6 | GFTFSSY AMS | 8 | TISNGGGYTYYADSVKG | 9 | HITTVRPTKYFD Y |
| Hu3A9-VH-v1 | 6 | GFTFSSY AMS | 7 | TISNGGGYTYYPDSVKG | 9 | HITTVRPTKYFD Y |
| Hu3A9-VH-v2 | 6 | GFTFSSY AMS | 7 | TISNGGGYTYYPDSVKG | 9 | HITTVRPTKYFD Y |

**[Table 2] Amino acid sequences of Light chain CDR1 to CDR3**

| Clone ID | SEQ ID NO | VL_CDR1 Amino acid sequence | SEQ ID NO | VL_CDR1 Amino acid sequence | SEQ ID NO | VL_CDR1 Amino acid sequence |
|---|---|---|---|---|---|---|
| ch11F11-VL | 10 | KSSQSLLYSSNQKNYLA | 11 | WASTRES | 12 | QQYYSYPWT |
| Hu11F11-VL1 | 10 | KSSQSLLYSSNQKNYLA | 11 | WASTRES | 12 | QQYYSYPWT |
| Hu11F11-VL2 | 10 | KSSQSLLYSSNQKNYLA | 11 | WASTRES | 12 | QQYYSYPWT |
| Hu11F11-VL3 | 10 | KSSQSLLYSSNQKNYLA | 11 | WASTRES | 12 | QQYYSYPWT |
| Hu11F11-VL4 | 10 | KSSQSLLYSSNQKNYLA | 11 | WASTRES | 12 | QQYYSYPWT |
| Hu11F11-VL5 | 10 | KSSQSLLYSSNQKNYLA | 11 | WASTRES | 12 | QQYYSYPWT |
| Hu11F11-VLv3 4c | 10 | KSSQSLLYSSNQKNYLA | 11 | WASTRES | 12 | QQYYSYPWT |
| ch3A9-VL | 13 | KASQNVGTTVA | 14 | SASNRYT | 15 | QQYSNYPLT |
| Hu3A9-VL1 | 13 | KASQNVGTTVA | 14 | SASNRYT | 15 | QQYSNYPLT |
| Hu3A9-VL2 | 13 | KASQNVGTTVA | 14 | SASNRYT | 15 | QQYSNYPLT |
| Hu3A9-VL3 | **13** | KASQNVGTTVA | **14** | SASNRYT | **15** | QQYSNYPLT |
| Hu3A9-VL4 | **13** | KASQNVGTTVA | **14** | SASNRYT | **15** | QQYSNYPLT |
| Hu3A9-VL-v1 | **13** | KASQNVGTTVA | **14** | SASNRYT | **15** | QQYSNYPLT |
| Hu3A9-VL-v2 | **13** | KASQNVGTTVA | **14** | SASNRYT | **15** | QQYSNYPLT |
| Hu3A9-VL-v2 | **13** | KASQNVGTTVA | **14** | SASNRYT | **15** | QQYSNYPLT |

In an embodiment, the anti-alpha-synuclein antibody or antigen-binding fragment thereof can include a heavy chain variable region including a heavy chain CDR1 (H-CDR1) comprising an amino acid sequence of SEQ ID NO: 1, heavy chain CDR2 (H-CDR2) comprising an amino acid sequence selected from the amino acid sequences of SEQ ID NOs: 2 to 4, and a heavy chain CDR3 (H-CDR3) comprising an amino acid sequence of SEQ ID NO: 5; and a heavy chain variable region including a light chain CDR1 (L-CDR1) comprising an amino acid sequence of SEQ ID NO: 10, a light chain CDR2 (L-CDR2) comprising an amino acid sequence of SEQ ID NO: 11, and a light chain CDR3 (L-CDR3) comprising an amino acid sequence of SEQ ID NO: 12.

Additionally, in an embodiment, the anti-alpha-synuclein antibody or antigen-binding fragment thereof can include a heavy chain variable region including a heavy chain CDR1 (H-CDR1) comprising an amino acid sequence of SEQ ID NO: 6, heavy chain CDR2 (H-CDR2) comprising an amino acid sequence selected from the amino acid sequences of SEQ ID NOs: 7 to 8, and a heavy chain CDR3 (H-CDR3) comprising an amino acid sequence of SEQ ID NO: 9; and a heavy chain variable region including a light chain CDR1 (L-CDR1) comprising an amino acid sequence of SEQ ID NO: 13, a light chain CDR2 (L-CDR2) comprising an amino acid sequence of SEQ ID NO: 14, and a light chain CDR3 (L-CDR3) comprising an amino acid sequence of SEQ ID NO: 15.

In other embodiment, the anti-alpha-synuclein antibody or antigen-binding fragment thereof can include
a heavy chain framework (H-FR1) located at N-terminus of H-CDR1 comprising a polypeptide fragment including an amino acid sequence selected from SEQ ID NOs: 16 to 17 and SEQ ID NOs: 35 to 40,
a heavy chain framework (H-FR2) located between H-CDR1 and H-CDR2 comprising a polypeptide fragment including an amino acid sequence selected from SEQ ID NOs: 18 to 19 and SEQ ID NOs: 41 to 44,
a heavy chain framework (H-FR3) located between H-CDR2 and H-CDR3 comprising a polypeptide fragment including an amino acid sequence selected from SEQ ID NOs: 20 to 31 and SEQ ID NOs: 45 to 50,
a heavy chain framework (H-FR4) being located at C-terminus of H-CDR3 comprising a polypeptide fragment including an amino acid sequence selected from SEQ ID NOs: 32 to 34 and SEQ ID NOs: 51 to 52,
a light chain framework (L-FR1) located at N-terminus of L-CDR1 comprising a polypeptide fragment including an amino acid sequence selected from SEQ ID NOs: 53 to 58 and SEQ ID NOs: 71 to 77,
a light chain framework (L-FR2) located between L-CDR1 and L-CDR2 comprising a polypeptide fragment including an amino acid sequence selected from SEQ ID NOs: 59 to 61 and SEQ ID NOs: 78 to 81,
a light chain framework (L-FR3) located between L-CDR2 and L-CDR3 comprising a polypeptide fragment including an amino acid sequence selected from SEQ ID NOs: 62 to 67 and SEQ ID NOs: 82 to 88, and/or
a light chain framework (L-FR4) located at C-terminus of L-CDR3 comprising a polypeptide fragment including an amino acid sequence selected from SEQ ID NOs: 68 to 70 and SEQ ID NO: 89.

As the amino acid sequences being useful as frameworks herein, the heavy chain framework sequences are exemplified in Tables 3 and 4, and light chain frameworks sequences are exemplified in Tables 5 and 6.

**[Table 3] Amino acid sequences of heavy chain frameworks 1 to 2**

| Clone ID | SEQ ID NO | VH-FR1-Sequence | SEQ ID NO | VH-FR2 Sequence |
|---|---|---|---|---|
| ch11F11-VH | 16 | EVQLQESGGGLVQPGGSLRLSCATS | 18 | WVRQPPGKRLEWIA |
| Hu11F11-VH1 | 17 | EVQLVESGGGLVQPGGSLRLSCAAS | 19 | WVRQAPGKGLEWIA |
| Hu11F11-VH2 | 17 | EVQLVESGGGLVQPGGSLRLSCAAS | 19 | WVRQAPGKGLEWIA |
| Hu11F11-VH3 | 17 | EVQLVESGGGLVQPGGSLRLSCAAS | 19 | WVRQAPGKGLEWIA |
| Hu11F11-VH4 | 17 | EVQLVESGGGLVQPGGSLRLSCAAS | 19 | WVRQAPGKGLEWVA |
| Hu11F11-VHv3 | 17 | EVQLVESGGGLVQPGGSLRLSCATS | 18 | WVRQPPGKRLEWIA |
| Hu11F11-VHvlmul newmu | 17 | EVQLVESGGGLVQPGGSLRLSCATS | 18 | WVRQPPGKRLEWIA |
| Hu11F11-VHv3 newmu | 17 | EVQLVESGGGLVQPGGSLRLSCATS | 18 | WVRQPPGKRLEWIA |
| Hu11F11-VH-v1 | 17 | EVQLVESGGGLVQPGGSLRLSCATS | 18 | WVRQPPGKRLEWIA |
| Hu11F11-VH-v2 | 17 | EVQLVESGGGLVQPGGSLRLSCATS | 18 | WVRQPPGKRLEWIA |
| Hu11F11-VH-v3 | 17 | EVQLVESGGGLVQPGGSLRLSCATS | 18 | WVRQPPGKRLEWIA |
| Hu11F11-VH-v4 | 17 | EVQLVESGGGLVQPGGSLRLSCATS | 18 | WVRQPPGKRLEWIA |
| ch3A9-VH | 35 | EVQLQESGGGLVKPGGSLKLSCAAS | 41 | WVRQTPEKRLEWVA |
| Hu3A9-VH1 | 36 | QVQLLESGGGLVQPGGSLRLSCAAS | 42 | WVRQAPGKGLEWVA |
| Hu3A9-VH2 | 37 | EVQLVQSGGGLVQPGGSLRLSCAAS | 43 | WVRQAPDKGLEWVA |
| Hu3A9-VH3 | 38 | EVQLVESGGGLVQPGGSLRLSCAAS | 42 | WVRQAPGKGLEWVA |
| Hu3A9-VH4 | 38 | EVQLVESGGGLVQPGGSLRLSCAAS | 42 | WVRQAPGKGLEWVA |
| Hu3A9-VH-v1 | 39 | EVQLLESGGGLVQPGGSLRLSCAAS | 44 | WVRQTPEKGLEWVA |
| Hu3A9-VH-v2 | 40 | EVQLLESGGGLVQPGGSLKLSCAAS | 44 | WVRQTPEKGLEWVA |

**[Table 4] Amino acid sequences of heavy chain frameworks 3 to 4**

| clone | SEQ ID NO | VH-FR3-Sequence | SEQ ID NO | VH-FR4 Sequence |
|---|---|---|---|---|
| ch11F11-VH | 20 | RFIVSRDTSQSILYLQMNALRAEDTAIYYCAR | 32 | WGQGTL VTVSA |
| Hu11F11-VH1 | 21 | RFTVSRDTSKNSLYLQMNSLKTEDTAVYYCA R | 33 | WGQGTL VTVSS |
| Hu11F11-VH2 | 22 | RFTISRDTSKNSLYLQMNSLKTEDTAVYYCA R | 33 | WGQGTL VTVSS |
| Hu11F11-VH3 | 23 | RFTVSRDTSQNTLYLQMNSLRAEDTAVYYCA R | 33 | WGQGTL VTVSS |
| Hu11F11-VH4 | 24 | RFTISADTSKNTAYLQMNSLRAEDTAVYYCS R | 33 | WGQGTL VTVSS |
| Hu11F11-VHv3 | 25 | RFTISRDDSKSSLYLQMNSLRAEDTAIYYCAR | 34 | WGQGTTVTVSS |
| Hu11F11-VHvlmu 1 newmu | 26 | RFTISRDTSQSSLYLQMNSLKTEDTAVYYCAR | 34 | WGQGTTVTVSS |
| Hu11F11-VHv3 newmu | 27 | RFTISRDTSQSSLYLQMNSLRAEDTAIYYCAR | 34 | WGQGTTVTVSS |
| Hu11F11-VH-v1 | 28 | RFTISRDDSKSSLYLQMNSLRAEDTAIYYCAR | 34 | WGQGTTVTVSS |
| Hu11F11-VH-v2 | 29 | RFTVSRDDSKSSLYLQMNSLRAEDTAIYYCA R | 34 | WGQGTTVTVSS |
| Hu11F11-VH-v3 | 30 | RFTISRDTSKSSLYLQMNSLRAEDTAIYYCAR | 34 | WGQGTTVTVSS |
| Hu11F11-VH-v4 | 31 | RFTVSRDTSKSSLYLQMNSLRAEDTAIYYCAR | 34 | WGQGTTVTVSS |
| ch3A9-VH | 45 | RFTISRDNAKNTLYLQMSSLRSEDTAMYYCA R | 51 | WGQGTTLTVSS |
| Hu3A9-VH1 | 46 | RFTISRDNAKNTLYLQMNSLRSEDSAMYYCA R | 52 | WGQGTL VTVSS |
| Hu3A9-VH2 | 47 | RFTISRDNAKNTLYLQMSSLKAEDSAVYYCA R | 52 | WGQGTL VTVSS |
| Hu3A9-VH3 | 48 | RFTISRDNSKNTLYLQMNSLRAEDTAVYYCA R | 52 | WGQGTL VTVSS |
| Hu3A9-VH4 | 49 | RFTISADTSKNTAYLQMNSLRAEDTAVYYCA R | 52 | WGQGTL VTVSS |
| Hu3A9-VH-v1 | 50 | RFTISRDNSKNTLYLQMSSLRAEDTAMYYCA R | 51 | WGQGTTLTVSS |
| Hu3A9-VH-v2 | 50 | RFTISRDNSKNTLYLQMSSLRAEDTAMYYCA R | 51 | WGQGTTLTVSS |

**[Table 5] Amino acid sequences of light chain frameworks 1 to 2**

| clone | SEQ ID NO | VH-FR1-Sequence | SEQ ID NO | VH-FR2 Sequence |
|---|---|---|---|---|
| ch11F11-VL | 53 | DIVMTQSPSSLAVSVGEKVTMSC | 59 | WYQQKPGQSPKLLIY |
| Hu11F11-VL1 | 54 | DIVMTQSPDSLAVSLGERATINC | 60 | WYQQKPGQPPKLLIY |
| Hu11F11-VL2 | 55 | DIVMTQSPSSLAVSLGERVTITC | 60 | WYQQKPGQPPKLLIY |
| Hu11F11-VL3 | 56 | DIVMTQSPSSLAVSLGERATINC | 60 | WYQQKPGQPPKLLIY |
| Hu11F11-VL4 | 57 | DIQMTQSPSSLSASVGDRVTITC | 61 | WYQQKPGKAPKLLIY |
| Hu11F11-VL5 | 53 | DIVMTQSPSSLA VSVGEKVTMSC | 60 | WYQQKPGQPPKLLIY |
| Hu11F11-VLv3 4c | 58 | DIVMTQSPSSLA VSLGERVTMSC | 59 | WYQQKPGQSPKLLIY |
| ch3A9-VL | 71 | DIVMTQSPKFMSTSVGDRVSITC | 78 | WYQQKPGQSPKLLIY |
| Hu3A9-VL1 | 72 | DIQMTQSPSSLSASVGDRVTITC | 79 | WYQQKPGKAPKLLIY |
| Hu3A9-VL2 | 73 | DIVMTQSPSTLSASVGDRVTITC | 80 | A WYQQKPGKAPKLLIY |
| Hu3A9-VL3 | 74 | DIVMTQSPATLSVSLGERATLSC | 81 | WYQQKPGQAPRLLIY |
| Hu3A9-VL4 | 75 | DIQMTQSPSSLSASVGDRVTITC | 79 | WYQQKPGKAPKLLIY |
| Hu3A9-VL-v1 | 76 | DIQMTQSPSSLSASVGDRVTITC | 78 | WYQQKPGQSPKLLIY |
| Hu3A9-VL-v2 | 77 | DIVMTQSPSSMSTSVGDRVTITC | 78 | WYQQKPGQSPKLLIY |
| Hu3A9-VL-v2 | 77 | DIVMTQSPSSMSTSVGDRVTITC | 78 | WYQQKPGQSPKLLIY |

**[Table 6] Amino acid sequences of light chain frameworks 3 to 4**

| clone | SEQ ID NO | VH-FR3-Sequence | SEQ ID NO | VH-FR4 Sequence |
|---|---|---|---|---|
| ch11F11-VL | 62 | GVPDRFTGSGSGTDFTLTISSVKAEDLAVYYC | 68 | FGGGTKLEIK |
| Hu11F11-VL1 | 63 | GVPDRFSGSGSGTDFTLTISSLQAEDVAVYYC | 69 | FGGGTKVEIK |
| Hu11F11-VL2 | 64 | GVPDRFSGSGSGTDFTLTISSVQAEDVAVYYC | 68 | FGGGTKLEIK |
| Hu11F11-VL3 | 63 | GVPDRFSGSGSGTDFTLTISSLQAEDVAVYYC | 68 | FGGGTKLEIK |
| Hu11F11-VL4 | 65 | GVPSRFSGSGSGTDFTLTISSLQPEDFATYYC | 70 | FGQGTKVEIK |
| Hu11F11-VL5 | 66 | GVPDRFSGSGSGTDFTLTISSVQAEDLAVYYC | 68 | FGGGTKLEIK |
| Hu11F11-VLv3 4c | 67 | GVPDRFTGSGSGTDFTLTISSVKAEDVAVYYC | 68 | FGGGTKLEIK |
| ch3A9-VL | 82 | GVPDRFTGSGSGTDFTLTISNMQSEDLADYFC | 89 | FGAGTKLELR R |
| Hu3A9-VL1 | 83 | GVPDRFSGSGSGTDFTLTISSLQPEDFATYYC | 89 | FGAGTKLELR R |
| Hu3A9-VL2 | 84 | GVPSRFSGSGSGTEFTLTISSLQPDDFASYYC | 89 | FGAGTKLELR R |
| Hu3A9-VL3 | 85 | GIPARFSGSGSGTEFTLTISSLQSEDFAVYYC | 89 | FGAGTKLELR R |
| Hu3A9-VL4 | 86 | GVPSRFSGSGSGTDFTLTISSLQPEDFATYYC | 89 | FGAGTKLEL R |
| Hu3A9-VL-v1 | 87 | GVPDRFSGSGSGTDFTFTISSMQSEDIATYFC | 89 | FGAGTKLELR R |
| Hu3A9-VL-v2 | 88 | GVPDRFSGSGSGTDFTLTISSMQSEDLADYYC | 89 | FGAGTKLELR R |
| Hu3A9-VL-v2 | 88 | GVPDRFSGSGSGTDFTLTISSMQSEDLADYYC | 89 | FGAGTKLELR R |

In other embodiment, the anti-alpha-synuclein antibody or antigen-binding fragment thereof can include a heavy chain variable region comprising a heavy chain CDR1 (H-CDR1) comprising an amino acid selected from SEQ ID NO: 1 and SEQ ID NO: 6, a heavy chain CDR2 (H-CDR2) comprising an amino acid selected from SEQ ID NOs: 2 to 4 and SEQ ID NOs: 7 to 8, and a heavy chain CDR3 (H-CDR3) comprising an amino acid selected from SEQ ID NO: 5 and SEQ ID NO: 9,
in which the heavy chain variable region further comprises a heavy chain framework (H-FR1) comprising an amino acid sequence selected from SEQ ID NOs: 16 to 17 and SEQ ID NOs: 35 to 40, a heavy chain framework (H-FR2) comprising an amino acid sequence selected from SEQ ID NOs: 18 to 19 and SEQ ID NOs: 41 to 44, a heavy chain framework (H-FR3) comprising an amino acid sequence selected from SEQ ID NOs: 20 to 31 and SEQ ID NOs: 45 to 50, and a heavy chain framework (H-FR4) comprising an amino acid sequence selected from SEQ ID NOs: 32 to 34 and SEQ ID NOs: 51 to 52. More specifically, the heavy chain variable region comprises a heavy chain framework (H-FR1) comprising an amino acid sequence of SEQ ID NO: 17, a heavy chain framework (H-FR2) comprising an amino acid sequence of SEQ ID NO: 18, a heavy chain framework (H-FR3) comprising an amino acid sequence selected from SEQ ID NOs: 29 to 31, and a heavy chain framework (H-FR4) comprising an amino acid sequence of SEQ ID NO: 34.

In addition, the anti-alpha-synuclein antibody or antigen-binding fragment thereof can include a light chain variable region comprising a light chain CDR1 (L-CDR1) comprising an amino acid sequence selected from SEQ ID NO: 10 and SEQ ID NO: 13, a light chain CDR2 (L-CDR2) comprising an amino acid sequence selected from SEQ ID NO: 11 and SEQ ID NO: 14, and a light chain CDR3 (L-CDR3) comprising an amino acid sequence selected from SEQ ID NO: 12 and SEQ ID NO: 15,
in which the light chain variable region further comprises a light chain framework (L-FR1) comprising an amino acid sequence selected from SEQ ID NOs: 53 to 58 and SEQ ID NOs: 71 to 77, a light chain framework (L-FR2) comprising a an amino acid sequence selected from SEQ ID NOs: 59 to 61 and SEQ ID NOs: 78 to 81, a light chain framework (L-FR3) comprising an amino acid sequence selected from SEQ ID NOs: 62 to 67 and SEQ ID NOs: 82 to 88, and a light chain framework (L-FR4) comprising an amino acid sequence selected from SEQ ID NOs: 68 to 70 and SEQ ID NO: 89. More specifically, the light chain variable region comprises a light chain framework (L-FR1) comprising a polypeptide fragment including an amino acid sequence of SEQ ID NO: 58, a light chain framework (L-FR2) comprising a polypeptide fragment including an amino acid sequence of SEQ ID NO: 59, a light chain framework (L-FR3) comprising a polypeptide fragment including an amino acid sequence of SEQ ID NO: 67 and a light chain framework (L-FR4) comprising a polypeptide fragment including an amino acid sequence of SEQ ID NO: 34.

However, the anti-alpha-synuclein antibody or antigen-binding fragment thereof of the present invention does not include a mouse antibody or a chimeric antibody, or antigen-binding fragment thereof including the antibody consisting of heavy chain CDR1-CDR3 consisting of amino acid sequences of SEQ ID NOs: 1, 2 and 5, light chain CDR1-CDR3 consisting of SEQ ID NOs: 10, 11 and 12, heavy chain frameworks 1 to 4 consisting of amino acid sequences of SEQ ID NOs: 16, 18, 20 and 32, and light chain frameworks 1 to 4 consisting of amino acid sequences of SEQ ID NO: 53, 59, 62 and 68. In addition, the anti-alpha-synuclein antibody or antigen-binding fragment thereof of the present invention does not include a mouse antibody or a chimeric antibody, or antigen-binding fragment thereof including the antibody consisting of heavy chain CDR1-CDR3 consisting of amino acid sequences of SEQ ID NOs: 6, 7 and 9, light chain CDR1-CDR3 consisting of SEQ ID NOs: 13, 14 and 15, heavy chain frameworks 1 to 4 consisting of amino acid sequences of SEQ ID NOs: 35, 41, 45 and 51, and light chain frameworks 1 to 4 consisting of amino acid sequences of SEQ ID NO: 71, 78, 82 and 89. As a specific example, the anti-alpha-synuclein antibody of the present invention does not include an antibody consisting of heavy chain variable region of SEQ ID NO: 90 (ch11F11-VH), and a light chain variable region of SEQ ID NO: 109 (ch11F11-VL), or Ch3A9 antibody consisting of heavy chain variable region of SEQ ID NO: 102 (ch11F11-VH), and a light chain variable region of SEQ ID NO: 116 (ch11F11-VL).

The antibody or antigen binding fragment thereof according to an embodiment of the present invention can comprise a heavy chain variable region comprising a heavy chain CDR1 (H-CDR1) comprising an amino acid sequence of SEQ ID NO: 1, a heavy chain CDR2 (H-CDR2) comprising an amino acid sequence selected from SEQ ID NO: 2 to 4, and a heavy chain CDR3 (H-CDR3) comprising an amino acid sequence of SEQ ID NO: 5. and a light chain variable region comprising a light chain CDR1 (L-CDR1) comprising an amino acid sequence of SEQ ID NO: 10, light chain CDR2 (L-CDR2) comprising an amino acid sequence of SEQ ID NO: 11, and a light chain CDR3 (L-CDR3) comprising an amino acid sequence of SEQ ID NO: 12,
in which the heavy chain variable region comprises a heavy chain framework (H-FR1) comprising an amino acid sequence selected from SEQ ID NOs: 16 to 17, a heavy chain framework (H-FR2) comprising an amino acid sequence selected from SEQ ID NOs: 18 to 19, a heavy chain framework (H-FR3) comprising an amino acid sequence selected from SEQ ID NOs: 20 to 31, and a heavy chain framework (H-FR4) comprising an amino acid sequence selected from SEQ ID NOs: 32 to 34, and
in which a light chain variable region comprises, a light chain framework (L-FR1) comprising an amino acid sequence selected from SEQ ID NOs: 53 to 58, a light chain framework (L-FR2) comprising an amino acid sequence selected from SEQ ID NOs: 59 to 61, a light chain framework (L-FR3) comprising an amino acid sequence selected from SEQ ID NOs: 62 to 67, and a light chain framework (L-FR4) comprising an amino acid sequence selected from SEQ ID NOs: 68 to 70.

Preferably, the antibody or antigen binding fragment thereof according to an embodiment of the present invention comprises a heavy chain variable region comprising a heavy chain CDR1 to CDR3 comprising amino acid sequences of SEQ ID NOs: 1, 2 and 5, and a light chain variable region comprising a light chain CDR1 to CDR3 comprising amino acid sequences of SEQ ID NOs: 10, 11, and 12, in which the heavy chain variable region comprises, a heavy chain framework1 (H-FR1) comprising an amino acid sequence of SEQ ID NO: 17, a heavy chain framework (H-FR2) comprising an amino acid sequence of SEQ ID NO: 18, a heavy chain framework (H-FR3) comprising an amino acid sequence selected from SEQ ID NOs: 29 to 31 and a heavy chain framework (H-FR4) comprising an amino acid sequence of SEQ ID NO: 34, and the light chain variable region comprises, a light chain framework1 (L-FR1) comprising an amino acid sequence of SEQ ID NO: 58, a light chain framework (L-FR2) comprising an amino acid sequence of SEQ ID NO: 59, a light chain framework (L-FR3) comprising an amino acid sequence of SEQ ID NO: 67, and a light chain framework (L-FR4) comprising an amino acid sequence of SEQ ID NO: 34,

The anti-alpha-synuclein antibody or antigen-binding fragment thereof of according to an embodiment of the present invention comprises, a heavy chain variable region comprising a heavy chain CDR1 (H-CDR1) comprising an amino acid sequence of SEQ ID NO: 6, a heavy chain CDR2 (H-CDR2) comprising an amino acid sequence selected from SEQ ID NOs: 7 to 8, and a heavy chain CDR3 (H-CDR3) comprising an amino acid sequence of SEQ ID NO: 9. and a light chain variable region comprising a light chain CDR1 (L-CDR1) comprising an amino acid sequence of SEQ ID NO: 13, light chain CDR2 (L-CDR2) comprising an amino acid sequence of SEQ ID NO: 14, and a light chain CDR3 (L-CDR3) comprising an amino acid sequence of SEQ ID NO: 15,
in which the heavy chain variable region comprises, a heavy chain framework1 (H-FR1) comprising an amino acid sequence selected from SEQ ID NOs: 35 to 40, a heavy chain framework (H-FR2) comprising an amino acid sequence selected from SEQ ID NOs: 41 to 44, a heavy chain framework (H-FR3) comprising an amino acid sequence selected from SEQ ID NOs: 45 to 50, and a heavy chain framework (H-FR4) comprising an amino acid sequence selected from SEQ ID NOs: 51 to 53, and
in which the light chain variable region comprises, a light chain framework1 (L-FR1) comprising an amino acid sequence selected from SEQ ID NOs: 71 to 77, a light chain framework (L-FR2) comprising an amino acid sequence selected from SEQ ID NOs: 78 to 81, a light chain framework (L-FR3) comprising an amino acid sequence selected from SEQ ID NOs: 82 to 88 and a light chain framework (L-FR4) comprising an amino acid sequence of SEQ ID NO: 89.

In the full length forms of light chain and heavy chain, the variable region and the constant region are joined by a "J" region in about 12 or more amino acid length, and the heavy chain also includes a "D" region in about 10 or more amino acid length. For example, Fundamental Immunology, 2nd ed., Ch. 7 (Paul, W., ed.) 1989, New York: Raven Press can be referred. Typically, the variable regions of the light chain and heavy chain pair in the antibody can form an antigen binding region.

The variable region of immunoglobulin chain has typically the same entire structure, and comprises the relatively-conserved framework region (FR) connected by three hypervariable regions called as "complementary determining region or domain" or CDR. The CDRs of the variable regions from each chain constituting the heavy chain / light chain pair are typically aligned by the framework regions, so as to form a structure specifically binding to a specific epitope of the target protein (alpha-synuclein). These elements of the naturally-occurring light chain variable region and heavy chain variable region are typically included in the following order from the N-terminus to the C-terminus: FR1, CDR1, FR2, CDR2, FR3, CDR3 and FR4. The position of the amino acid sequence corresponding to each of these elements in the variable region is designated according to the Kabat Numbering System (Kabat Sequences of Proteins of Immunological Interest), according to the disclosure of 1987 and 1991, NIH, Bethesda, MD)), or Chothia & Lesk, 1987, J. Mol. Biol. 196 : 901-917; Chothia et al., 1989, Nature 342: 878-883.

Various heavy chain variable regions and light chain variable regions disclosed herein are shown in Tables 7 and 8. Each of these variable regions can be coupled to the heavy chain constant region and light chain constant region to form heavy chain and light chain of intact antibodies. In addition, the obtained heavy chain sequence and light chain sequence can also be combined to form a complete antibody structure. For example, the anti-alpha-synuclein antibody or antigen-binding fragment thereof according to the present invention, can include a heavy chain variable region comprising an amino acid sequence selected from SEQ ID NOs: 90 to 101 or SEQ ID NOs: 102 to 108, and a light chain variable region containing an amino acid sequence selected from SEQ ID NOs: 109 to 115 or SEQ ID NOs: 116 to 123, and more specifically, a heavy chain variable region comprising an amino acid sequence selected from SEQ ID NOs: 90 to 101 and a light chain variable region comprising g an amino acid sequence selected from SEQ ID NOs: 109 to 115, or a heavy chain variable region containing an amino acid sequence selected from SEQ ID NOs: 102 to 108 and a light chain variable region comprising an amino acid sequence selected from SEQ ID NOs: 116 to 123.

In a specific embodiment of the present invention, the anti-alpha-synuclein antibody or antigen-binding fragment thereof can include a heavy chain variable region comprising an amino acid sequence selected from SEQ ID NOs: 98 to 101 and a light chain variable region comprising an amino acid sequence of SEQ ID NO: 115 (e.g., Hu11F11_ (ver.1), Hu11F11_ (ver.2), Hu11F11_ (ver.3), Hu11F11_ (ver.4), and etc.), or can include a heavy chain variable region comprising an amino acid sequence of SEQ ID NO: 92 and a light chain variable region comprising an amino acid sequence of SEQ ID NO: 113 (e.g., Hu11F11_ (ABL2-4) antibody).

However, the anti-alpha-synuclein antibody or antigen-binding fragment thereof does not include antibody consisting of a heavy chain variable region of SEQ ID NO: 90 and a light chain variable region of SEQ ID NO: 109, and antibody consisting of a heavy chain variable region of SEQ ID NO: 102 and the light chain variable region of SEQ ID NO: 116.

The amino acid sequences of the heavy chain variable region and the light chain variable region of the antibody or antigen-binding fragment according to an embodiment are exemplified in Tables 7 and 8.

**[Table 7] Heavy chain variable region**

| clone | SEQ ID NO | Amino acid sequence |
|---|---|---|
| ch11F11-VH | 90 | EVQLQESGGGLVQPGGSLRLSCATSGFTFSDFYMEWVRQPPGKRL EWIAASRNKANDYTTEYSASVKGRFIVSRDTSQSILYLQMNALRAE DTAIYYCARDAHGKPFAYWGQGTLVTVSA |
| Hu11F11-VH1 | 91 | EVQLVESGGGLVQPGGSLRLSCAASGFTFSDFYMEWVRQAPGKGL EWIAAIRNKANDYTTEYAASVKGRFTVSRDTSKNSLYLQMNSLKT EDTAVYYCARDAHGKPFAYWGQGTLVTVSS |
| Hu11F11-VH2 | 92 | EVQLVESGGGLVQPGGSLRLSCAASGFTFSDFYMEWVRQAPGKGL EWIAAIRNKANDYTTEYAASVKGRFTISRDTSKNSLYLQMNSLKTE DTAVYYCARDAHGKPFAYWGQGTLVTVSS |
| Hu11F11-VH3 | 93 | EVQLVESGGGLVQPGGSLRLSCAASGFTFSDFYMEWVRQAPGKGL EWIAAIRNKANDYTTEYADSVKGRFTVSRDTSQNTLYLQMNSLRA EDTAVYYCARDAHGKPFAYWGQGTLVTVSS |
| Hu11F11-VH4 | 94 | EVQLVESGGGLVQPGGSLRLSCAASGFTFSDFYMEWVRQAPGKGL EWVAAIRNKANDYTTEYADSVKGRFTISADTSKNTAYLQMNSLR AEDTAVYYCSRDAHGKPFAYWGQGTLVTVSS |
| Hu11F11-VHv3 | 95 | EVQLVESGGGLVQPGGSLRLSCATSGFTFSDFYMEWVRQPPGKRL EWIAATRNKANDYTTEYSASVKGRFTISRDDSKSSLYLQMNSLRA EDTAIYYCARDAHGKPFAYWGQGTTVTVSS |
| Hu11F11-VHvlmul newmu | 96 | EVQLVESGGGLVQPGGSLRLSCATSGFTFSDFYMEWVRQPPGKRL EWIAATRNKANDYTTEYSASVKGRFTISRDTSQSSLYLQMNSLKTE DTAVYYCARDAHGKPFAYWGQGTTVTVSS |
| Hu11F11- VHv3 newmu | 97 | EVQLVESGGGLVQPGGSLRLSCATSGFTFSDFYMEWVRQPPGKRL EWIAATRNKANDYTTEYSASVKGRFTISRDTSQSSLYLQMNSLRAE DTAIYYCARDAHGKPFAYWGQGTTVTVSS |
| Hu11F11-VH-v1 | 98 | EVQLVESGGGLVQPGGSLRLSCATSGFTFSDFYMEWVRQPPGKRL EWIAASRNKANDYTTEYSASVKGRFTISRDDSKSSLYLQMNSLRA EDTAIYYCARDAHGKPFAYWGQGTTVTVSS |
| Hu11F11-VH-v2 | 99 | EVQLVESGGGLVQPGGSLRLSCATSGFTFSDFYMEWVRQPPGKRL EWIAASRNKANDYTTEYSASVKGRFTVSRDDSKSSLYLQMNSLRA EDTAIYYCARDAHGKPFAYWGQGTTVTVSS |
| Hu11F11- VH-v3 | 100 | EVQLVESGGGLVQPGGSLRLSCATSGFTFSDFYMEWVRQPPGKRL EWIAASRNKANDYTTEYSASVKGRFTISRDTSKSSLYLQMNSLRAE DTAIYYCARDAHGKPFAYWGQGTTVTVSS |
| Hu11F11- VH-v4 | 101 | EVQLVESGGGLVQPGGSLRLSCATSGFTFSDFYMEWVRQPPGKRL EWIAASRNKANDYTTEYSASVKGRFTVSRDTSKSSLYLQMNSLRA EDTAIYYCARDAHGKPFAYWGQGTTVTVSS |
| ch3A9-VH | 102 | EVQLQESGGGLVKPGGSLKLSCAASGFTFSSYAMSWVRQTPEKRL EWVATISNGGGYTYYPDSVKGRFTISRDNAKNTLYLQMSSLRSED TAMYYCARHITTVRPTKYFDYWGQGTTLTVSS |
| Hu3A9-VH1 | 103 | QVQLLESGGGLVQPGGSLRLSCAASGFTFSSYAMSWVRQAPGKGL EWVATISNGGGYTYYPDSVKGRFTISRDNAKNTLYLQMNSLRSED SAMYYCARHITTVRPTKYFDYWGQGTLVTVSS |
| Hu3A9-VH2 | 104 | EVQLVQSGGGLVQPGGSLRLSCAASGFTFSSYAMSWVRQAPDKG LEWVATISNGGGYTYYADSVKGRFTISRDNAKNTLYLQMSSLKAE DSAVYYCARHITTVRPTKYFDYWGQGTLVTVSS |
| Hu3A9-VH3 | 105 | EVQLVESGGGLVQPGGSLRLSCAASGFTFSSYAMSWVRQAPGKGL EWVATISNGGGYTYYPDSVKGRFTISRDNSKNTLYLQMNSLRAED TAVYYCARHITTVRPTKYFDYWGQGTLVTVSS |
| Hu3A9-VH4 | 106 | EVQLVESGGGLVQPGGSLRLSCAASGFTFSSYAMSWVRQAPGKGL EWVATISNGGGYTYYADSVKGRFTISADTSKNTAYLQMNSLRAED TAVYYCARHITTVRPTKYFDYWGQGTLVTVSS |
| Hu3A9-VH-vl | 107 | EVQLLESGGGLVQPGGSLRLSCAASGFTFSSYAMSWVRQTPEKGL EWVATISNGGGYTYYPDSVKGRFTISRDNSKNTLYLQMSSLRAED TAMYYCARHITTVRPTKYFDYWGQGTTLTVSS |
| Hu3A9-VH-v2 | 108 | EVQLLESGGGLVQPGGSLKLSCAASGFTFSSYAMSWVRQTPEKGL EWVATISNGGGYTYYPDSVKGRFTISRDNSKNTLYLQMSSLRAED TAMYYCARHITTVRPTKYFDYWGQGTTLTVSS |
| ch9B11-VH | 124 | EVQLQESGGGLVQPKGSLKLSCAASGFTFNTYAMNWVRQAPGKG LEWVARIRSKSNNYATYYADSVKDRFTISRDDSQSMLYLQMNNL KTEDTAMYYCVRQDFDYWGQGTTLTVSS |

**[Table 8] Light chain variable region**

| clone | SEQ ID NO | Amino acid sequence |
|---|---|---|
| ch11F11-VL | 109 | DIVMTQSPSSLAVSVGEKVTMSCKSSQSLLYSSNQKNYLAWYQQK PGQSPKLLIYWASTRESGVPDRFTGSGSGTDFTLTISSVKAEDLAVY YCQQYYSYPWTFGGGTKLEIK |
| Hu11F11-VL1 | 110 | DIVMTQSPDSLAVSLGERATINCKSSQSLLYSSNQKNYLAWYQQK PGQPPKLLIYWASTRESGVPDRFSGSGSGTDFTLTISSLQAEDVAVY YCQQYYSYPWTFGGGTKVEIK |
| Hu11F11-VL2 | 111 | DIVMTQSPSSLAVSLGERVTITCKSSQSLLYSSNQKNYLAWYQQKP GQPPKLLIYWASTRESGVPDRFSGSGSGTDFTLTISSVQAEDVAVY YCQQYYSYPWTFGGGTKLEIK |
| Hu11F11- VL3 | 112 | DIVMTQSPSSLAVSLGERATINCKSSQSLLYSSNQKNYLAWYQQKP GQPPKLLIYWASTRESGVPDRFSGSGSGTDFTLTISSLQAEDVAVY YCQQYYSYPWTFGGGTKLEIK |
| Hu11F11-VL4 | 113 | DIQMTQSPSSLSASVGDRVTITCKSSQSLLYSSNQKNYLAWYQQKP GKAPKLLIYWASTRESGVPSRFSGSGSGTDFTLTISSLQPEDFATYY CQQYYSYPWTFGQGTKVEIK |
| Hu11F11- VL5 | 114 | DIVMTQSPSSLAVSVGEKVTMSCKSSQSLLYSSNQKNYLAWYQQK PGQPPKLLIYWASTRESGVPDRFSGSGSGTDFTLTISSVQAEDLAVY YCQQYYSYPWTFGGGTKLEIK |
| Hu11F11-VLv3 4c | 115 | DIVMTQSPSSLAVSLGERVTMSCKSSQSLLYSSNQKNYLAWYQQK PGQSPKLLIYWASTRESGVPDRFTGSGSGTDFTLTISSVKAEDVAV YYCQQYYSYPWTFGGGTKLEIK |
| ch3A9-VL | 116 | DIVMTQSPKFMSTSVGDRVSITCKASQNVGTTVAWYQQKPGQSPK LLIYSASNRYTGVPDRFTGSGSGTDFTLTISNMQSEDLADYFCQQY SNYPLTFGAGTKLELR |
| Hu3A9-VL1 | 117 | DIQMTQSPSSLSASVGDRVTITCKASQNVGTTVAWYQQKPGKAPK LLIYSASNRYTGVPDRFSGSGSGTDFTLTISSLQPEDFATYYCQQYS NYPLTFGGGTKLEIK |
| Hu3A9-VL2 | 118 | DIVMTQSPSTLSASVGDRVTITCKASQNVGTTVAWYQQKPGKAPK LLIYSASNRYTGVPSRFSGSGSGTEFTLTISSLQPDDFASYYCQQYS NYPLTFGQGTKVEIK |
| Hu3A9-VL3 | 119 | DIVMTQSPATLSVSLGERATLSCKASQNVGTTVAWYQQKPGQAPR LLIYSASNRYTGIPARFSGSGSGTEFTLTISSLQSEDFAVYYCQQYSN YPLTFGGGTKVEIK |
| Hu3A9-VL4 | 120 | DIQMTQSPSSLSA SVGDRVTITCKASQNVGTTVAWYQQKPGKAPKLLIYSASNRYTGV PSRFSGSGSGTDFTLTISSLQPEDFATYYCQQYSNYPLTFGQGTKVE |
| | | IK |
| Hu3A9-VL-v1 | 121 | DIQMTQSPSSLSASVGDRVTITCKASQNVGTTVAWYQQKPGQSPK LLIYSASNRYTGVPDRFSGSGSGTDFTFTISSMQSEDIATYFCQQYS NYPLTFGQGTKLEIK |
| Hu3A9-VL-v2 | 122 | DIVMTQSPSSMSTSVGDRVTITCKASQNVGTTVAWYQQKPGQSPK LLIYSASNRYTGVPDRFSGSGSGTDFTLTISSMQSEDIADYYCQQYS NYPLTFGQGTKLEIK |
| Hu3A9-VL-v2 | 123 | DIVMTQSPSSMSTSVGDRVTITCKASQNVGTTVAWYQQKPGQSPK LLIYSASNRYTGVPDRFSGSGSGTDFTLTISSMQSEDLADYYCQQY SNYPLTFGQGTKLEIK |
| ch9B11-VL | 125 | DIVMTQSPLSLPVSLGDQASISCRSSQSLVHSNGNTYLHWYLQKPG QSPKLLIYKVSNRFSGVPDRFSGSGSGTDFTLKISRVEAEDLGVYFC SQSTHVPLTFGAGTKLEQKR |

In addition, the exemplified antibodies comprising a combination of a heavy chain variable region and a light chain variable region, and a constant region of an antibody or antigen-binding fragment according to an embodiment are described in Table 9.

**[Table 9]**

| **sample Name** | **chain** | **Amino acid sequence** |
|---|---|---|
| hu11F11(ver.1) | heavy (SEQ ID NO: 127) | EVQLVESGGGLVQPGGSLRLSCATSGFTFSDFYMEWVRQPPGK RLEWIAASRNKANDYTTEYSASVKGRFTISRDDSKSSLYLQMN SLRAEDTAIYYCARDAHGKPFAYWGQGTTVTVSSASTKGPSVF PLAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHE ALHNHYTQKSLSLSPGK |
| | light (SEQ ID NO: 128 | DIVMTQSPSSLAVSLGERVTMSCKSSQSLLYSSNQKNYLAWYQ QKPGQSPKLLIYWASTRESGVPDRFTGSGSGTDFTLTISSVKAE DVAVYYCQQYYSYPWTFGGGTKLEIKRTVAAPSVFIFPPSDEQL KSGTASVVCLLNNFYPREAKVQWKVDNALQSGNSQESVTEQD SKDSTYSLSSTLTLSKADYEKHKVYACEVTHQGLSSPVTKSFNR |
| | | GEC |
| hu11F11(ver2) | heavy (SEQ ID NO: 129 | EVQLVESGGGLVQPGGSLRLSCATSGFTFSDFYMEWVRQPPGK RLEWIAASRNKANDYTTEYSASVKGRFTVSRDDSKSSLYLQMN SLRAEDTAIYYCARDAHGKPFAYWGQGTTVTVSSASTKGPSVF PLAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHE ALHNHYTQKSLSLSPGK |
| | light (SEQ ID NO: 130 | DIVMTQSPSSLAVSLGERVTMSCKSSQSLLYSSNQKNYLAWYQ QKPGQSPKLLIYWASTRESGVPDRFTGSGSGTDFTLTISSVKAE DVAVYYCQQYYSYPWTFGGGTKLEIKRTVAAPSVFIFPPSDEQL KSGTASVVCLLNNFYPREAKVQWKVDNALQSGNSQESVTEQD SKDSTYSLSSTLTLSKADYEKHKVYACEVTHQGLSSPVTKSFNR GEC |
| hu11F11(ver.3) | heavy (SEQ ID NO: 131) | EVQLVESGGGLVQPGGSLRLSCATSGFTFSDFYMEWVRQPPGK RLEWIAASRNKANDYTTEYSASVKGRFTISRDTSKSSLYLQMNS LRAEDTAIYYCARDAHGKPFAYWGQGTTVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHE ALHNHYTQKSLSLSPGK |
| | light (SEQ ID NO: 132 | DIVMTQSPSSLAVSLGERVTMSCKSSQSLLYSSNQKNYLAWYQ QKPGQSPKLLIYWASTRESGVPDRFTGSGSGTDFTLTISSVKAE DVAVYYCQQYYSYPWTFGGGTKLEIKRTVAAPSVFIFPPSDEQL KSGTASVVCLLNNFYPREAKVQWKVDNALQSGNSQESVTEQD SKDSTYSLSSTLTLSKADYEKHKVYACEVTHQGLSSPVTKSFNR GEC |
| hu11F11(ver.4) | heavy (SEQ ID NO: 133 | EVQLVESGGGLVQPGGSLRLSCATSGFTFSDFYMEWVRQPPGK RLEWIAASRNKANDYTTEYSASVKGRFTVSRDTSKSSLYLQMN SLRAEDTAIYYCARDAHGKPFAYWGQGTTVTVSSASTKGPSVF |
| | | PLAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHE ALHNHYTQKSLSLSPGK |
| | light (SEQ ID NO: 134 | DIVMTQSPSSLAVSLGERVTMSCKSSQSLLYSSNQKNYLAWYQ QKPGQSPKLLIYWASTRESGVPDRFTGSGSGTDFTLTISSVKAE DVAVYYCQQYYSYPWTFGGGTKLEIKRTVAAPSVFIFPPSDEQL KSGTASVVCLLNNFYPREAKVQWKVDNALQSGNSQESVTEQD SKDSTYSLSSTLTLSKADYEKHKVYACEVTHQGLSSPVTKSFNR GEC |
| hu11F11(H2L4) | Heavy (SEQ ID NO: 135 | EVQLVESGGGLVQPGGSLRLSCAASGFTFSDFYMEWVRQAPG KGLEWIAAIRNKANDYTTEYAASVKGRFTISRDTSKNSLYLQM NSLKTEDTAVYYCARDAHGKPFAYWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK |
| | light (SEQ ID NO: 136 | DIQMTQSPSSLSASVGDRVTITCKSSQSLLYSSNQKNYLAWYQQ KPGKAPKLLIYWASTRESGVPSRFSGSGSGTDFTLTISSLQPEDF ATYYCQQYYSYPWTFGQGTKVEIKRTVAAPSVFIFPPSDEQLKS GTASVVCLLNNFYPREAKVQWKVDNALQSGNSQESVTEQDSK DSTYSLSSTLTLSKADYEKHKVYACEVTHQGLSSPVTKSFNRGE C |
| hu3A9(VH5/L3) | heavy (SEQ ID NO: 137) | EVQLQESGGGLVKPGGSLKLSCAASGFTFSSYAMSWVRQTPEK GLEWVATISNGGGYTYYPDSVKGRFTISRDNAKNTLYLQMNSL RSEDTAVYYCARHITTVRPTKYFDYWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISRT PEVTCVVVDVSHEDPEVKFNWYVDGVEVHNAKTKPREEQYNS |
| | | TYRVVSVLTVLHQDWLNGKEYKCKVSNKALPAPIEKTISKAKG QPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK |
| | light (SEQ ID NO: 138 | DIVMTQSPATLSVSLGERATLSCKASQNVGTTVAWYQQKPGQ APRLLIYSASNRYTGIPARFSGSGSGTEFTLTISSLQSEDFAVYYC QQYSNYPLTFGGGTKVEIKRTVAAPSVFIFPPSDEQLKSGTASV VCLLNNFYPREAKVQWKVDNALQSGNSQESVTEQDSKDSTYS LSSTLTLSKADYEKHKVYACEVTHQGLSSPVTKSFNRGEC |

In order that the heavy chain variable region and the light chain variable region described herein form the heavy chain and the light chain of the intact antibody, each heavy chain variable region and the light chain variable region may be bound to the heavy chain constant region and the light chain constant region. Each of heavy chain and light chain which are produced as described above can combine properly to constitute the combination of heavy chain-light chain, and the combination of heavy chain-light chain can form the multimer (for example, dimer for IgG type antibody), resulting in construction of the intact antibody structure.

The constant region may be appropriately selected from heavy and light chain constant regions of immunoglobulins (e.g., human immunoglobulins). For example, the heavy chain constant region may be an IgG1 heavy chain constant region, an IgG3 heavy chain constant region, or an IgG4 heavy chain constant region, and the light chain constant region may be a kappa constant region or a lambda constant region, but is not limited thereto. Other types of the constant regions or modified constant regions may be appropriately selected and used for antibody stability, manufacturability, antigen affinity, and/or other desired characteristics, which are clearly understood by those skilled in the art.

In other embodiment, each of the heavy chain variable regions and the light chain variable regions disclosed in Tables 7 and 8 can be freely combined with each other to form various antibodies, and are linked in a single chain form to produce single chain antibodies such as scFv.

The antibody described herein may share a specific region or sequence with a different antibody disclosed herein. In an embodiment, the antibody may share the constant region of the other antibody or antigen-binding fragment thereof. In another embodiment, it may share Fc region.

In an embodiment, the anti-alpha-synuclein antibody of the present invention also includes a monoclonal antibody and a polyclonal antibody. In another embodiment, the anti-alpha-synuclein antibody may be a human antibody, a humanized antibody, a chimeric antibody or an antibody driven from animal. In other embodiment, the anti-alpha-synuclein antibody can be prepared recombinantly or chemically synthesized.

The antigen-binding fragments or antibody fragments of antibodies described herein may be at least one selected from the group consisting of the single chain antibody molecules of scFv, (scFv)₂, scFv-Fc, Fab, Fab ', F (ab')₂, minibody, diabody, scFv and the like.

In other embodiment, the antibody provided herein may be a humanized antibody or human antibody and may be various isotypes (for examples, IgA, IgG1, IgG2, IgG3, IgG4, IgE, or IgD), particularly IgG1-, IgG2- IgG3- or IgG4-type, such as IgG1- or IgG2-types.

In another embodiment, the anti-alpha-synuclein antibody may comprise only heavy chain or light chain described above. In other embodiment, the anti-alpha-synuclein antibody has only heavy chain variable region or only light chain variable region.

Those skilled in the art will understand that when an antibody comprises one or more CDRs disclosed herein, each of the CDRs disclosed may be independently selected from each other and combined. Therefore, the antibodies can be prepared to include 1, 2, 3, 4, 5, or 6 of CDRs selected independently. It will also be understood by those skilled in the art that when a CDR is selected for combination, the same kind of CDR is not used repeatedly, and for example, an antibody is generally not produced to include two CDR-H2 regions.

In an embodiment, the antibody may be a monoclonal antibody or polyclonal antibody. The antibodies disclosed herein include monoclonal antibodies binding to alpha-synuclein. The monoclonal antibodies can be produced by using any technique known in the art. For example, it can be produced by immortalizing spleen cells harvested from immunized transgenic animals. The monoclonal antibodies secreted by hybridoma cell lines can be purified using techniques known in the art.

In other embodiments, the antibody may be an animal-derived antibody (e.g., mouse antibody, etc.), a chimeric antibody (e.g., mouse-human chimeric antibody), a humanized antibody, or a human antibody. The antibody can also be modified in a various ways for various purposes. The chimeric antibodies and the humanized antibodies are also provided. The chimeric antibodies are antibodies in which polypeptide fragments derived from different antibodies are covalently linked to form an immunologically-functional light chain, heavy chain, or fragment thereof.

In such a monoclonal antibody, a specific amino acid residue, which typically constitutes the non-antigen recognition portion of the antibody, is modified to be homologous to the corresponding residue of the isotype corresponding to the human antibody. Humanization can be performed with various known methods, for example, substituting at least a portion of the rodent variable region with the corresponding region of a human antibody (U.S. Pat. Nos. 5,585,089 and 5,693,762; Jones et al. , Nature 1986, 321: 522-525; Riechmann et al., Nature 1988, 332: 323-27; Verhoeyen et al., Science 1988, 239: 1534-1536).

The complete human antibody may be produced by immunizing a transgenic animal (commonly, mouse) which can produce a human antibody by lacking production of an endogenous immunoglobulin. The complete human antibody may be also derived from a phage display library. If the complete human antibodies are used, it can minimize the immunogenic and allergic reactions that can be caused by administration of a mouse or mouse-derived mAb to humans.

In an embodiment, the human alpha-synuclein antibodies of the present invention are selected through the phage display method. The monoclonal phage antibodies specific to alpha-synuclein which is selected through phage screening method, is inverted into full IgG form by using the recombinant method. The sequence of each monoclonal phage antibody is obtained for recombinantly producing anti-alpha-synuclein antibodies. After linking the heavy chain variable region sequence to the heavy chain constant region sequence and the light chain variable region sequence to the light chain constant region sequence in the obtained sequences, the amino acid sequences are converted into nucleotide sequence with the codon optimization method. After the obtained nucleotide sequences are cloned into vectors used for an animal cell culture, the host cells used for protein production such as CHO cells is transformed with the vectors and cultured. In order to purify the antibodies contained in the culture medium, the recombinant antibodies are separated and purified using a purification technique such as affinity chromatography.

In other embodiment, the antibody can have a typical structure of a naturally-occurring antibody, or modified structure.

The antibody having the typical structure may have a multimeric structure including structural units comprising two different polypeptide chains (i.e., heavy chain and light chain). The two different polypeptide chains comprise one full-length light chain (about 25kDa) and one full-length heavy chain (about 50 to 70kDa). Each chain shows a characteristic folding pattern, and consists of several immunoglobulin domains, consisting of about 90 to 110 amino acids. These domains are basic units consisting of an antibody polypeptide. The amino-terminal part of each chain typically comprises a part called a variable region or V region that is a part recognizing an antigen. The carboxy-terminal part is conserved evolutionarily more than the amino-terminal, and it comprises a part called a constant region or C region. The human light chain is commonly classified as kappa (κ) and lambda (λ) light chains, and these comprise one variable region and one constant region. The heavy chain is typically classified as mu (µ), delta (δ), gamma (γ), alpha (α) or epsilon (ε) chain, and these are defined as IgM, IgD, IgG, IgA and IgE isotypes, respectively. IgG includes IgG1, IgG2, IgG3 and IgG4, but unlimited numerous subtypes without limited thereto. The heavy chain constant region includes typically one or more domains showing an effector function. The number of the domains in heavy chain constant region varies depending on the isotypes. IgG heavy chain, for example, comprises three C region domains known as CHI, CH2 and CH3, respectively. The antibody disclosed herein may be any one of these isotypes and subtypes. In one embodiment, the antibody of the present invention is an IgG1, IgG2, IgG2a, IgG2b, IgG3 or IgG4 subtypes.

The antibody disclosed herein is also a variant of the antibody disclosed herein. For example, a part of antigen comprises conservative amino acid substitution in one or more of residues in the heavy chain or light chain, variable region or CDR sequence disclosed above. Those skilled in the art will determine an appropriate variant of the polypeptide disclosed herein using a known technique. Those skilled in the art will find a region being capable of changing a protein without destroying the activity, by targeting a region which is not considered as an important region for activity in the polypeptide.

The present invention also provides a derivative of the antibody disclosed herein. The derivatized antibody or its fragment may comprise any molecule or material providing desired properties, for example, an increased half-life in certain uses to the antibody or its fragment. The derivatized antibody may comprise, for example, a detectable (or labeling) residue (e.g., a radioactive, colorimetric, antigenic, or enzyme molecule), a detectable bead (e.g., magnetic or electron-dense (e.g., gold) bead), or a molecule binding to other molecules (e.g., biotin or streptavidin), a therapeutic or diagnostic residue (e.g., radioactive, cytotoxic, or pharmaceutically active residue), or a molecule increasing suitability of the antibody for special uses (for example, administration to a subject such as a human subject, or other in vivo or in vitro uses).

Other embodiment relates to a dimer comprising two fusion proteins in which the alpha-synuclein binding protein is fused to Fc region of an antibody. For example, the dimer may be prepared by inserting a fusion gene encoding a fusion protein into an appropriate expression vector, expressing the fusion gene in a host cell transformed by a recombinant expression vector, and allowing the expressed fusion protein to combine similarly to an antibody molecule, in which the interchain disulfide bond is formed between Fc residues to collect the dimer.

The term "Fc polypeptide" used herein is a polypeptide derived from an Fc region of an antibody, and includes a wildtype or mutant type. The cleavage form of polypeptide comprising a hinge region facilitating the dimerization is also included. The fusion protein comprising Fc or oligomer formed therefrom has an advantage of easy separation which is performed with an affinity chromatography using a protein A or protein G column.

In another aspect, the present invention also provides an isolated polynucleotide encoding the antibody or antigen-binding fragment according to the present invention. In another aspect, the present invention provides an expression vector comprising the polynucleotide according to the present invention. In another aspect, the present invention also provides a prokaryotic or eukaryotic cell or cell line transformed with the expression vector according to the present invention.

In another embodiment, the present invention further provides a method of producing an isolated antibody specifically binding to alpha-synuclein or antigen-binding fragment thereof, comprising culturing the cell of the present invention under the condition sufficiently expressing the antibody or an antigen-binding fragment thereof; and isolating the antibody or antigen-binding fragment thereof from the cell line.

In addition, the antibody or antigen-binding fragment disclosed herein has various useful applications. For example, it may be used for specific binding analysis, alpha-synuclein purification based on affinity, or screening method for alpha-synuclein antagonist investigation, and the like. In an embodiment, the antibody and antigen-binding fragment thereof can be useful for inhibiting alpha-synuclein aggregate formation, removing of alpha-synuclein aggregate in the nerve system, suppressing the intercellular transfer of the alpha-synuclein aggregates, and removing or reducing alpha-synuclein aggregates outside the cell by phagocytosis to the alpha-synuclein aggregate.

The antibody and antigen-binding fragment thereof of the present invention can be used for detection and/or treatment of various diseases associated with alpha-synuclein or alpha-synuclein aggregate. For examples, the antibody and antigen-binding fragment thereof can be useful for detection, treatment, alleviation, or improvement of the diseases, symptoms or disorder associated with alpha-synuclein aggregate. In addition, the antibodies according to the present disclosure can be used for diagnosis of various diseases or symptoms associated with alpha-synuclein or alpha-synuclein aggregates or for the presence or absence of alpha-synuclein or alpha-synuclein aggregates.

The antibody or antigen-binding fragment thereof of the present invention has at least an property selected from the group consisting of (i) specific binding to alpha-synuclein or alpha-synuclein aggregate, (ii) reduction of level of alpha-synuclein or alpha-synuclein aggregate in nerve system of a subject, (iii) reduction or inhibition of cell-to-cell transmission of alpha-synuclein or alpha-synuclein aggregate between the nerve cells, and (vi) increased phagocytic uptake to alpha-synuclein or alpha-synuclein aggregate in nerve cells. Preferably, the antibody or antigen-binding fragment thereof of the present invention has all the properties of (i) to (iv).

An embodiment of the present invention relates to a composition for prevention, alleviation, improvement or treatment of synucleinopathies, comprising an antibody or an antigen-binding fragment thereof being capable of specifically binding to alpha-synuclein, especially alpha-synuclein aggregate.

An embodiment of the present invention relates to a method of prevention, alleviation, improvement or treatment of synucleinopathies, comprising a step of administering to a subject in need, an antigen-binding fragment thereof being capable of specifically binding to alpha-synuclein, especially alpha-synuclein aggregate. The administered amount of the antigen or antigen-binding fragment thereof can be an effective amount to reduce the level of alpha-synuclein, especially alpha-synuclein aggregate in nerve system, more specifically extracellular region of the nerve cell in the nerve system of a subject.

An embodiment of the present invention relates to a method of reducing the level of alpha-synuclein, especially alpha-synuclein aggregate in nerve system, more specifically extracellular region of the nerve cell in the nerve system of a subject, or an use of reducing the level of alpha-synuclein, especially alpha-synuclein aggregate in extracellular region of the nerve cell in the nerve system, comprising an antibody or an antigen-binding fragment thereof being capable of specifically binding to alpha-synuclein, especially alpha-synuclein aggregate.

In another aspect, the present invention provides a composition or a kit comprising an antibody or an antigen-binding fragment thereof according to the present invention. The composition can be provided as a pharmaceutical use or a diagnosis use, and the pharmaceutical composition can further include a pharmaceutically acceptable carrier or excipient and the diagnosing composition can further include an agent required for the diagnosis or detection.

Herein, the term "blood brain barrier" or BBB is a barrier formed by tight junctions in the capillary endothelial membrane of the brain existing between the brain and spine and its surrounding circulatory system. This barrier is so sturdy that it also limits the passage of molecules having low molecular weight of about 60 Da to the brain. The blood brain barrier of the brain, the vascular spinal cord barrier of the spine and the vascular retinal barrier of the retina are continuous capillary barriers in the central nervous system, commonly referred to as BBB.

In the present invention, the term "blood brain barrier transporter" may pass through the blood brain barrier and deliver an antibody or an antigen-binding fragment thereof according to the present invention, and for example, include a protein including g peptide and polypeptide, a nucleic acid, an antibody, or a small molecular compound.

Herein, "therapeutic agent" means a molecule to be administered to a subject for a desired therapeutic effect. The subject includes a non-human mammal such as primates, or a human. The examples of the therapeutic agent include a protein comprising a peptide and a polypeptide, a nucleic acid, an antibody or a small molecular compound. In other aspect, the therapeutic agent can be used as a therapeutic agent of diseases related with alpha-synuclein aggregate by binding with the antibody of the present invention.

Although the accurate mechanisms by which the accumulation of alpha-synuclein in synucleinopathies induces typical properties of neurodegeneration and the symptoms inherent in synucleinopathies are not fully understood, the recent studies have shown that the abnormal formation and accumulation of alpha-synuclein aggregates are related to the occurrence of potential synucleinopathies and degenerative progression.

According to the present invention, the term "alpha-synucleinopathies" include all neurodegenerative disorders characterized by pathological synuclein aggregates. Parkinson's disease, Parkinson's disease dementia (PDD), Dementia with Lewy body (DLB), Lewy body disease, Dementia accompanied with Lewy bodies, Parkinson's syndrome with Dementia, Multiple system atrophy (MSA), multiple nervous system atrophy, and neurodegeneration type I with brain iron accumulation (NBIA Type I), are collectively grouped as synucleinopathies. In addition, the aggregations of alpha-synucleins have been also found secondary in Alzheimer's disease (Kim et al. Alzheimer's Research & Therapy 2014, 6:73).

The synucleinopathies are diverse groups of neurodegenerative disorders that share common pathological properties: In neuropathic aspects, the distinct lesions can be detected as the abnormal aggregation of alpha-synuclein in the selected groups of neurons and oligodendrocytes. Alpha-synuclein (formerly known as PARK1 and PARK4) is a protein comprised of 140 amino acids that is widely expressed in the neocortex, hippocampus, dentate gyrus, posterior neurosphere, striatum, thalamus and cerebellum. Alpha-synuclein is also highly expressed in hematopoietic cells including monocytes such as B cells T cells and NK cells and platelets. The accurate role in these cells has been unknown, but associated with differentiation of megakaryocytes (platelet precursors).

The alpha-synuclein antibody specifically recognizing to alpha-synuclein aggregate with high affinity according to the present invention, can be useful for diagnosis or detection of these diseases. Furthermore, the alpha-synuclein antibody specifically recognizing the alpha-synuclein aggregate of the present invention inhibits the formation of alpha-synuclein aggregates, degrades alpha-synuclein aggregates, or inhibits the intercellular transmission of aggregates, and thus can be used effectively for treating synucleinopathies such as Parkinson's disease.

Herein "a disease associated with alpha-synuclein aggregates" is a group of neurodegenerative diseases called as synucleinopathies, in which alpha-synuclein aggregates are found in lesions including neurons and glia, and has characteristics such as dopamine system degeneration, mobility change, cognitive impairment, and formation of Lewy bodies and/or Lewy Neurites (Kim et al. Alzheimer's Research & Therapy 2014, 6:73; McKeith et al., Neurology (1996) 47:1113-24). These diseases include Parkinson's disease, Parkinson's disease dementia, Lewy body dementia, Lewy body variant of Alzheimer's disease, combined Alzheimer's and Parkinson's disease, multiple system atrophy, and many other neuroaxonal diseases, but are not limited to. In one embodiment, the antibody according to the present invention is effectively used for treating Parkinson's disease.

Herein, "effective dose" commonly means an amount enough to reduce seriousness and/or occurrence frequency of symptoms due to a disease, particularly, a disease related to α-syn, remove symptoms due to a disease, particularly, a disease related to alpha-synuclein and/or a root cause of disease occurrence, or prevent occurrence of symptoms due to a disease, particularly, a disease related to alpha-synuclein and/or a root cause, and/or improve or correct damages due to a disease, particularly, a disease related to α-syn. In some embodiments, the effective dose is a therapeutic effective dose or a prophylactic effective dose. The "therapeutic effective dose" is an amount enough to treat a disease, particularly symptoms or conditions related to α-syn, or prevent, delay a disease, particularly symptoms or conditions related to α-syn, or reverse its progress. The "prophylactic effective dose" is an amount for prevent or delay occurrence or reoccurrence of a disease, particularly, a disease related to alpha-synuclein or symptoms of the disease, particularly, a disease related to α-syn, and reduce its probability. The complete therapeutic or prophylactic effect can be caused by several times of administration of dose, rather than by a single administration of dose. Therefore, the therapeutic or prophylactic effective dose may be delivered by once or more of administration.

In other aspect, the present invention provides the use of the antibody or antigen-binding fragment of the present invention or a composition comprising the same, for the treatment of the synucleinopathies of a subject suffering from synucleinopathies. The antibody or antigen-binding fragment or the composition comprising the same inhibits the intercellular transmission of alpha-synuclein aggregates, degrades alpha-synuclein aggregate, inhibits the formation of alpha-synuclein aggregates, or increases the phagocytes by microglia to alpha-synuclein aggregates in a subject, or in bran of subject.

Herein, the term "treatment" means all actions including alleviation or removal of an disease or symptom or condition of the disease, including reduction, relief, alleviation or removal of, disease or symptom of the disease, making a patient being able to withstand symptom or condition of the disease, or delaying the deterioration rate of disease or symptom or morbid condition of the disease. The term "subject" or "patient" includes a human or human patient.

A pharmaceutical composition comprising a therapeutically effective dose of the antibody and a pharmaceutically acceptable diluent, carrier, solubilizer, emulsifier, preservative and/or supplement is also provided. In addition, for example, a method for treating a cancer patient by administering such a pharmaceutical composition is included. The term "patient" includes a human patient related with α-syn. Once the pharmaceutical composition is formulated, this may be stored in a sterile vial as a solution, suspension, gel, emulsion, solid, crystal or dehydrated or lyophilized powder. This formulation may be stored in an immediately usable form or a form restructured just before administration (for example, lyophilized).

The administration route of the pharmaceutical composition may use known methods, for example, injection through oral; intravenous, intraperitoneal, intracerebral (interstitial), intraventricular, intramuscular, intraocular, intra-arterial, intraportal or intralesional route; sustained-release system or implant device. In a specific embodiment, the composition may be continuously administered by bolus injection, or injection or implant device.

The pharmaceutical composition or kit can be used for treating synucleinopathies, for examples Parkinson's disease, Parkinson's disease dementia, dementia with Lewy bodies, Lewy body variant of Alzheimer's disease, Combined Alzheimer's and Parkinson disease, and multiple system atrophy.

In another aspects, the present invention provide a method of treating synucleinopathy in a subject or modulating a concentration of alpha-synuclein aggregate in a subject, comprising a step of administering an antibody or antigen-binding fragment of the present invention or a composition comprising the same to a subject in need of treating α-synucleinopathy, and/or modulating a concentration of alpha-synuclein aggregates.

In one embodiment, an antibody according to the present disclosure may be used in a connected form to a transporter for passage through the brain blood barrier (BBB). Many methods for delivering medication through the brain blood barrier are disclosed. For example, there is a method of breaking down the osmotic pressure of the BBB by using a method such as Brad quinine or HIGU (Hign density foucues ultrasound). They also involve the use of cellular delivery system, for example, glucose and amino acid transport, and receptor-mediated transcytosis of insulin or transferrin, or the blocking of efflux transporter of glycoproteins.

In another embodiment, the antibodies according to the present invention can be used in combination with other therapeutic agents to treat diseases associated with alpha-synuclein aggregates. A pharmaceutical composition comprising a therapeutically effective dose of the antibody and a pharmaceutically acceptable diluent, carrier, solubilizer, emulsifier, preservative and/or supplement is also provided.

An acceptable formulation material is non-toxic to a recipient, in used capacity and concentration. In a specific embodiment, a pharmaceutical composition comprising a therapeutically effective dose of human alpha-synuclein antibody is provided. In a specific embodiment, the acceptable formulation material is preferably non-toxic in used capacity and concentration. In one embodiment, for example, the pharmaceutical composition may comprise a specific formulation material for pH, osmolality, viscosity, transparency, color, isotonicity, scent, sterility, stability, rate of dissolution or release, modification, maintenance or conservation of absorption or penetration, of the composition.

In a specific embodiment, the optimum pharmaceutical composition may be determined by those skilled in the art according to for example, a targeting administration route, delivery method and a targeting capacity (see, the above REMINGTON'S PHARMACEUTICAL SCIENCES). In a specific embodiment, this composition may affect the physical condition, stability, release rate in vivo and rate of clearance in vivo of the antibody disclosed herein. In a specific embodiment, the major vehicle or carrier in the pharmaceutical composition may be aqueous or non-aqueous. For example, an appropriate vehicle or carrier may be possibly injection water, physiological saline solution supplemented by common other materials in a composition for parenteral administration. Further, in a specific embodiment, the human alpha-synuclein antibody may be formulated as a lyophilizing using a proper excipient, for example, sucrose.

The pharmaceutical composition may be delivered parenterally, and the therapeutic composition can human alpha-synuclein binding protein in a pharmaceutically acceptable vehicle, or is provided in the form of an aqueous solution acceptable for parenteral that does not contain pyrogen.

Once the pharmaceutical composition is formulated, this may be stored in a sterile vial as a solution, suspension, gel, emulsion, solid, crystal or dehydrated or lyophilized powder. This formulation may be stored in an immediately usable form or a form restructured just before administration (for example, lyophilized). A kit for producing a single-dose administration unit is also provided. This kit comprises a primary container having a dried protein and a secondary container having an aqueous formulation. The administration frequency may be affected by pharmacokinetics parameters of the used formulation of human alpha-synuclein antibody.

In addition, it may be preferable to use the human alpha-synuclein antibody pharmaceutical composition ex vivo. In this case, a cell, tissue or organ removed from a patient may be exposed for the pharmaceutical composition of human alpha-synuclein antibody, and then be subsequently implanted into the patient. In particular, the human alpha-synuclein antibody may be delivered by implanting a specific cell which is genetically engineered by using methods described herein, in order to express and secret the polypeptide.

In another embodiment, the present invention provides a method of detecting alpha-synuclein aggregate in a biological sample, comprising providing an antibody or antigen-binding fragment according to the present invention, and contacting the antibody or antigen-binding fragment thereof with a biological sample to be detected for alpha-synuclein aggregate. The biological samples include various samples required for detection of the alpha-synuclein aggregate, including, for example, cerebrospinal fluid, blood including plasma or urine, and cells, tissues or organs. The method may be performed *In vitro* or *In vivo.* The *In vivo* imaging can be performed by using, for example, positron emission tomography (PET), single photon emission tomography (SPECT), near infrared (NIR) optical imaging or magnetic resonance imaging (MRI).

In another embodiment, the present invention provides a method of diagnosing synucleinopathy in a subject in need of diagnosing synucleinopathy, wherein the method comprises measuring/detecting a concentration or intercellular location of the alpha-synuclein aggregate in the subject using any antibody or antigen binding fragment thereof of the present invention; and comparing the concentration or the intercellular location of the alpha-synuclein aggregate measured in the subject with that of a control sample, and wherein the similarity or difference compared to the control sample result represents the subject with α-synucleinopathy. The control group can be normal sample or a sample of patient with α-synucleinopathy. For the detection or diagnosis use, the antibody can be typically labeled by a detectable labeling material.

The antibody disclosed herein may be usefully used for detection, diagnosis, or monitoring of diseases or symptoms related with alpha-synuclein. In one embodiment, the method of the present invention is a method of diagnosing synucleinopathy in a subject in need of diagnosing synucleinopathy, wherein the method comprises measuring a concentration or intercellular location of the alpha-synuclein aggregate in the subject using the antibody and antigen-binding fragments according to the present invention; and comparing the concentration or the intercellular location of the alpha-synuclein aggregate measured in the subject with the result of the control sample, and wherein the similarity or difference compared to the control sample result represents the subject with synucleinopathy.

In the method, the subject includes patients who have no symptoms or are before occurrence of symptoms. In one embodiment, the control group may be patient with synucleinopathy including Parkinson's disease (PD), Dementia with Lewy bodies (DLB), or multiple system atrophy (MSA), and the similarity to the control group in the comparison step represents the diagnosis the subject as a patient with synucleinopathy. In another embodiment, when the control group is a sample derived from a normal subject, the difference from the control group in the comparison step, for example the increased concentrate of aggregates represents the diagnosis the subject as a patient with synucleinopathy. In another embodiment, the age of the subject and the control can be matched. The method may be performed *in vivo* or in a biological sample separated from subject, such as blood, cerebrospinal fluid (CSF), or urine samples.

For the diagnosis use, the antibody may be typically labeled with a detectable labeling substance. The appropriate labeling substances include a radioisotope or radioactive nuclide (for example, ³H, ¹⁴C, ¹⁵N, ³⁵S, ⁹⁰Y, ⁹⁹Tc, ¹¹¹In, ¹²⁵I, ¹³¹I), fluorescent material (for example, FITC, rhodamine, lanthanoid fluorescent substance), enzyme (for example, horse radish peroxidase, β-galactosidase, luciferase, alkaline phosphatase), a chemiluminescent group, a biotinyl group, or polypeptide epitope recognized by a secondary reporter (for example, leucine zipper pair sequence, secondary antibody binding site, metal binding domain, epitope tag), but not limited thereto.

In other aspect, the antibody of the present invention may be used for identification of a tissue including alpha-synuclein aggregate. In a specific embodiment, the antibody is labeled by a labeling substance, and the binding to alpha-synuclein aggregate of the labeled antibody is detected. In one embodiment, the binding of the antibody to alpha-synuclein aggregate is detected in vivo.

In other aspect, the present invention discloses detecting or selecting test materials competing with the antibody disclosed herein for binding to alpha-synuclein aggregate. For example, a step of detecting the amount of unbound antibodies in a solution comprising alpha-synuclein aggregate in the presence or absence of test materials is comprised.

The increased concentration of the free antibody, that is, the antibody which is not bound to alpha-synuclein aggregate may represent that the test materials can compete with the antibody for binding to alpha-synuclein. In one embodiment, the antibody is labeled by a labeling group. Alternatively, the test materials are labeled and the amount of free test materials is monitored by the presence or absence of the antibody.

In addition to that, the antibody or antigen-binding fragment disclosed herein has various useful applications. For example, it may be used for specific binding analysis, alpha-synuclein purification based on affinity, or screening method for investigating alpha-synuclein antagonist and the like.

The antibodies disclosed herein are useful for the detection of alpha-synuclein, particularly alpha-synuclein aggregates such as Lewy body in a biological sample, and the identification of cells or tissues containing alpha-synuclein aggregate. For example, an ant-alpha-synuclein antibody can be used for diagnosis, for example, the detection and/or quantitative analysis of alpha-synuclein aggregates in a biological sample such as blood containing serum, cerebrospinal fluid (CSF), or urine, tissue or cells, and the diagnosis of α-synucleinopathy based thereon.

### [EFFECT OF INVENTION]

The antibodies disclosed herein preferentially bind to alpha-synuclein, especially alpha-synuclein aggregates with a high binding affinity. The antibody or antigen-binding fragment thereof having a high affinity according to the present invention can reduce alpha-synuclein aggregate formation, thereby lowering the concentration of aggregates in the brain. In addition, the antibody or antigen-binding fragment thereof having a high affinity for alpha-synuclein aggregates according to the present invention can reduce the formation of alpha-synuclein aggregates outside the central nervous system, and change the equilibrium state of alpha-synuclein forms bounded by the blood brain barrier, resulting in the effect of lowering the concentration of aggregates inside the central nervous system. In addition, it has the advantage that it can be administered at low dosages due to its high affinity. Since sufficient efficacy can be obtained, for example, by simpler subcutaneous injection, but not limited thereto, it has a great advantage in clinical practice.

The antibodies disclosed herein can effectively remove or promote degradation of alpha-synuclein aggregates, and inhibit intercellular transmission of alpha-synuclein, and thus can be usefully applied for the treatment of diseases associated with accumulation of alpha-synuclein aggregate.

### [BRIEF DESCRIPTION OF THE DRAWING]

Fig. 1 is a dot-blot result showing that the monoclonal antibody prepared in an embodiment of the present invention specifically binds to the native alpha-synuclein in the aggregated form.
Fig. 2 is a result of ELISA analysis for the affinity of the monoclonal antibody prepared in an embodiment of the present invention.
Fig. 3a is a result of BIAcore analysis for the specificity and affinity of the preferential binding of the monoclonal antibody prepared in an embodiment of the present invention to alpha-synuclein aggregate. Fig. 3b is a table showing the results of Fig. 3a.
Fig. 4a is a result of Octet analysis for the preferential binding specificity of the monoclonal antibody prepared in an embodiment of the present invention to the alpha-synuclein aggregate. Fig. 4b is a table showing the results of Fig. 4a.
Fig. 5 shows the principle analyzing whether the monoclonal antibody prepared in an embodiment of the present invention inhibits the cell to cell transmission (upper part), and the experimental result.
Fig. 6a is a result of staining and measuring the mouse brain tissue by using p-129 α-Syn antibody after administering antibody to mouse, in order to test whether the monoclonal antibody prepared in an embodiment of the present invention can remove the alpha-synuclein aggregate in the mouse model (TG) overexpressing human alpha-synuclein. In the drawing, HP refers to Hippocampus, p-129 α-syn refers to a form having the phosphorylated 129th residue and a maker for aggregate, and the arrow indicates the phosphorylated alpha-synuclein.
Fig. 6b is a result of staining and measuring according to the substantially same experiment as Fig. 6a by staining with the antibody against all alpha-synucleins as a maker. The arrow indicates human alpha-synuclein. The antibodies 9B11, 11F4, and 11F11 inhibited the accumulation of all alpha-synuclein.
Fig. 7a is a result of staining and measuring the mouse brain tissue with Iba-1(microgliosis) antibody as a marker after administering antibody to mouse, in order to test whether the monoclonal antibody prepared in an embodiment of the present invention can reduce microgliosis in vivo, and the arrow indicates the activated microglia.
Fig. 7b is a result of staining and measuring the mouse brain tissue with GFAP (astrogliosis) antibody as a marker after administering antibody to mouse, in order to test whether the monoclonal antibody prepared in an embodiment of the present invention can reduce astrogliosis in vivo, and the arrow indicates the activated astrocyte.
Fig. 7c is a result of staining and measuring the mouse brain tissue with IL-1 beta (IL-1β) antibody as a marker after administering antibody to mouse, in order to test whether the monoclonal antibody prepared in an embodiment of the present invention can reduce the inflammatory cytokines in vivo, and the arrow indicates the cells expressing IL-1 beta.
Fig. 7d is a result of staining and measuring the mouse brain tissue with IL-6 antibody as a marker after administering antibody to mouse, in order to test whether the monoclonal antibody prepared in an embodiment of the present invention can reduce the inflammatory cytokines in vivo, and the arrow indicates the cells expressing IL-6.
Fig. 8a and Fig. 8b show the result of measuring that the monoclonal antibodies of 3A9 and 11F11 in an embodiment of the present invention can specifically recognize the Lewy bodies and Lewy neurites in human brain tissue, respectively. It shows that the antibodies of the present invention bind to the Lewy bodies (arrow) and the Lewy neurites (thread-like shape in lower left part).
Fig. 9 is a schematic representation of the epitope mapping results for the antibodies of an embodiment of the present invention, in which the antibodies of present invention bind mostly to the C-terminal region.
Fig. 10a and Fig. 10b is a result of analyzing the activity of the chimeric antibody and the humanized antibody to promote the phagocytic action of microglial cells to alpha-synuclein aggregates, and Fig.10a refers to 11F11 humanized antibody and Fig. 10b refers to 3A9 humanized antibody.
Fig. 11 is a result of analyzing the activity of the chimeric antibody and the humanized antibody to inhibit the binding of alpha-synuclein aggregates to the surface of nerve cells.
FIG. 12 is a result of analyzing the efficacy of chimeric antibodies and humanized antibodies of the present invention to inhibit the cell-to-cell transmission in which the alpha-synuclein aggregates are secreted from nerve cells overexpressing alpha-synuclein and transfer to normal nerve cells.
Fig. 13 is a result of ELISA analysis of the affinity of the chimeric antibody and the 11F11 humanized antibody prepared in an embodiment of the present invention.
Fig. 14 is a result of BIAcore analysis for the specificity and affinity of the preferential binding of the chimeric antibody and the 11F11 humanized antibody prepared in an embodiment of the present invention to alpha-synuclein aggregates.
Fig. 15a, Fig. 15b and Fig. 15c are dot-blot results showing that the chimeric antibody prepared in an embodiment of the present invention specifically binds to alpha-synuclein, especially alpha-synuclein aggregate, but not beta-synuclein, gamma-synuclein, or aggregates derived from amyloid beta₁₋₄₂, or tau.

### [MODE FOR INVENTION]

Hereinafter, the present invention will be described in more detail with reference to examples, but these are merely illustrative and are not intended to limit the scope of the present invention.

### Example 1: Preparation of alpha-synuclein antibody

### Immunization

Alpha-synuclein monomer with a full length (140 residues) or cleaved with C-terminal 21 residues (119 residues) were placed in a thermomixer at 37 °C, aggregated with shaking at 1050 rpm for 14 days, and sonicated. Each of 140 residues and 119 residues of the α-syn fibril at 1 mg / ml was mixed with the adjuvant at a ratio of 1: 1 (vol: vol).

| Homo sapiens alpha-synuclein | Amino acid sequence |
|---|---|
| SEQ ID NO:126 | |

Then, 200 µL of the prepared mixture was injected subcutaneously into 5 to 7 week old BALB/c female mice. After 2 weeks, 200 µL of the prepared mixture was further injected subcutaneously for antibody boosting. After one week of boosting, blood was collected and immunization titration was performed by the ELISA method using the administered antigen. Subsequently, third boosting was performed by subcutaneous injection of antigen alone.

### Hybridoma production

The spleen of the immunized mouse was removed, and the spleen cells were obtained from the spleen. The spleen cells were suspended in Hybridoma-SFM medium (Thermo Fisher Scientific, USA) supplemented with 10% FBS. To prepare the hybridoma, the spleen cells and SP2/0-Ag14 of a murine myeloma cell were mixed in a Hybridoma-SFM medium without serum, and followed by centrifugation to remove the medium. Then, PEG was added to the obtained cell pellet and incubated at 37 °C for 1 minute to induce cell fusion.

### Single cell cloning

After 2 weeks in the fusion, the fusion with mouse B cells producing antibodies was confirmed with an ELISA method using the antigen administered to the mouse and a cell culture medium. Then, single-cell cloning was carried out using a hybridoma to select 16 hybridomas producing monoclonal antibodies. 9B11 clones (IgG1 kappa) were obtained using the aggregate of full length (140 residues) α-Syn as an antigen, and Clones of 3A9 and 11F11 (IgG2b kappa, and IgG2b kappa, respectively) were obtained using α-Syn aggregates with cleaved C-terminal 21 residues as antigens.

### Purification of antibodies

In order to purifying the antibody, each hybridoma was cultured in RPMI1640 medium containing 10% FBS. For antibody production, the culture medium was replaced with serum-free SFM medium and cultured for about 4 days. The cell culture supernatant was separated, centrifuged, filtered with a 0.22 µm filter, and purified with a protein G column for IgG1 type and the protein A column for the remaining antibodies.

### Determination of variable region sequence

The variable region and CDR sequences were determined by referring to the disclosure Ahn et of al, Mol. Cells 2004, 18 (2): 237-241. Hybridomas were cultured and centrifuged to isolate only the cells. The RNA was isolated from the isolated hybridoma by the addition of a triazole and was used for synthesize cDNA as a template. The variable region and CDR sequence were confirmed by sequencing.

### Example 2. Analysis of antigen binding specificity and binding affinity using alpha-synuclein antibody

### 2-1: Dot blot analysis using anti-alpha-synuclein antibody

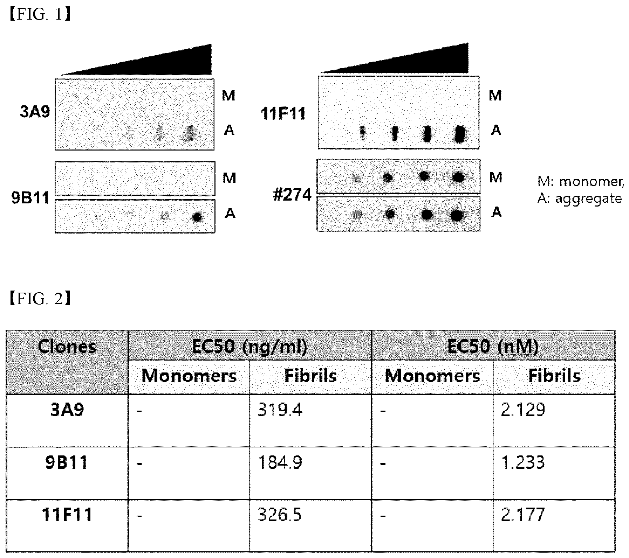

Dot blot experiments were performed to analyze whether the antibody of the present invention bound to monomers or aggregates in the native state. For the experiment, 50 ng or 100 ng of α-syn monomer or fibrin protein (manufactured by Professor Lee Seung-jae of Seoul National University; Bae et al., J. Neurosci 32: 13454, 2012) were spot-loaded on the nitrocellulose membrane. Twice-fold diluted monomer or fibril proteins were loaded sequentially from the right side of the membrane to the left (12.5, 25, 50, 100 ng). The membrane was blocked with 5% non-fat dry milk of TBST composition for 1 hour at room temperature. 1 mg/ml of the α-syn antibody prepared in Example 1 was added to TBST containing 1% bovine serum albumin and were incubated at room temperature for 1 hour. After washing with TBST, signals were analyzed using a chemiluminscence substrate (NEN) as substrate and secondary antibody conjugated with HRP (horse radish peroxidase) according to the manufacturer's manual. The results were imaged using a LAS-3000 Luminescent Image Analysis System (FUJIFILM Life Science). The results are shown in FIG. 1.

As shown in Fig. 1, the alpha-synuclein antibody of the present invention was found to preferentially bind only to aggregates as compared to alpha-synuclein monomers. Particularly, 9B11, 3A9 and 11F11 bound only to the aggregate. 274 antibody (Bae et al., J Neurosci. 2012 Sep 26; 32 (39): 13454-13469) was used as a comparative antibody that binds to both monomers and aggregates.

### 2-2: ELISA analysis using monoclonal anti-alpha-synuclein antibody

ELISA analysis was performed to quantitatively analyze the binding affinity of the antibody of the present invention to the antigen. For this, alpha-synuclein antibody obtained in Example 1 was coated on a 96-well plate at a concentration of 1 ug/ml and treated with alpha, synuclein fibril aggregates at 10, 100, 1000 and 10,000 ng/ml. After washing with PBS, streptavidin conjugated with HRP and secondary antibody conjugate with biotin was treated and then reacted with TMB as a substrate. The absorbance was measured and the results are shown in FIG.2.

As shown in Fig. 2, the antibodies of the present invention were found to preferentially bind to aggregates with a high binding affinity. The ELISA results showed that the antibodies binding preferentially to aggregates have the affinity of 0.1 x 10⁻⁹ M to 2 x 10⁻⁹ M, while the antibodies binding to both monomers and aggregates showed higher affinity of ∼ 1 x 10⁻¹⁰ M than these antibodies. The antibody of the present invention preferentially bound to alpha-synuclein aggregates with a high affinity, but the affinity for the monomer could not be obtained, because it bound to monomer with a lower affinity than aggregate or did not bind to monomer. These results indicate that the antibody can remove effectively or inhibit the acting of the agent causing neurodegenerative diseases associated with alpha synuclein etiology such as Parkinson's disease.

### 2-3. BIAcore analysis using anti-alpha-synuclein antibody

The quantitative analysis of the binding of alpha-synuclein antibody prepared in Example 1 to monomer and aggregate antigens was performed using BIAcore analysis.

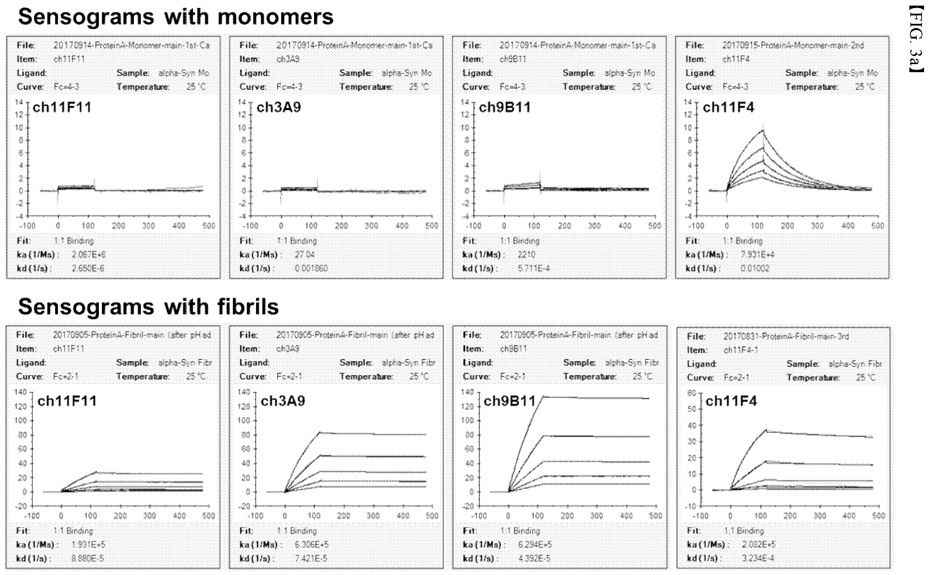

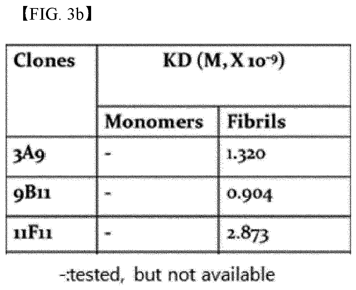

The used instrument was T200 (GE Healthcare, S / N: 1565888). Protein A is used as a chip (GE Healthcare, Cat. 29-1275-56). 10 mM Glycine-HCl pH 1.5 (GE Healthcare, Cat. BR-1003-54) was regeneration buffer. The running buffer, analyte dilution, and the sample dilution buffer were HBS-EP. The α-syn antibodies (3A9, 9B11 and 11F11) prepared in Example 1 were diluted with 1 × HBS-EP (GE Healthcare, Cat. BR-1006-69), and alpha-synuclein monomer (1 mg/ml) and fibril protein (3 mg / ml) were serially diluted in duplicate and analyzed at 6 concentrations (0, 0.39, 1.56, 6.25, 25, 100 nM) including 0 nM in total. For the capture, the monomer was for RU of 800 (theoretical), and a fibril was for RU of 100 (theoretical). The capture phase was performed at contact time of 60 seconds, a flow rate of 30 µl / min, and a stabilization period of 180 seconds. The association phase was performed at the association time of 120 seconds and the flow rate was 30 µl/min. The dissociation phase was performed at the dissociation time of 360 seconds and the flow rate of 30 µl/min. The regeneration phase was performed twice time at the regeneration time of 240 seconds (primary) and 60 seconds (secondary) and a flow rate of 30 µl/min. The fitting was carried out suing 1: 1 binding model, and the evaluation software was BIACore T200 Evaluation software (GE healthcare). The results are shown in Figs. 3a and 3b.

Fig. 3a is a result of BIAcore analysis of the preferential binding specificity and affinity of the monoclonal antibody prepared in an example of the present invention to the alpha-synuclein aggregates. It shows that the antibody of the present invention binds to the aggregate with a high affinity. These results indicate that the antibody can remove effectively or inhibit the acting of the agent causing neurodegenerative diseases associated with alpha synuclein etiology such as Parkinson's disease. Fig. 3b is a table showing the results of Fig. 3a.

As shown in Fig. 3a and Fig. 3b, 3A9, 9B11 and 11F11 of which the preferential binding to aggregates was confirmed among analyzed four alpha-synuclein antibodies by the other methods described above, bound only to aggregates in BIAcore analysis as a high affinity of about 1 x 10 ⁻⁹ M to 3 x 10 ⁻⁹ M of high affinity.

### 2-4. Octet analysis using anti-alpha-synuclein antibody

The quantitative analysis of the binding of the alpha-synuclein antibodies (3A9, 9B11, 11F11) prepared in Example 1 to monomer and aggregate antigens was performed using Octet.

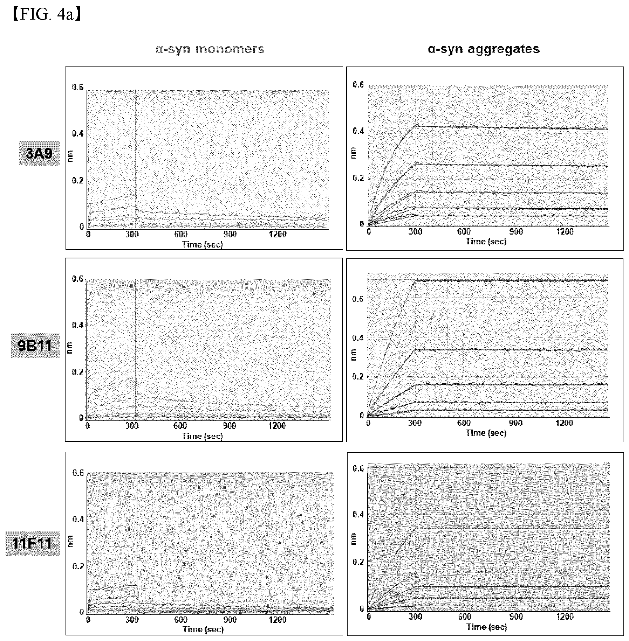

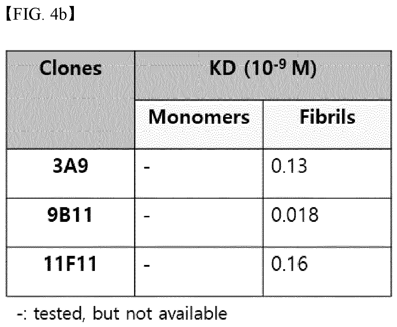

Specifically, the running buffer was 1 × KB buffer (cat. 18-1092) or 1 × PBS buffer at 1000 rpm, and the immobilization buffer was sodium acetate, pH 5 (10 mM, Cat 18-1068. α-syn monomers was immobilized the α-syn antigen, and fibrils was immobilized the test antibody. The target concentrations were 20 µg/ml for the monomer and 0.4 µg/ml for the fibril. The kinetics concentration was diluted sequentially two times from 50 nM for monomers and from 100 nM for fibrils, to give 7 points in total, respectively. Association/dissociation time was 5 min / 20 min for monomer and 5 min / 25 min for fibril. Biosensor was ARG2 and fitting was performed using 1: 1 fitting model. The results are shown in FIG. 4. Fig. 4a is Octet analysis result for the preferential binding specificity of the alpha-synuclein antibody of an embodiment of the present invention binds preferentially to the α-Syn aggregate.

As shown in Fig. 4a, 3A9, 9B11 and 11F11 had been shown to hardly bind to monomers (red dotted box), but bind well to the aggregates (ascending line graph in red dotted box). These results are similar to or consistent with the results in Dot blot, Octet and ELISA analyses, and show that 3A9, 9B11 and 11F11 of which the preferential binding to aggregates are confirmed among analyzed four alpha-synuclein antibodies by the other methods described above, binds only to aggregates in Octet analysis. Fig. 4b is a table showing the results of Fig. 4a. The results of FIG. 4a and FIG. 4b show the preferential binding to alpha-synuclein aggregates, and are consistent with the results of FIG. 1. The # 274 antibody used as a control group was found to bind well to both monomers and aggregates.

### Example 3. Inhibitory effect analysis of anti-alpha-synuclein antibody on intercellular transfer of alpha-synuclein aggregate

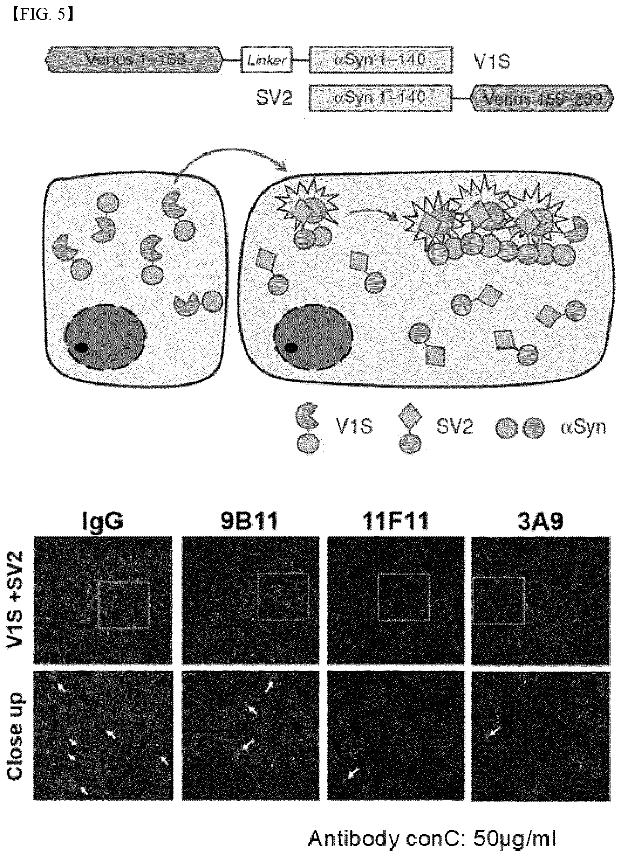

Since the intercellular transfer of alpha-synuclein aggregates is presented as a pathogenic cause of diseases related with alpha-synuclein aggregate, the antibodies being capable of inhibiting it may be useful as therapeutic agents. For this purpose, bimolecular fluorescence complementation (BiFC) analysis was performed as follows. The BiFC principle is shown schematically in the upper part of Fig.5.

Fig. 5 shows the principle analyzing (upper part) whether the monoclonal antibody prepared in an embodiment of the present invention inhibits the cell to cell transmission, and the experimental result. In the drawing, the blue color indicates the nucleus, and the green color is the signal produced when the alpha-synuclein released from outside one cell propagates into inside other cells and meets other alpha-synuclein to form aggregates.

### Cell culture for BiFC analysis

SH-SY5Y human neuroblastoma cell lines expressing alpha-synuclein and a half of Venus fluorescent proteins (Venus 1-αSyn (V1S) and αSyn-Venus2 (SV2)), respectively, were cultured as previously described (Lee HJ et al., J. Neurosci. 2004; 24: 1888-1896), Prior to the experiment, 180,000 cells expressing VIS and SV2 were mixed on a cover slip and cultured for 3 days. Co-culture was sub-cultured every 48 hours to confirm the continuous intercellular migration of alpha-synuclein. Because the degree of transfer was maximum at the passage number of 6 in a separate experiment (data not shown), the inhibitory effect of the antibody on the cell-to-cell transport was analyzed using the co-culture of six passages.

### BiFC experiment / antibody treatment

On the day before imaging, 50 µg / ml of IgG or test antibody (3A9, 9B11, 11F11) was added to the co-culture. The signal in the Venus channel in each co-culture represents the aggregates produced by the aggregation of alpha-synuclein, which was considered as the aggregate formed by the migration of α-syn from one cell to another cell. These signals were automatically analyzed with the IN Cell Analyzer (instrument setup: puncta size: 0.1-0.4 µm, intensity: 4000-7000). The results are shown in Fig. 5.

Specifically, the blue color indicates the nucleus, and the green color indicates that released from outside one cell propagates into inside other cells and meets other alpha-synuclein to form aggregates. The graph on the right side shows the intensity of the signal. In 9B11, 11F11, and 3A9 treatment groups, the number of cells showing a green signal was decreased compared to the negative control IgG. The result indicates that the antibodies of present invention can effectively inhibit intercellular transmission of alpha-synuclein.

### Example 4. Analysis of in vivo removal effect of alpha-synuclein aggregates by anti- alpha-synuclein antibody

In order to analyze the effect of the alpha-synuclein antibody produced in the present invention *in vivo,* 10 mg/kg of human alpha-synuclein or IgG was administered intraperitoneally every week for 3 months to transgenic mouse overexpressing human alpha-synuclein (mThy-1 human alpha-synuclein, UC San Diego). Six mice per a group were used, and non-transgenic littermates were used as control group. The, the perfusion was performed as follows.

For pathological analysis of brain, after completion of the last administration, the animals were anesthetized with chloral hydrate according to humanitarian regulations and then heart perfusion was performed with 0.9% saline. One half (saggital section) of the perfused brain was stored in 4% paraformaldehyde in phosphate buffer (pH 7.4, 4 °C) until subsequent analysis and the other half was stored at frozen (70 °C) immediately.

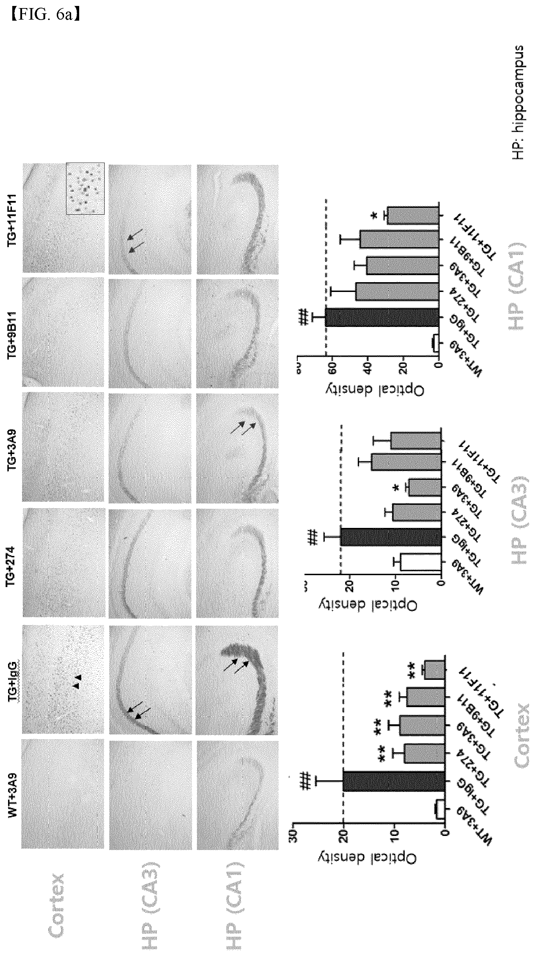

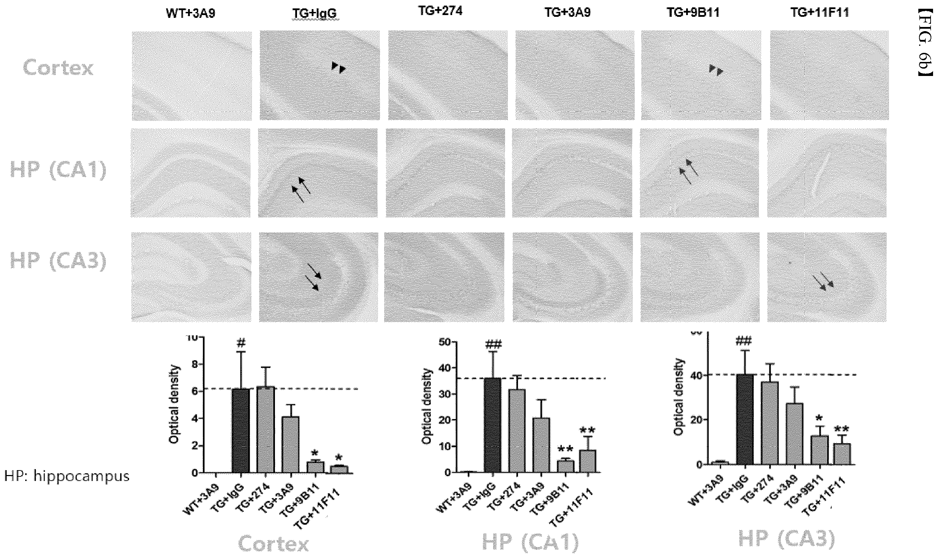

Pathological analysis was performed as follows. The half section of brain fixed in paraformaldehyde was cut into the continuous sections in 40 µm thick by free-floating method using a vibratome. To investigate the expression level of alpha-synuclein in the brain of the administered group, the section containing cortex, striatum and hippocampus were incubated with alpha-synuclein antibodies (p129 α-syn antibody as a maker of aggregate, abcam, ab59264 or all alpha-synuclein antibody, Cell Signaling Technology, # 2642) overnight at 4 °C. To investigate the activity of astrocytes or the activity of microglia, the section were treated with GFAP antibody (glial fibrillary acidic protein)(AB5804, millipore) or Ibal antibody (019-19741, Wako) (019-19741, Wako), respectively. In order to intestigate the degree of neuroinflammation, the section was treated with IL-6 antibody (NB600-1131, Novus Biologicals) or IL-1β antibody (ab9722, abcam), respectively. After incubation with the primary antibodies, biotin-conjugated goat anti-rabbit IgG (1: 100, Vector Laboratories) and Avidin D-horseradish peroxidase (1: 200, ABC Elite, Vector Laboratories) were treated and detected with diaminobenzidine (DAB). Each immunostained sections were observed with a bright field microscope to measure optical density. The results are shown in Fig. 6a and 6b. Fig. 6a is a result of staining and measuring the mouse brain tissue by using p-129 α-Syn antibody after administering antibody to mouse, in order to test whether the monoclonal antibody prepared in an embodiment of the present invention can remove the alpha-synuclein aggregate in the mouse model (TG) overexpressing human alpha-synuclein. In the drawing, HP refers to Hippocampus, p-129 α-syn refers to a form having the phosphorylated 129th residue and a maker for aggregate, and the arrow indicates the phosphorylated alpha-synuclein.

Fig. 6a is a result of analysis using p-129 α-Syn antibody as a maker for detecting alpha-synuclein aggregate and shows that the aggregate level has been decreased significantly in CA1 and CA3 of cortex and hippocampus of TG (TransGenic) mouse administered by the antibody prepared in an embodiment of the present invention, compared to the control mouse administered by only IgG (the relevant part is indicated as arrowhead in IgG sample). These results represents that the antibody of the present invention can significantly remove alpha-synuclein. WT and IgG are negative controls, and 274 antibody is a comparative group binding to both of monomer and aggregate. These results represents that the antibody of the present invention can effectively inhibit the accumulation of alpha-synuclein aggregate, and thus can be effectively used for the prevention and/or treatment of diseases associated with alpha-synuclein etiology.

Fig. 6b is a result of the substantially same experiment as Fig. 6a, but it is obtained by staining with antibody against all alpha-synucleins as a maker. The arrow indicates human alpha-synuclein. Increased human alpha-synuclein in TG mouse was effectively removed by the administration of the antibodies. These results indicate that the antibody according to the present invention effectively reduces alpha-synuclein aggregate and can be effectively used for the treatment of α-synucleinopathy such as Parkinson's disease. 9B11, 11F4, and 11F11 antibodies effectively inhibited the accumulation of all alpha-synucleins. The detection of all alpha-synuclein indicates that the antibody according to the present invention has the ability to clear the alpha-synuclein itself (clearing) and inhibit the intercellular transfer (cell to cell transmission). In another aspect, it can also be interpreted as inhibiting the formation of aggregates from monomers or removing all monomers.

### Example 5: Analysis for the reduction of microgliosis and astrogliosis and reduction of inflammatory cytokine release by anti-alpha-synuclein antibody

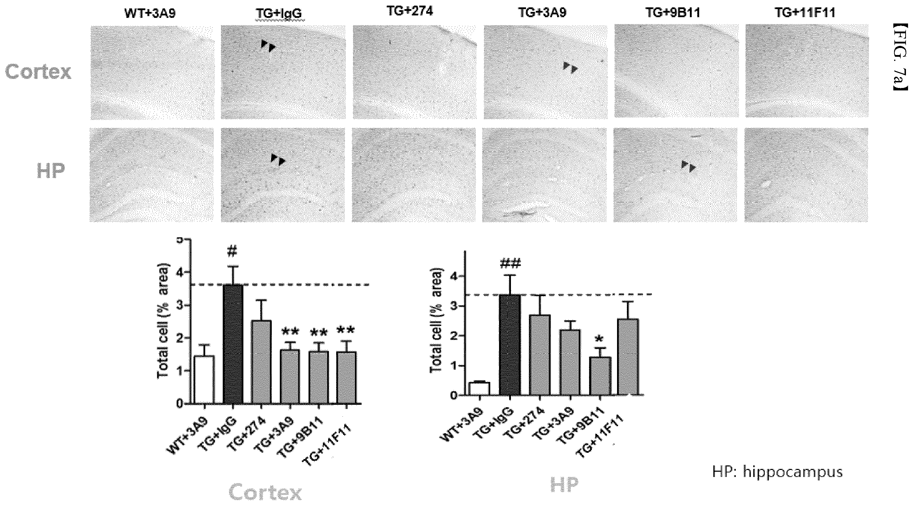

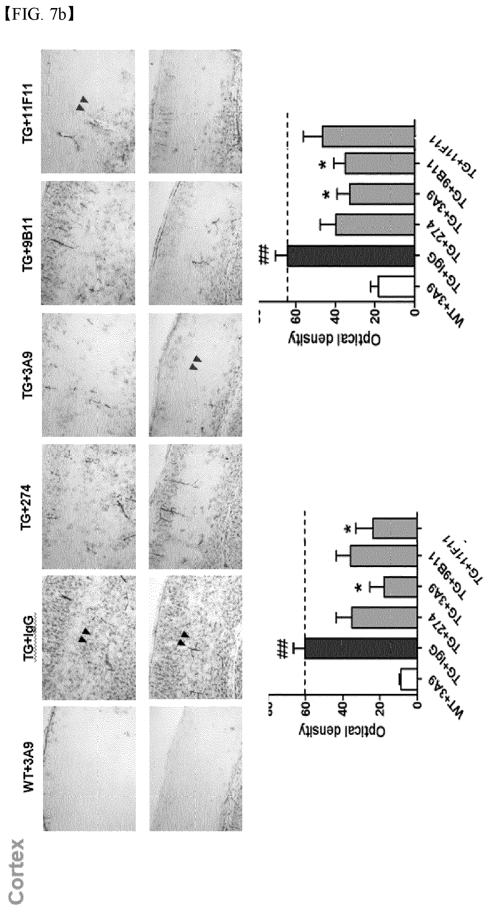

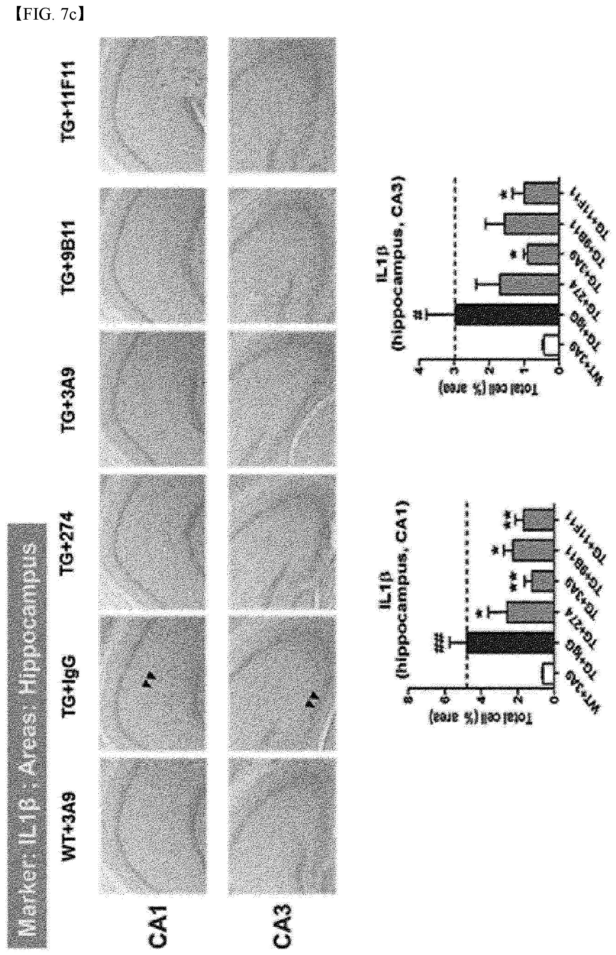

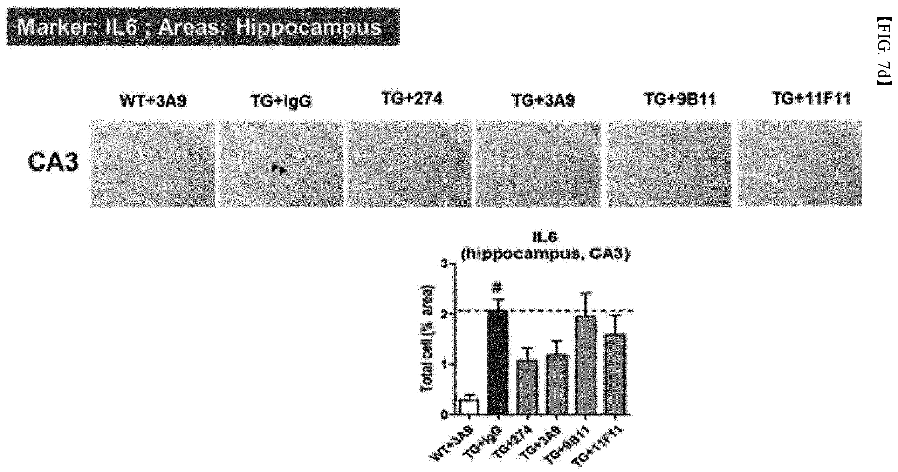

Gliosis is a nonspecific reaction in glial cells that responds to damage of the central nervous system triggered by BBB damage, TGF-beta or interleukin. Representative examples include microgliosis and astrogliosis, for which each of Iba-1 and GFAP protein is used as marker respectively. As described in Example 4, the effect of the antibody according to the present invention on the reduction of microgliosis and astrogliosis and the reduction of inflammatory cytokine release triggering them was analyzed by administering the antibody to mouse. The results are shown in Figs. 7a, 7b, 7c and 7d.

Fig. 7a is a result of staining and measuring the mouse brain tissue with Iba-1(microgliosis) antibody as a marker after administering antibody to mouse, in order to test whether the monoclonal antibody prepared in an embodiment of the present invention can reduce microgliosis in vivo, and the arrow indicates the activated microglia. It was confirmed that the activation of microglia was significantly decreased in the mouse administered with 3A9, 9B11 and 11F11 antibodies.

Fig. 7b is a result of staining and measuring the mouse brain tissue with GFAP (astrogliosis) antibody as a marker after administering antibody to mouse, in order to test whether the monoclonal antibody prepared in an embodiment of the present invention can reduce astrogliosis in vivo, and the arrow indicates the activated astrocyte. It was confirmed that 3A9, 9B11 and 11F11 antibodies inhibited effectively the astrogliosis to a significant level.

Fig. 7c is a result of staining and measuring the mouse brain tissue with IL-1 beta (IL-1β) antibody as a marker after administering antibody to mouse, in order to test whether the monoclonal antibody prepared in an embodiment of the present invention can reduce the inflammatory cytokines in vivo, and the arrow indicates the cells expressing IL-1 beta. IL-1 beta causes inflammation and thus, induces the death and inflammatory response of various nerve cells. IL-1 beta was significantly reduced in the brain tissue of mouse administered with the antibody according to the present invention.

Fig. 7d is a result of staining and measuring the mouse brain tissue with IL-6 antibody as a marker after administering antibody to mouse, in order to test whether the monoclonal antibody prepared in an embodiment of the present invention can reduce the inflammatory cytokines in vivo, and the arrow indicates the cells expressing IL-6. IL-6 was reduced in the brain tissue of mouse administered with the antibody according to the present invention.

As shown in the drawings, the antibodies of the present invention were found to reduce microgliosis and astrogliosis, and reduce the release of the inflammatory cytokines of IL-1beta and IL-6, which triggers microgliosis and astrogliosis, compared to the control group.

### Example 6. Detection of Lewy body in human brain tissue by anti-alpha-synuclein antibody

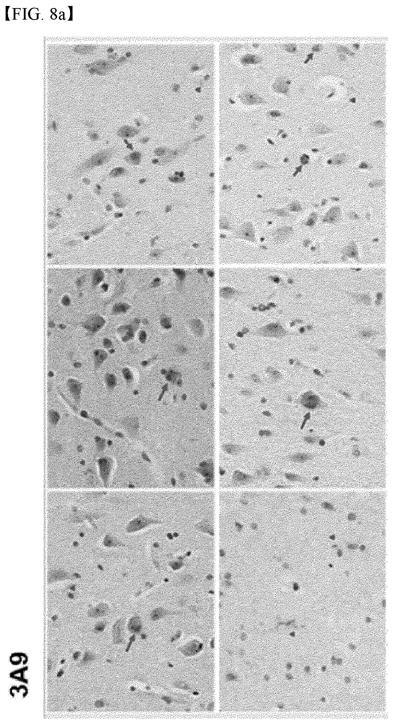

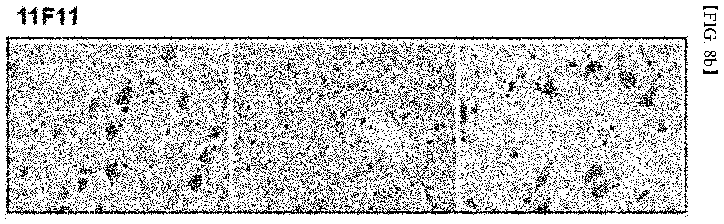

The Lewy body and Lewy neurite in the paraffin-embedded brain sections in a thickness of ten micrometers obtained from patients died of Parkinson's disease (Dr. Halliday, University of Sydney) were stained by using the antibody used in Example 5 as follows. The tissue sections were treated with 90% formic acid for 3 minutes to process the antigen retrieval, and then peroxidase activity of the tissue itself was inhibited by 1% H₂O₂ (50% ethanol base). 10% normal horse serum was treated to prevent non-specific binding of the tissues. Subsequently, after washing with phosphate buffer, the adjacent sections were treated with 3A9, 11F11 and 11F11 antibodies of the present invention at 4 °C overnight. After washing with phosphate buffer, biotin-conjugated anti-human IgG antibody was treated at 37 °C for 30 minutes, and the avidin-biotin complex was reacted at room temperature for 30 minutes (Vectastatin Elite kit; Vector Laboratories). Then, the color was developed with DAB containing 0.005% H₂O₂, and the sections were counter-stained with 0.5% cresyl violet, to discriminate each cell. The results are shown in Figs. 8a and 8b.

Fig. 8a and Fig. 8b show the result of measuring that the monoclonal antibodies of 3A9 and 11F11 according to an embodiment of the present invention can specifically recognize the Lewy bodies and Lewy neurites in human brain tissue, respectively. They show that the antibodies of the present invention bind to the Lewy bodies (arrow) and the Lewy neurites (thread-like shape in lower left part).

As shown in Fig. 8a and Fig. 8b, the antibody of the present invention was shown to effectively bind to the Lewy body and Lewy neurite (indicated by an arrow). These results suggest that it is able to bind effectively to the alpha-synuclein aggregate of a component of the Lewy body in human brain tissue. It is shown that the antibodies delivered to the human brain can effectively and specifically bind to the alpha-synuclein aggregate. These results indicate that the antibodies of the present invention can specifically bind to alpha-synuclein aggregates in human brain tissue actually, and can be effectively used for the prevention and/or treatment of diseases associated with alpha-synuclein etiology.

### Example 7. Epitope analysis of anti-alpha-synuclein antibody

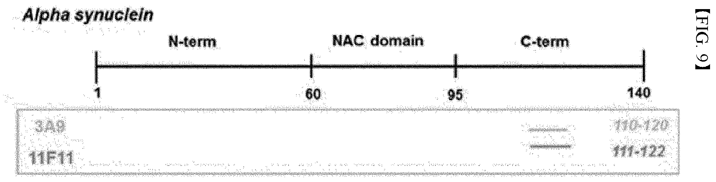

The epitope mapping for 3A9 and 11F11 antibodies of the present invention was performed by requesting PEPSCAN (The Netherlands) for peptide allele analysis. The results are shown in Fig. 9. Fig. 9 is a schematic representation of the epitope mapping results for the antibodies of an embodiment of the present invention.

As shown in Fig. 9, most of the antibodies of the present invention have been shown to bind to C-terminal region. The alpha-synuclein antibodies recognizing N-terminus could not recognize the aggregates of other diseases, such as multiple system atrophy belonging to synucleinopathies which collectively refer to alpha-synuclein-related diseases. In contrast, the alpha-synuclein antibodies recognizing the C-terminal region have an advantage in recognizing the aggregates of other various synucleinopathies as well as Parkinson's disease. Particularly, as described above, the antibodies recognizing the region between 110 and 122 residues have been shown to preferentially bind to the aggregates.

### Example 8. Preparation of anti-alpha-synuclein (chimeric) antibodies

### Cloning

By using the nucleotide sequences of the heavy chain variable region and the light chain variable region antibody obtained after humanization, gblock (m.biotech) of a short nucleotide fragment was synthesized, and cloned into the animal cell culture vector (pcDNA3.4). The gblock was synthesized by including about 20 bp overlapped seqeuence before and after the variable region, and the part of the pcDNA3.4 vector excluding the variable region was amplified by PCR and cloned by Gibson assembly method.

### Transfection and Expression

The prepared vector was used for maxi-prep (Qiagen) to obtain a large amount of plasmid DNA, and then introduced into cells as follows. The day before transfection, the concentration of ExpiCHO ™ (Gibco, Cat: A29127) cells was adjusted to concentration of 3 x 10E6 to 4 x 10E6 viable cells / mL in in ExpiCHO ™ expression medium (Gibco, Cat: A29100-01) and cultured at 8% CO₂, at 37 ° C and 120 rpm for 1 day. On the day of DNA transfection, the cells that were grown to 7 x 10E6 to 10 x 10E6 viable cells / mL and had survival rates of 95% or more were prepared by diluting using fresh medium.to 6 x 10⁶ viable cells / mL.

In order to transfect the parent cells, ExpiFectamine™ CHO & plasmid DNA complex was prepared by using the ExpiFectamine ™ CHO transfection kit (Gibco, Cat: A29129). DNA and ExpiFectamine ™ CHO reagents were prepared at appropriate concentrations by dispensing with cold OptiPRO ™ SFM® (Gibco, Cat: 12309019) medium, were respectively inoculated, and mixed to stand at room temperature for 5 minutes. The product was inoculated into parent cells, and cultured after transfection. The day after transfection, the enhancer and feed included in the ExpiFectamine ™ CHO transfection kit were inoculated into transfected cells, and after 5 days, the feed was additionally inoculated, followed by incubation for 10 days at 8% CO2, 37 ° C, and 120 rpm to produce the transfected cells.

In order to obtain the culture solution, the culture medium is transferred to a centrifuge bottle for centrifugation and centrifuged at 4 °C and 6500 rpm for 30 minutes, followed by filtering with a filter having a size of 0.2 µm to obtain a culture medium with removing suspended solids, and then the culture medium was used for subsequent purification.

### Purification of antibody

The culture was purified using HiTrap MabSelectSure (GE Healthcare, 11-0034-94). After equilibrating with an equilibration buffer (50 mM Tris-HCl pH7.2, 100 mM NaCl), the recovered culture was loaded onto a column. When the loading was completed, the medium was washed with 50 mM Sodium Citrate (pH 5.0), and then eluted using 50 mM Sodium Citrate (pH 3.4). 1M Tris-HCl pH 9.0 was added to the eluate to neutralize to pH 6.0. Then, the eluate was buffer exchanged and concentrated with PBS (phosphate buffered saline, pH 7.4) and stored at 4 ° C until subsequent use. When additional purification was required, a second purification was performed based on the size of the eluted sample by passing the first purified product through 1X PBS buffer on the HiLoad 26/600 superdex 200 column. The amino acid sequence of the purified antibody was analyzed by mass spectrometry, and confirmed to be consistent with the variable region of the mouse-derived monoclonal antibody.

The backbone variable region portion of the human IgG1 isotype was replaced with the variable regions of the 3A9, 9B11, and 11F11 antibodies identified by the above method to prepare a chimeric human IgG1 antibody. Among the obtained chimeric antibodies, especially Ch11F11 antibody is an antibody in the form of IgG and comprises a combination of the heavy chain variable region sequence of SEQ ID NO: 90 (ch11F11-VH) and the light chain variable region sequence of SEQ ID NO: 109 (ch11F11-VL), and Ch 3A9 antibody is an antibody in the form of IgG and comprises a combination of heavy chain variable region sequence of SEQ ID NO: 102 (ch11F11-VH) and light chain variable region sequence of SEQ ID NO: 116 (ch11F11-VL).

### Example 9: Preparation of the humanized antibody

### Library phage preparation

A mini-library in which a mouse or human-derived sequence was introduced into each CDR residue was constructed, while binding the human framework to the CDR1, CDR2, and CDR3 residues of the chimeric antibody.

The competent cells of the produced min library were inoculated in 2X YT medium [17 g of Tripton (CONDA, 1612.00), 10 g of yeast extract (CONDA, 1702.00) and 5 g of NaCl (Sigma, S7653)] containing 34 µg/ml of chloramphenicol (Sigma, C0857), 2% glucose (Sigma, G5400) and 5 mM MgCl₂ (Sigma, C0857) at 30 °C for 3 hours to be OD600 of 0.5 to 0.7. Then, the cells were infected with a helper phage, and cultured in 2X YT medium containing 34 µg/ml of chloramphenicol, 5 mM MgCl₂, 70 µg/ml of kanamycin (Sigma, K1876) and 1 mM IPTG (ELPISBIO, IPTG025) at 30 °C for 6 hours to induce the phage packing. The culture solution was centrifuged at 4500 rpm at 4 ° C for 15 minutes. The supernatant was added with 4% PEG 6000 (Fluka, 81253) and 3% NaCl (Sigma, S7653) and incubated for 1 hour on ice. The product was centrifuged at 8000 rpm for 20 minutes at 4 °C, and then, the pellet was suspended in PBS and centrifuged again at 4 °C and 12,000 rpm for 10 minutes to obtain a supernatant containing the phage library. The obtained supernatant was stored at 4 °C until subsequent use.

### Phage display panning

In order to select antibodies that preferentially bind to alpha-synuclein aggregates to the monomers, the panning was performed using the full-length alpha-synuclein aggregates prepared in Example 1, and total three panning were performed as follows.

Bovine serum albumin (BSA) was added to the cells at a concentration of 3 % in a test tube at 4 °C overnight, adding 10 µg/ml of recombinant alpha-synuclein aggregates and monomers to the PBS in an immunotube (maxisorp 444202) solution was added to the test tube and the surface of which alpha-synuclein aggregates and monomers were not adsorbed was protected. After emptying the test tube, the antibody phage library of 10¹² CFU dispersed in BSA 3% solution was put into the immunotube in which the alpha-synuclein aggregates and monomers were absorbed and reacted for 1 hour (negative selection). Then, the phages were not bound to alpha-synuclein aggregates and monomers were recovered and reacted for 2 hours at room temperature in the alpha-synuclein aggregates and monomers were adsorbed. Phosphate buffered saline (0.05% Tween 20) solution was used to recover 100 µM triethylamine solution, which was recovered by using a PBS-T solution. E. coli at 37 ° C for 1 hour, and the infected E. coli was painted out on a 2X YT agar medium and cultured at 37 ° C overnight (pH 7.4), they were infected by ER2537 . Next day, the cultured E. coli was suspended in a 4 ml of 2X YT culture solution containing carbenicillin and 15% glycerol was added, and a part was stored at -80 °C and the rest was used for preparing phages for next experiments. By repeating this process at 3 rounds in total, alpha-synuclein antigen-specific phage pool was amplified and concentrated. As the panning round progressed, the number of washing using PBS-T was increased to amplify and concentrate the antigen-specific phage.

### Single clone screening

To sort monoclonal antibodies specifically binding to alpha-synuclein aggregate from the phage pool obtained through the panning, the experiment as follows was performed.

To isolate monoclones from the concentrated pool, after painting out the phage pool on a LB-tetracycline/carbenicillin agar medium and culturing, a single colony was secured. Then, after inoculating monoclones on a 96-deep well plate in which 400 *µ*ℓ of 2X YT-tetracycline/carbenicillin medium was put per well and growing overnight, 10 *µ*ℓ culture solution was put on a new 96-deep well plate in which 390 *µ*ℓ of 2X YT-tetracycline/carbenicillin medium was put and it was cultured at 37°C for 4 hours. ImM IPTG was put into the culture solution and it was cultured at 30°C overnight. The culture solution cultured overnight was centrifuged to take a supernatant.

Then, the clones expressing a monoclone-soluble scFv which binds to alpha-synuclein aggregate were selected by using the ELISA method as follows (Steinberger. Rader and Barbas III. 2000. Phage display vectors. In: Phage Display Laboratory Manual. 1sted. Cold Sprin gHarbor Laboratory Press. NY. USA. pp.11.9-11.12*)*. specifically, the selected 7B7 antibody in Example 1-1 was put on a 96-well plate (*Nunc-Immuno Plates, NUNC, USA*) and it was coated at 4°C overnight. 3% BSA was added to each well in an amount of 200 µL, followed by blocking at 37 °C for 2 hours. Then, the alpha-synuclein aggregates and the monomer were loaded at a concentration of 100 ng/well, reacted at 37 °C for 2 hours and washed five times with 300 µL of PBS-T. The prepared single clone supernatant was mixed with 3% BSA in a volume ratio of 1: 1 (vol: vol), and 100 µL of the solution was loaded on the plate bound to the aggregate and the monomer, followed by reaction at 37 C for 2 hours. The cells were washed five times with 300 µL of PBS-T, and incubated at 37 °C for 1 hour with an anti-HA HRP-conjugated antibody, followed by washing with PBS-T five times. After adding 100 µL of TMB (Tetramethylbenzidine, Sigma, T0440), the reaction was stopped by adding 50 µL of 1 N H 2 SO 4 to measure the absorbance at 450 nm. Clones with an absorbance of 0.5 or greater were regarded as positive reaction by binding and clones bind to BSA nonspecifically were excluded.

The CDR residues of clones found in the library are analyzed in silico in parallel and the clones to cause serious problems with binding to the framework or clones that do not have T-cell epitope, B cell epitope, and MHCII epitope in the framework parts other than CDR were selected.

Subsequently, for five antibodies prepared by the following combination of heavy chain and light chain variable regions, the humanized antibody variable region of the human IgG1 isotype was replaced with the backbone variable region portion to prepare five IgGI backbone humanized antibodies. Specifically, Hu11F11_(ABL2-4) is an antibody of IgG type, and includes a combination of heavy chain variable region sequence of SEQ ID NO: 92 (Hu11F11-VH2) and light chain variable region sequence of SEQ ID NO: 113 (Hu11F11-VL4). Hu11F11_(ver.1) is an antibody of IgG type, and includes a combination of heavy chain variable region sequence of SEQ ID NO: 98 (Hu11F11-VH-v1) and light chain variable region sequence of SEQ ID NO: 115 (Hu11F11) -VLv3 4c). Hu11F11_(ver.2) is an antibody of IgG type and includes a combination of heavy chain variable region sequence of SEQ ID NO: 99 (Hu11F11-VH-v2) and light chain variable of SEQ ID NO: 115 (Hu11F11-VLv3 4c). Hu11F11_ (ver.3) is an IgG type antibody, and includes heavy chain variable region sequence of SEQ ID NO: 100 (Hu11F11-VH-v3) and light chain variable region sequence of SEQ ID NO: 115 (Hu11F11-VLv3 4c).

Hu11F11 (ver.4) is a combination of heavy chain variable region sequence of SEQ ID NO: 101 (Hu11F11-VH-v4) and light chain variable region sequence of SEQ ID NO: 115 (Hu11F11-VLv3 4c).

### Example 10. Analysis of phagocytosis promoting activity of humanized antibody

In order to test the efficacy of promoting the phagocytic action of microglial cells to extracellular alpha-synuclein aggregates, for the chimeric antibody obtained in Example 8 and the humanized antibodies obtained in Example 9, hu11F11 (ver.1), hu11F11 (ver.2), hu11F11 (ver.3), hu11F11 (ver.4), and ABL2-4, the phagocytosis promoting activity was analyzed as follows.

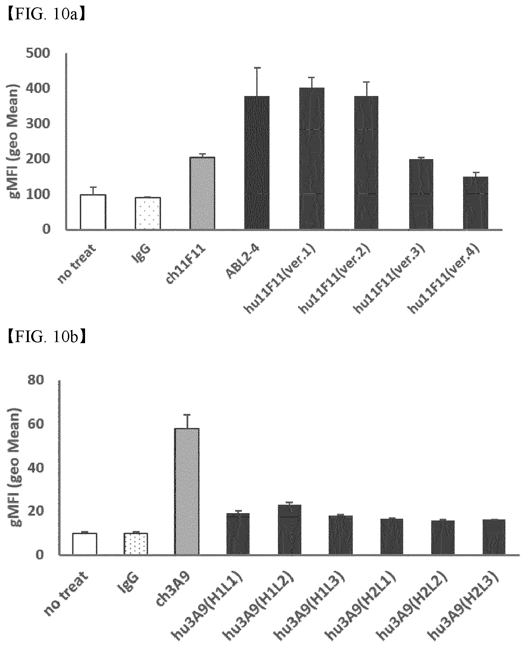

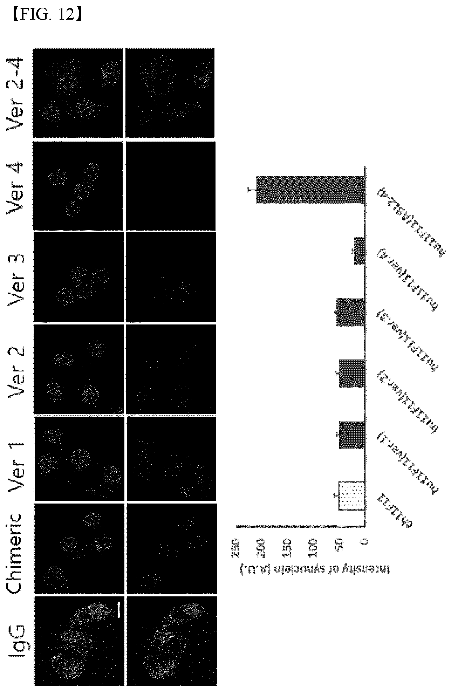

Specifically, the mouse microglia cell line BV-2 was cultured with RPMI1640 and 10% FBS, prepared at a concentration of 2X10⁶ cells/ml, and then dispensed 100 ul in U-bottom 96-well plates. Alpha-synuclein aggregates and chimeric 11F11 or Hu11F11_11F11 (ver.1) or Hu11F11_11F11 (ver.2) or Hu11F11_11F11 (ver.3) or Hu11F11_11F11 (ver.4), or Hu11F11_ABL2-4 antibodies prepared in Examples 8 and 9 were incubated at room temperature for 20 minutes, and their mixture was added to cell lines BV-2 in 96-well plate and reacted for 15 minutes. After removing unbound alpha-synuclein aggregates and antibodies with centrifuge at 1,200 rpm for 5 minutes, the cells were fixed with 4% formaldehyde, washed at three times, and permeablized with 0.5% triton X-100. The antibody recognizing synuclein (Santa Cruz) was incubated for 1 hour, washed 3 times with PBS containing 0.1% Tween20, and then incubated for 1 hour with the anti-rabbit-Alexa-488 antibody. Then, the resultant was washed and analyzed by FACS caliber. The experimental results are shown in FIG. 12a and FIG. 12b, which show the analysis results of the ability of chimeric and humanized antibodies to promote phagocytosis of microglial cells to alpha-synuclein aggregates. Fig. 10a relates to humanized 11F11 antibody, and FIG. 10b relates to humanized 3A9.

As shown in FIG. 10a and Fig. 10b, the chimeric antibody described in the present invention increased the uptake of alpha-synuclein aggregates by about 2 times compared to the IgG control group, and the humanized antibodies increased up to 4 times. Among them, the humanized antibody variants of 11F11, namely Hu11F11_ABL2-4 (combination of Hu11F11-VH2 and Hu11F11-VL4), Hu11F11_(ver.1) (combination of Hu11F11-VH-v1 and Hu11F11-VLv3 4c), and Hu11F11_(ver.2) (combination of Hu11F11-VH-v2 and Hu11F11-VLv3 4c) had excellent activities to promote phagocytosis of BV-2 compared to the chimeric 11F11 antibody. Hu11F11_(ver.3) (combination of Hu11F11-VH-v3 and Hu11F11-VLv3 4c), and hu11F11_(ver.4) (combination of Hu11F11-VH-v4 and Hu11F11-VLv3 4c) showed activities to promote phagocytosis which were similar to that of the chimeric 11F11 antibody.

### Example 11: Fibril binding assay

A fibril binding assay was performed to analyze the efficacy of an antibody to inhibit the binding of alpha-synuclein aggregates to the nerve cells, namely he outpost stage of the phenomenon in which alpha-synuclein aggregates were transmitted to neighboring nerve cells through intercellular propagation.

Specifically, SH-SY5Y cells, which are neuronal cell lines, were treated for 10 minutes with 1 uM of the alpha-synuclein aggregates, and 20 nM of each a chimeric 11F11 antibody and humanized 11F11 variants, namely Hu11F11_ver1, Hu11F11_ver2, Hu11F11_ver3, Hu11F11_ver4, and Hu11F11_ABL2-4, Then, the cells were fixed with 4% formaldehyde. After washing, the alpha-synuclein antibody (BD) that recognizes all forms of alpha-synuclein under non-permeable conditions was treated with 1: 250, washed, and imaged with a confocal microscope. The cell nuclei were shown by DAPI treatment just before imaging.

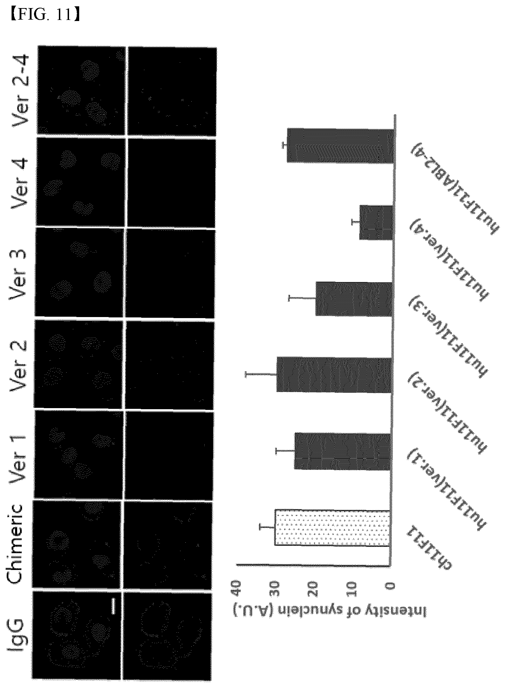

As a result, the antibodies of the present application effectively reduced the degree of cell membrane binding of alpha-synuclein aggregates compared to the IgG control group, and especially, the humanized antibodies of 11F11 reduced the binding degree similarly to or more efficiently than 11F11 chimeric antibody. Fig. 11 is a result of analyzing the ability of the chimeric antibody and the humanized antibodies of the present invention to inhibit the binding of alpha-synuclein aggregates to the surface of nerve cells.

As shown in FIG. 11, the antibodies of the present invention effectively reduced the degree of cell membrane binding of alpha-synuclein aggregates by up to 23 times compared to the IgG control group, and especially, the humanized antibodies of 11F11 reduced the binding degree similarly to or more efficiently than 11F11 chimeric antibody. Hhu11F11_(ver.1) (combination of Hu11F11-VH-v1 and Hu11F11-VLv3 4c), hu11F11_(ver.3) (combination of Hu11F11-VH-v3 and Hu11F11-VLv3 4c), and hu11F11_(ver.4) (combination of Hu11F11-VH-v4 and Hu11F11-VL-v3 4c) showed superior efficacy compared to 11F11 chimeric antibody. ABL2-4 (combination of Hu11F11-VH2 and Hu11F11-VL4) and hu11F11 (ver.2) (Hu11F11-VH-v2 and Hu11F11-VLv3 4c) showed reduction ability similar to 11F11 chimeric antibody.

### Example 12: Dual chamber assay

The dual chamber assay was performed to more directly analyze the efficacy of the humanized antibodies of the present invention to inhibit intercellular propagation of alpha-synuclein aggregates. After plating SH-SY5Y cells (donor cells) overexpressing alpha-synuclein on the insert equipped with permeable membrane, and normal SH-SY5Y cells (recipient cells) on a coverslip in a 12-well plate respectively, the insert including the cultured cell lines overexpressing alpha-synuclein was overlaid on top and incubated overnight. At the same time, the recipient cells were treated with 20 nM of the antibody of the present invention. Then, the recipient cells were fixed with 4% formaldehyde, washed, and then treated with 0.5% TrtonX-100 to create a permeable condition. After washing, the same detection antibody and DAPI as used in Example 14 were treated and imaged with a confocal microscope. The analyzed image is shown in FIG. 12.

As a result, the humanized antibodies of the present invention effectively reduced the propagation degree of alpha-synuclein aggregates to recipient cells compared to the IgG control group, and especially, the humanized antibodies of 11F11 reduced the propagation of alpha-synuclein aggregates to the recipient cells similarly to or more efficiently, than the 11F11 chimeric antibody.

FIG. 12 is a result of analyzing the efficacy of chimeric antibodies and humanized antibodies of the present invention to inhibit the intercellular transmission in which the alpha-synuclein aggregates are secreted from nerve cells overexpressing alpha-synuclein and transfer to normal nerve cells. The SH-SY5Y nerve cell lines overexpressing alpha-synuclein plated on the top chamber secreted alpha-synucleins outside the cell, and the secreted alpha-synucleins were transferred to the normal SH-SY5Y cell line plated in bottom 12-well plate. The antibodies of the present invention significantly reduced the amount of alpha-synuclein inflexed into the normal SH-SY5Y cell line of the bottom plate. In particular, the humanized antibodies of 11F11 clone among the humanized antibodies of the present invention showed to further increase the reduction degree of alpha-synucleins influxed into the recipient cells in bottom plate compared to the chimeric antibodies. More specifically, hu11F11 (ver.2) (combination of Hu11F11-VH-v2 and Hu11F11-VLv3 4c), and hu11F11 (ver.4) (combination of Hu11F11-VH-v4 and Hu11F11-VLv3 4c) had superior inhibitory activity to chimeric antibody, and Hu11F11 (ver.1) (combination of Hu11F11-VH-v1 and Hu11F11-VLv3 4c) and hu11F11 (ver.3) (combination of Hu11F11-VH-v3 and Hu11F11-VLv3 4c) showed the inhibitory ability similar to the chimeric antibody.

The propagation degree of alpha-synuclein aggregates was effectively reduced up to about 22 times by the humanized antibodies of 11F11 clone, compared to the IgG control group. In particular, the humanized antibodies of 11F11 reduced the propagation of alpha-synuclein aggregates to recipient cells similarly or more efficiently than 11F11 chimeric antibodies.

### Example 13. ELISA Analysis of the humanized antibody

The sandwich ELISA was performed to quantitatively analyze the binding affinity of the chimeric antibody (Ch11F11) obtained in Example 8, and the humanized antibodies (Hu11F11) obtained in Example 9, according to the substantially same method of Example 2-2.

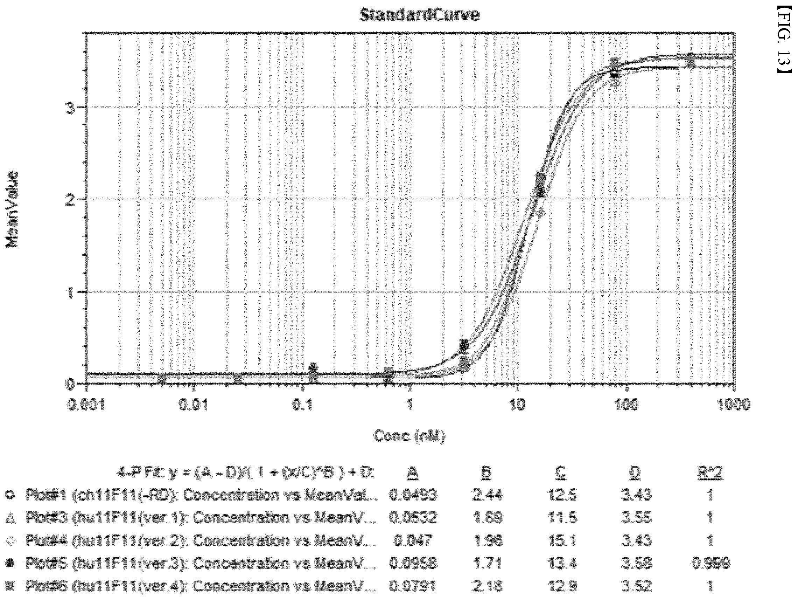

Specifically, each antibody was diluted at 1/10 ratio of concentrations 0.04 to 400 nM and was coated on a 96-well plate, and then each well was treated with synuclein fibril aggregates were treated at 2000ng/ml. After washing with 1XPBS, streptavidin conjugated with HRP and secondary antibody conjugate with biotin was treated and then reacted with TMB as a substrate. The absorbance was measured. The results are shown in FIG.13.

As shown in FIG. 13, it was confirmed that the humanized antibodies according to the present invention, especially the humanized antibodies (humanized 11F11 antibodies) derived from 11F11 chimeric antibody showed the binding affinity equivalent to the chimeric 11F11 clone. The humanized antibodies, especially the variants derived from 11F11 clone such as hu11F11 (ver.1) including a combination of Hu11F11-VH-v1 and Hu11F11-VLv3 4c, hu11F11 (ver.2) including a combination of Hu11F11-VH-v2 and Hu11F11-VLv3 4c, hu11F11 (ver.3) including a combination of Hu11F11-VH-v3 and Hu11F11-VLv3 4c, hu11F11 (ver.4) including a combination of Hu11F11-VH-v4 and Hu11F11-VLv3 4c showed the binding affinity equivalent to the chimeric 11F11 clone, and their EC₅₀ values were 11.5 to 15.1 nM, which were similar to 12.5 nM of EC₅₀ of 11F11 chimeric antibody.

### Example 14: BIAcore analysis using anti-alpha-synuclein antibody

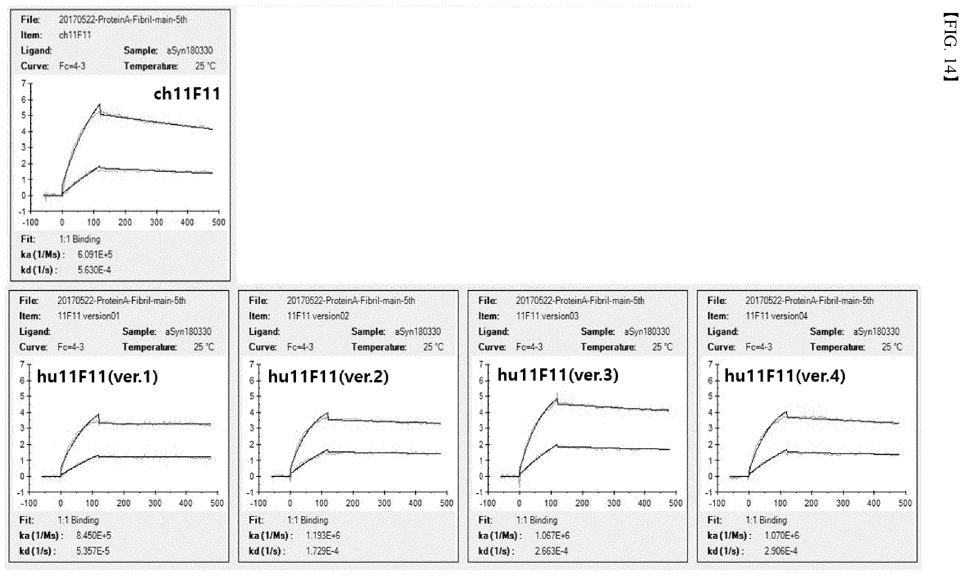

The BIAcore analysis was performed to quantitatively analyze the binding affinity of the chimeric antibodies (Ch11F11) obtained in Example 8, and the humanized antibodies (Hu11F11) obtained in Example 9, according to the substantially same method of Example 2-3. The analysis results are shown in Fig. 14 and the following table.

**[Table 10]**

| Clone name | KD(nM) |
|---|---|
| Ch11F11 | 0.02472 |
| Hu11F11_v2 | 0.0596 |
| Hu11F11_v3 | 0.0316 |
| Hu11F11_v4 | 0.0204 |

As a result, the humanized antibodies of the present invention, especially the variants of clone 11F11, that is hu11F11(ver.2) (combination of Hu11F11-VH-v2 and Hu11F11-VLv3 4c), hu11F11(ver.3) (combination of Hu11F11-VH-v3 and Hu11F11-VLv3 4c), and hu11F11(ver.4) (combination of Hu11F11-VH-v4 and Hu11F11-VLv3 4c) showed KD values similar to that of chimeric 11F11 clone. As for the binding affinity, the humanized clones showed KD values as low as 0.02∼0.06 X 10⁻⁹ M and chimeric 11F11 clone showed KD values as low as 0.02 X 10⁻⁹M, which indicated that they have high binding affinity to the aggregates.

### Example 15. Evaluation of productivity of the humanized antibody

11F11 chimeric antibody and four (4) humanized antibody variants of 11F11 (Hu11F11(ver.1), Hu11F11(ver.2), Hu11F11(ver.3), Hu11F11(ver.4)) were obtained by transfection and culture, primary and secondary purification according to the substantially same method of Example 8, and the amount and the purity of antibody in the finally-purified samples were analyzed.

The antibody amount was analyzed with Thermo Scientific™ NanoDrop™ spectrophotometer. Specifically, after adjusting zero point by loading 1XPBS, a certain amount of purified antibody sample was loaded and measured at UV wavelength of 280 nm. Total yield was obtained by multiplying the sample concentration measured by NanoDrop™ with total volume of the sample, and then the productivity (yield (g/L)) was obtained by dividing the total yield with total volume. The purity of finally-purified product was analyzed with HLC-001(Agilent 1200 series) HPLC. Specifically, the purity was obtained by quantitatively analyzing the ratio of main peak using 1X PBS and 200mM Arginine-HCl(pH 6.8) as mobile phase in TSK gel G3000SWXL. The productivity analysis results are shown in table.

**[Table 11]**

| Sample antibody | concentration (mg/mL) | Purity (injection weight %) | Yield (g/L) |
|---|---|---|---|
| Ch11F11(-RD) | 5.2 | 99.5 | 0.282 |
| Hu11F11_v1 | 5 | 100 | 0.155 |
| Hu11F11 _v2 | 15 | 98.96 | 0.423 |
| Hu11F11 _v3 | 5 | 100 | 0.100 |
| Hu11F11 _v4 | 5 | 99.87 | 0.068 |

As a result, 11F11 chimeric antibody and the humanized antibody variants of 11F11 such as hu11F11(ver.1) (combination of Hu11F11-VH-v1 and Hu11F11-VLv3 4c), hu11F11(ver.2) (combination of Hu11F11-VH-v2 and Hu11F11-VLv3 4c), hu11F11(ver.3) (combination of Hu11F11-VH-v3 and Hu11F11-VLv3 4c), or hu11F11(ver.4) (combination of Hu11F11-VH-v4 and Hu11F11-VLv3 4c) had 0.068 ∼ 0.423 g/L of productivity, which was higher that the yield of the antibody producing by transient transfection generally. Particularly, hu11F11(ver.2) (combination of Hu11F11-VH-v2 and Hu11F11-VLv3 4c) showed 0.423 g/L of productivity, which was higher than 0.282 g/L of 11F11 chimeric antibody.

### Example 16. Evaluation of specific binding to alpha-synuclein by the chimeric antibody

After producing three chimeric antibodies of 3A9, 9B11 and 11F11 according to Example 8, the binding specificities of the antibodies to alpha-synuclein were compared with ELISA analysis results of the beta-synuclein and gamma-synuclein, which were homologs of alpha-synuclein.

To perform the evaluation, each of human beta-synuclein (Uniprot: Q16143) proteins (CUSABIO, Cat: CSB-EP624090HU) and human gamma-synuclein (Uniprot: O76070) proteins (CUSABIO, Cat: CSB-EP021915HU) was coated on 96-well plate at the concentration of 100 ng/ml, washed and then blocked with 5% BSA for 2 hours. At this time, pan-Syn antibody (santacruz, Cat: FL-140, sc-10717, rabbit) that bind to all alpha-synuclein, beta-synuclein, and gamma-synuclein were used as positive control. After washing, the chimeric antibodies (3A9, 9B11, 11F11) diluted to 1/10 concentration of 400 nM to 0.04 nM, were treated for 2 hours, washed with PBS, and then were treated with HRP-bound goat anti-hFc antibodies as detection antibodies. After that, the absorbance was measured at 450nm and 650nm after reacting with TMB as a substrate. For the positive control, HRP-bound goat anti-rabbit was used as a detection antibody.

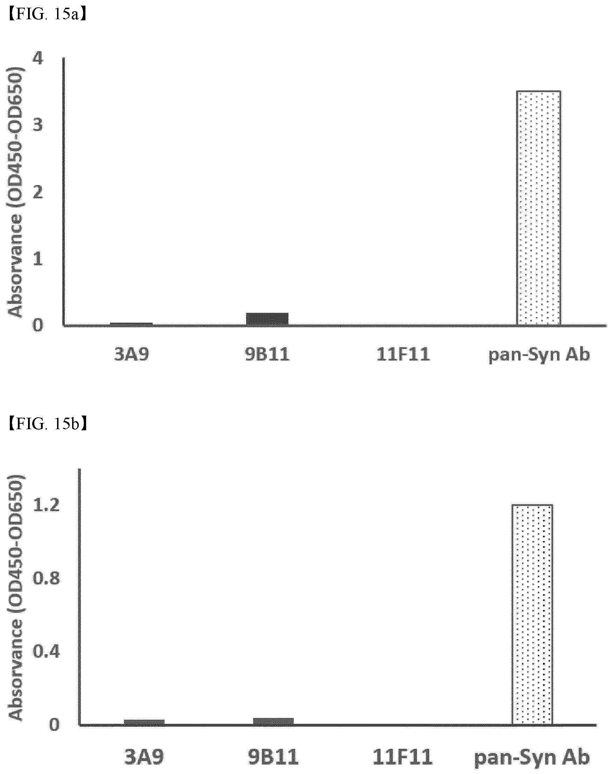

The experimental results are shown in Fig. 15a and Fig. 15b. Fig. 15a and Fig. 15b are ELISA analysis results showing that the chimeric antibodies (3A9, 9B11, 11F11) had very low binding affinities to human beta-synuclein and human gamma-synuclein. In contrast, the pan-Syn antibody as a positive control showed high binding affinities to beta-synuclein and gamma-synuclein. Fig. 15a relates to human beta-synuclein, and Fig. 15b relates to human gamma-synuclein.

In addition, the three antibodies were reacted in Dot blotting with the aggregates of amyloid beta₁₋₄₂ (Uniprot: P05067; CAS number: 107761-42-2) and Tau protein (Uniport: P10636-8), and then, the specific binding abilities of the antibodies to the alpha-synuclein aggregate were analyzed. The reason for such analysis is explained below. Amyloid beta₁₋₄₂ and tau form the aggregates which are thought to be important etiologies for neurodegenerative diseases, especially Alzheimer's disease, and the aggregates have been known to have the structures of oligomers, protofibrils, and fibrils, like the alpha-synuclein aggregates. Therefore, the dot blot analysis is performed to investigate that the chimeric antibodies 3A9, 9B11, and 11F11 recognize specific sequence of alpha-synuclein and do not recognize the common structure of the aggregates derived from alpha-synuclein, amyloid beta, and tau.

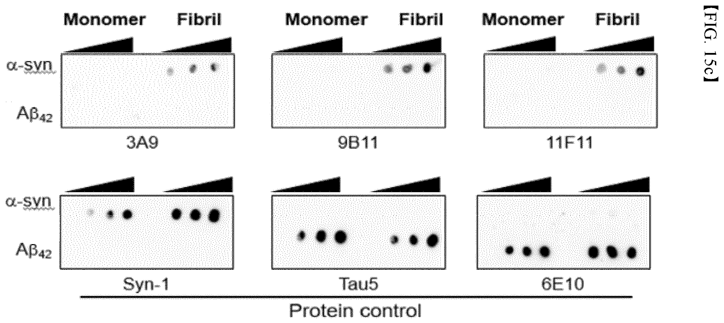

The dot blot method of this example was carried out in substantially the same manner as in Example 2-3, and recombinant amyloid beta₁₋₄₂, tau protein, and aggregates thereof were obtained from Professor Seung-jae Lee of Seoul National University. Syn-1, 6E10, and Tau5 are the antibodies known to bind to alpha-synuclein, amyloid beta₁₋₄₂, and tau proteins, respectively. The results of the analysis are shown in Figure 15c.

The experimental results are shown in FIG. 15c, which indicates that the chimeric antibodies 3A9, 9B11, and 11F11 of the present invention specifically bind only to alpha-synuclein-derived aggregates and not to amyloid beta₁₋₄₂ and tau-derived aggregates. In contrast, 6E10 and Tau5 antibodies, known to bind to amyloid beta₁₋₄₂ and Tau respectively, showed the binding to the corresponding aggregates on the dot blot.

According to the above results, no binding of the chimeric antibodies to the homologs of alpha-synuclein and the aggregates derived from different proteins, represents that the antibodies efficiently bind to only target protein and thus can have superior efficacy to the antibodies or drugs which bind to the homologs and the aggregates derived from the different proteins.

## Claims

**1.** An antibody or antigen binding fragment thereof binding to α-Syn aggregates, comprising
a heavy chain CDR1 (H-CDR1) comprising an amino acid sequence selected from SEQ ID NO: 1 and SEQ ID NO: 6,
a heavy chain CDR2 (H-CDR2) comprising an amino acid sequence selected from SEQ ID NOs: 2 to 4 and SEQ ID NOs: 7 to 8,
a heavy chain CDR3 (H-CDR3) comprising an amino acid sequence selected from SEQ ID NO: 5 and SEQ ID NO: 9,
a light chain CDR1 (L-CDR1) comprising an amino acid sequence selected from SEQ ID NO: 10 and SEQ ID NO: 13,
a light chain CDR2 (L-CDR2) comprising an amino acid sequence selected from SEQ ID NO: 11 and SEQ ID NO: 14, and
a light chain CDR3 (L-CDR3) comprising an amino acid sequence selected from SEQ ID NO: 12 and SEQ ID NO: 15.

**2.** The antibody or antigen binding fragment thereof according to Claim 1, wherein the antibody or antigen binding fragment thereof comprises a heavy chain variable region comprising a heavy chain CDR1 (H-CDR1) comprising an amino acid sequence of SEQ ID NO: 1, a heavy chain CDR2 (H-CDR2) comprising an amino acid sequence selected from SEQ ID NOs: 2 to 4, and a heavy chain CDR3 (H-CDR3) comprising an amino acid sequence of SEQ ID NO: 5. and
a light chain variable region comprising a light chain CDR1 (L-CDR1) comprising an amino acid sequence of SEQ ID NO: 10, a light chain CDR2 (L-CDR2) comprising an amino acid sequence of SEQ ID NO: 11, and a light chain CDR3 (L-CDR3) comprising an amino acid sequence of SEQ ID NO: 12.

**3.** The antibody or antigen binding fragment thereof according to Claim 1, wherein the antibody or antigen binding fragment thereof comprises a heavy chain variable region comprising a heavy chain CDR1 (H-CDR1) comprising an amino acid sequence of SEQ ID NO: 6, a heavy chain CDR2 (H-CDR2) comprising an amino acid sequence selected from SEQ ID NOs: 7 to 8, and a heavy chain CDR3 (H-CDR3) comprising an amino acid sequence of SEQ ID NO: 9. and
a light chain variable region comprising a light chain CDR1 (L-CDR1) comprising an amino acid sequence of SEQ ID NO: 13, a light chain CDR2 (L-CDR2) comprising an amino acid sequence of SEQ ID NO: 14, and a light chain CDR3 (L-CDR3) comprising an amino acid sequence of SEQ ID NO: 15.

**4.** The antibody or antigen binding fragment thereof according to Claim 1, wherein the heavy chain variable region comprises
a heavy chain variable region framework1 (H-FR1) located at N-terminus of H-CDR1 comprising an amino acid sequence selected from SEQ ID NOs: 16 to 17 and SEQ ID NOs: 35 to 40,
a heavy chain variable region framework (H-FR2) located between H-CDR1 and H-CDR2 comprising an amino acid sequence selected from SEQ ID NOs: 18 to 19 and SEQ ID NOs: 41 to 44,
a heavy chain variable region framework (H-FR3) located between H-CDR2 and H-CDR3 comprising an amino acid sequence selected from SEQ ID NO: 20 to 31 and SEQ ID NOs: 45 to 50, and
a heavy chain variable region framework (H-FR4) located at C-terminus of H-CDR3 comprising an amino acid sequence selected from SEQ ID NOs: 32 to 34 and SEQ ID NOs: 51 to 52.

**5.** The antibody or antigen binding fragment thereof according to Claim 1, wherein the light chain variable region comprises,
a light chain variable region framework1 (L-FR1) located at N-terminus of L-CDR1 comprising an amino acid sequence selected from SEQ ID NOs: 53 to 58 and SEQ ID NOs: 71 to 77,
a light chain variable region framework (L-FR2) located between L-CDR1 and L-CDR2 comprising an amino acid sequence selected from SEQ ID NOs 59 to 61 and SEQ ID NOs: 78 to 81,
a light chain variable region framework (L-FR3) located between L-CDR2 and L-CDR3 comprising an amino acid sequence selected from SEQ ID NOs: 62 to 67 and SEQ ID NOs: 82 to 88, and
a light chain variable region framework (L-FR4) located at C-terminus of L-CDR3 comprising an amino acid sequence selected from SEQ ID NOs: 68 to 70 and SEQ ID NO: 89.

**6.** The antibody or antigen binding fragment thereof according to Claim 2, wherein the heavy chain variable region comprises,
a heavy chain framework1 (H-FR1) located at N-terminus of H-CDR1 comprising an amino acid sequence selected from SEQ ID NOs: 16 to,
a heavy chain framework (H-FR2) located between H-CDR1 and H-CDR2 comprising an amino acid sequence selected from SEQ ID NOs: 18 to 19,
a heavy chain framework (H-FR3) located between H-CDR2 and H-CDR3 comprising an amino acid sequence selected from SEQ ID NOs: 20 to 31, and
a heavy chain framework (H-FR4) located at C-terminus of H-CDR3 comprising an amino acid sequence selected from SEQ ID NOs: 32 to 34, and
a light chain variable region comprises,
a light chain framework1 (L-FR1) located at N-terminus of L-CDR1 comprising an amino acid sequence selected from SEQ ID NOs: 53 to 58,
a light chain framework (L-FR2) located between L-CDR1 and L-CDR2 comprising an amino acid sequence selected from SEQ ID NOs: 59 to 61,
a light chain framework (L-FR3) located between L-CDR2 and L-CDR3 comprising an amino acid sequence selected from SEQ ID NOs: 62 to 67, and
a light chain framework (L-FR4) located at C-terminus of L-CDR3 comprising an amino acid sequence selected from SEQ ID NOs: 68 to 70.

**7.** The antibody or antigen binding fragment thereof according to Claim 3, wherein the heavy chain variable region comprises,
a heavy chain framework1 (H-FR1) located at N-terminus of H-CDR1 comprising an amino acid sequence selected from SEQ ID NOs: 35 to 40,
a heavy chain framework (H-FR2) located between H-CDR1 and H-CDR2 comprising an amino acid sequence selected from SEQ ID NOs: 41 to 44,
a heavy chain framework (H-FR3) located between H-CDR2 and H-CDR3 comprising an amino acid sequence selected from SEQ ID NOs: 45 to 50 and
a heavy chain framework (H-FR4) located at C-terminus of H-CDR3 comprising an amino acid sequence selected from SEQ ID NOs: 51 to 52, and
the light chain variable region comprises,
a light chain framework1 (L-FR1) located at N-terminus of L-CDR1 comprising an amino acid sequence selected from SEQ ID NOs: 71 to 77,
a light chain framework (L-FR2) located between L-CDR1 and L-CDR2 comprising an amino acid sequence selected from SEQ ID NOs: 78 to 81,
a light chain framework (L-FR3) located between L-CDR2 and L-CDR3 comprising an amino acid sequence selected from SEQ ID NOs: 82 to 88, and
a light chain framework (L-FR4) located at C-terminus of L-CDR3 comprising an amino acid sequence of SEQ ID NO: 89.

**8.** The antibody or antigen binding fragment thereof according to Claim 1, wherein the heavy chain variable region comprises an amino acid sequence selected from SEQ ID NOs: 90 to 101 or SEQ ID NOs: 102 to 108; an amino acid sequence having sequence identity of at least 90% or more with the amino acid sequence selected from SEQ ID NOs: 90 to 101 or SEQ ID NOs: 102 to 108; or an amino acid sequence having sequence identity of at least 95% or more with the amino acid sequence selected from SEQ ID NOs: 90 to 101 or SEQ ID NOs: 102 to 108.

**9.** The antibody or antigen binding fragment thereof according to Claim 1, wherein the light chain variable region comprises an amino acid sequence selected from SEQ ID NOs: 109 to 115 or SEQ ID NOs: 116 to 123; an amino acid sequence having sequence identity of at least 90% or more with the amino acid sequence selected from SEQ ID NOs: 109 to 115 or SEQ ID NOs: 116 to 123; or an amino acid sequence having sequence identity of at least 95% or more with the amino acid sequence selected from SEQ ID NOs: 109 to 115 or SEQ ID NOs: 116 to 123.

**10.** The antibody or antigen binding fragment thereof according to Claim 1, wherein the antibody or antigen binding fragment thereof comprises a heavy chain variable region comprising an amino acid sequence selected from SEQ ID NOs: 90 to 101 and a light chain variable region comprising an amino acid sequence selected from SEQ ID NOs: 109 to 115.

**11.** The antibody or antigen binding fragment thereof according to Claim 1, wherein the antibody or antigen binding fragment thereof comprises a heavy chain variable region comprising an amino acid sequence selected from SEQ ID NOs: 102 to 108 and a light chain variable region comprising an amino acid sequence selected from SEQ ID NOs: 116 to 123.

**12.** The antibody or antigen binding fragment thereof according to Claim 1, wherein the antibody or antigen binding fragment thereof comprises
a heavy chain variable region of SEQ ID NO: 127 and a light chain variable region of SEQ ID NO: 128,
a heavy chain variable region of SEQ ID NO: 129 and a light chain variable region of SEQ ID NO: 130,
a heavy chain variable region of SEQ ID NO: 131 and a light chain variable region of SEQ ID NO: 132,
a heavy chain variable region of SEQ ID NO: 133 and a light chain variable region of SEQ ID NO: 134,
a heavy chain variable region of SEQ ID NO: 135 and a light chain variable region of SEQ ID NO: 136, or
a heavy chain variable region of SEQ ID NO: 137 and a light chain variable region of SEQ ID NO: 138.

**13.** The antibody or antigen binding fragment thereof which is isolated and binds to alpha-synuclein aggregate according to Claim 1, wherein the antibody or antigen binding fragment thereof is a monoclonal antibody, Fab fragment, Fab' fragment, F(ab') fragment, diabody or scFV.

**14.** The antibody or antigen binding fragment thereof which is isolated and binds to alpha-synuclein aggregate according to Claim 13, wherein the monoclonal antibody is IgG1, IgG2, IgG3 or IgG4 form.

**15.** An antibody or antigen binding fragment thereof which is isolated and binds to alpha-synuclein aggregate according to any one of Claims 1 to 14, wherein the antibody or antigen binding fragment thereof inhibits an intercellular transmission of alpha-synuclein aggregate; degrades alpha-synuclein aggregate; or inhibits formation of alpha-synuclein aggregate.

**16.** An isolated polynucleotide encoding an antibody or antigen binding fragment thereof according to any one of Claims 1 to 14.

**17.** An expression vector comprising the polynucleotide according to Claim 16.

**18.** A cell transfected with the expression vector according to Claim 17.

**19.** A composition comprising an antibody or antigen binding fragment thereof according to any one of Claims 1 to 14.

**20.** The composition according to Claim 19, wherein the composition used for the treatment of α-synucleinopathy comprises further a pharmaceutically-acceptable carrier or excipient.

**21.** The composition according to Claim 20, wherein the α-synucleinopathy includes Parkinson's disease (PD), Parkinson's disease dementia (PDD), dementia with Lewy bodies (DLB), Lewy body variant of Alzheimer's disease (LBV), Combined Alzheimer's and Parkinson disease, or multiple system atrophy (MSA).

**21.** A method of in vivo or in vitro detection for alpha-synuclein aggregate in a biological sample, comprising providing an antibody or antigen binding fragment thereof according to any one of Claims 1 to 14, and contacting the biological sample to be detected of alpha-synuclein aggregate with the antibody or antigen binding fragment thereof.

**22.** A method of treating α-synucleinopathy of a subject or modulating a concentration of alpha-synuclein aggregate in a subject, comprising a step of administering an antibody or an antigen binding fragment thereof according to any one of Claims 1 to 14, or a composition comprising the same at a pharmaceutically effective amount, to a subject in need of treating α-synucleinopathy and/or regulating a concentration of alpha-synuclein aggregates.

**23.** The method according to Claim 22, wherein the α-synucleinopathy includes Parkinson's disease (PD), Parkinson's disease dementia (PDD), dementia with Lewy bodies (DLB), Lewy body variant of Alzheimer's disease (LBV), Combined Alzheimer's and Parkinson disease, or multiple system atrophy (MSA).

**24.** A method of diagnosing α-synucleinopathy in a subject, wherein the method comprises
measuring a concentration and/or intercellular location of alpha-synuclein aggregate in the subject by using an antigen binding fragment thereof according to any one of Claims 1 to 14; and
comparing the concentration and/or the intercellular location of the alpha-synuclein aggregate measured in the subject with that of a control sample, wherein the similarity or difference compared to the control sample result represents the subject with α-synucleinopathy.

**25.** The method according to Claim 24, wherein the α-synucleinopathy includes Parkinson's disease (PD), Parkinson's disease dementia (PDD), dementia with Lewy bodies (DLB), Lewy body variant of Alzheimer's disease (LBV), Combined Alzheimer's and Parkinson disease, or multiple system atrophy (MSA).

**26.** A use of the antibody or antigen binding fragment thereof according to any one of Claims 1 to 14, or a composition comprising the same, for the treatment of α-synucleinopathy in a subject, wherein the antigen binding fragment thereof inhibits an intercellular transmission of alpha-synuclein aggregate; degrades alpha-synuclein aggregate; or inhibits formation of alpha-synuclein aggregate in brain of the subject.

**27.** The use according to Claim 26, wherein the α-synucleinopathy includes Parkinson's disease (PD), Parkinson's disease dementia (PDD), dementia with Lewy bodies (DLB), Lewy body variant of Alzheimer's disease (LBV), Combined Alzheimer's and Parkinson disease, or multiple system atrophy (MSA).

**28.** A use of the antibody or antigen binding fragment thereof according to any one of Claims 1 to 14, or a composition comprising the same, for regulating a concentration of alpha-synuclein aggregate in brain cells, wherein the antibody or an antigen binding fragment thereof or a composition comprising the same inhibits an intercellular transmission of alpha-synuclein aggregate; degrades alpha-synuclein aggregate; or inhibits formation of alpha-synuclein aggregate in brain of the subject.

**29.** An antibody or antigen binding fragment thereof according to any one of Claims 1 to 14, for use in the regulation of a concentration of alpha-synuclein aggregate in brain cells, by inhibiting an intercellular transmission of alpha-synuclein aggregate; degrading alpha-synuclein aggregate; or inhibiting formation of alpha-synuclein aggregate.

**30.** An antibody or antigen binding fragment thereof of any one of Claims 1 to 14 for use in the treatment of α-synucleinopathy.

**31.** The antibody or an antigen binding fragment thereof of any one of Claims 1 to 14 according to Claim 30, wherein the α-synucleinopathy includes Parkinson's disease (PD), Parkinson's disease dementia (PDD), dementia with Lewy bodies (DLB), Lewy body variant of Alzheimer's disease (LBV), Combined Alzheimer's and Parkinson disease, or multiple system atrophy (MSA).
